# EUROPÄISCHE PATENTSCHRIFT

(11) **EP 2 027 107 B1**
(45) Veröffentlichungstag und Bekanntmachung des Hinweises auf die Patenterteilung: **15.02.2012**
(21) Anmeldenummer: 07729171.4
(22) Anmeldetag: 15.05.2007
(51) Int. Cl.: C07D 401/12, C07D 401/14, C07D 405/12, C07D 405/14, C07D 409/14, C07D 417/14, A61P 35/00, A61K 31/506

(54) **2, 4 -DIAMINO PYRIMIDINE ALS INHIBITOREN VON ZELLZYKLUSKINASEN**
2, 4 -DIAMINO PYRIMIDINES AS CELL CYCLE KINASE INHIBITORS
2, 4 -DIAMINO PYRIMIDINE EN TANT QU'INHIBITEURS DE KINASES DU CYCLE CELLULAIRE

(30) Priorität: 15.05.2006 EP 06113967
(43) Veröffentlichungstag der Anmeldung: 25.02.2009
(60) Teilanmeldung: 11159659.9
(73) Patentinhaber: Boehringer Ingelheim International GmbH, 55216 Ingelheim am Rhein (DE)
(72) Erfinder: ZAHN, Stephan Karl, 1130 Wien (AT); BISTER, Bojan, 1120 Wien (AT); BOEHMELT, Guido, 2531 Gaaden (AT); GUERTLER, Ulrich, 1120 Wien (AT); MANTOULIDIS, Andreas, 1120 Wien (AT); REISER, Ulrich, 1130 Wien (AT); SCHOOP, Andreas, 82061 Neuried (DE); SOLCA, Flavio, 1230 Wien (AT); TONTSCH-GRUNT, Ulrike, A-2500 Baden (AT); TREU, Matthias, 1110 Wien (AT)
(74) Vertreter: Hammann, Heinz
(86) Internationale Anmeldenummer: PCT/EP2007/054723
(87) Internationale Veröffentlichungsnummer: WO 2007/132010

(56) Entgegenhaltungen:
- EP-A- 1 598 343
- WO-A-01/72717
- WO-A-02/04429
- WO-A-2004/014382
- WO-A-2006/021544
- WO-A-2007/003596
- US-A1- 2004 224 966
- US-A1- 2005 113 398
- US-A1- 2006 035 891

## Beschreibung

Die vorliegende Erfindung betrifft ausgewählte Verbindungen der allgemeinen Formel (1) 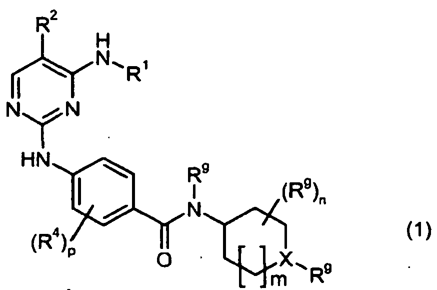 gemäß Anspruch 1,
wobei die Reste R¹, R², R⁴, R^{g}, X, m, n und p die in der Beschreibung genannten Bedeutungen haben, deren Isomere, Verfahren zur Herstellung dieser Pyrimidine sowie deren Verwendung als Arzneimittel.

Der vorliegenden Erfindung liegt die Aufgabe zugrunde, neue Wirkstoffe aufzuzeigen, welche zur Vorbeugung und/oder Behandlung von Krankheiten, die durch exzessive oder anomale Zellproliferation charakterisiert sind, eingesetzt werden können. US 2006/035891 A1 offenbart strukturähnliche 2,4-pyrimidindiamine.

### Detaillierte Beschreibung der Erfindung

Es wurde nun überraschenderweise gefunden, dass Verbindungen der allgemeinen Formel (1), worin die Reste R¹, R², R⁴, R^{g}, X, m, n und p die nachstehend genannten Bedeutungen haben, als Inhibitoren spezifischer Zellzykluskinasen wirken. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft Verbindungen der allgemeinen Formel (1) 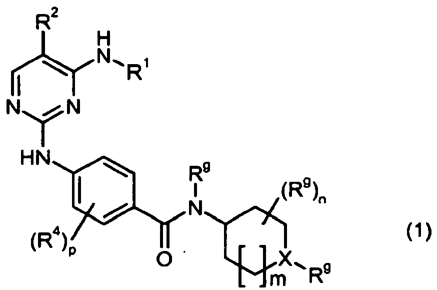 die ausgewählt sind von der folgenden Gruppe:
4-[4-((1R2S)-2-Isopropylcarbamoyl-cyclopentylamino)-5-trifluoromethylpyrimidin-2-ylamino]-*N*-methyl-*N*-(1,2,2,6,6-pentamethyl-piperidin-4-yl)-benzamid;
(±)-4-[4-((1R*,2S*)-2-Isopropylcarbamoyl-cyclopentylamino)-5-trifluoromethyl-pyrimidin-2-ylamino]-*N*-methyl-*N*-(1-methyl-piperidin-4-yl)-benzamid;
4-[4-((1R,2S)-2-Isopropylcarbamoyl-cyclopentylamino)-5-trifluoromethylpyrimidin-2-ylamino]-*N*-methyl-*N*-(1-methyl-piperidin-4-yl)-benzamid;
4-[4-((1S,2R)-2-Isopropylcarbamoyl-cyclopentylamino)-5-trifluoromethylpyrimidin-2-ylamino]-*N*-methyl-*N*-(1-methyl-piperidin-4-yl)-benzamid ;
*N*-(1-Ethyl-piperidin-4-yl)-4-[4-((1R,2S)-2-isopropylcarbamoylcyclopentylamino)-5-trifluoromethyl-pyrimidin-2-ylamino]-*N*-methylbenzamid;
*N*-Isopropyl-4-[4-((1R,2S)-2-isopropylcarbamoyl-cyclopentylamino)-5-trifluoromethyl-pyrimidin-2-ylamino]-*N*-(1-methyl-piperidin-4-yl)-benzamid;
(±)-*N*-Cyclohexyl-4-[4-((1R*,2S*)-2-isopropylcarbamoylcyclopentylamino)-5-trifluoromethyl-pyrimidin-2-ylamino]-*N*-methylbenzamid;
4-[4-((1R,2S)-2-Isopropylcarbamoyl-cyclopentylamino)-5-trifluoromethylpyrimidin-2-ylamino]-*N*-methyl-*N*-(1-methyl-pyrrolidin-3-yl)-benzamid;
4-[4-((1R,2S)-2-Ethylcarbamoyl-cyclopentylamino)-5-trifluoromethylpyrimidin-2-ylamino]-*N*-methyl-*N*-(1-methyl-piperidin-4-yl)-benzamid;
4-[4-((1R,2S)-2-Cyclopropylcarbamoyl-cyclopentylamino)-5-trifluoromethyl-pyrimidin-2-ylamino]-*N*-(1-methyl-piperidin-4-yl)-benzamid;
4-{4-[(1R*,2S*)-2-((R)-2-Hydroxy-1-methyl-ethylcarbamoyl)-cyclopentylamino]-5-trifluormethyl-pyrimidin-2-ylamino}-*N*-methyl-*N*-(1-methyl-piperidin-4-yl)-benzamid;
(±)-4-[4-((1R*,2S*)-2-Isobutylcarbamoyl-cyclopentylamino)-5-trifluoromethyl-pyrimidin-2-ylamino]-*N*-methyl-*N*-(1-methyl-piperidin-4-yl)-benzamid;
(±)-4-{4-[(1R*,2S*)-2-(2-Ethyl-butylcarbamoyl)-cyclopentylamino]-5-trifluoromethyl-pyrimidin-2-ylamino}-*N*-methyl-*N*-(1-methyl-piperidin-4-yl)-benzamid;
(±)-4-{4-[(1R*,2S*)-2-(Cyclohexylmethyl-carbamoyl)-cyclopentylamino]-5-trifluoromethyl-pyrimidin-2-ylamino}-*N*-methyl-*N*-(1-methyl-piperidin-4-yl)-benzamid;
4-{4-[(1R,2S)-2-((S)-2-Hydroxy-1-methyl-ethylcarbamoyl)-cyclopentylamino]-5-trifluoromethyl-pyrimidin-2-ylamino}-*N*-methyl-*N*-(1-methyl-piperidin-4-yl)-benzamid;
(±)-*N*-Methyl-*N*-(1-methyl-piperidin-4-yl)-4-{5-trifluoromethyl-4-[(1R*,2S*)-2-(3,3,3-trifluoro-propylcarbamoyl)-cyclopentylamino]-pyrimidin-2-ylamino}-benzamid;
(±)-4-[4-((1R*-,2S*)-2-Cyclopropylcarbamoyl-cyclopentylamino)-5-trifluoromethyl-pyrimidin-2-ylamino]-*N*-methyl-*N*-(1-methyl-piperidin-4-yl)-benzamid;
4-[4-((1R,2S)-2-Cyclopropylcarbamoyl-cyclopentylamino)-5-trifluoromethyl-pyrimidin-2-ylamino]-*N*-methyl-*N*-(1-methyl-piperidin-4-yl)-benzamid;
(±)-(2S*,3R*)-3-(2-{4-[Methyl-(1-methyl-piperidin-4-yl)-carbamoyl]-phenylamino}-5-trifluoromethyl-pyrimidin-4-ylamino)-bicyclo[2.2.2]octan-2-carbonsäure- isopropylamid;
(±)-(2S*,3R*)-3-(2-{4-[Methyl-(1-methyl-piperidin-4-yl)-carbamoyl]-phenylamino}-5-trifluoromethyl-pyrimidin-4-ylamino)-bicyclo[2.2.2]octan-2-carbonsäure- cyclopentylamid;
4-[4-(3-Isopropylcarbamoyl-cyclobutylamino)-5-trifluoromethyl-pyrimidin-2-ylamino]-*N*-methyl-*N*-(1-methyl-piperidin-4-yl)-benzamid;
4-[4-(3-Ethylcarbamoyl-cyclobutylamino)-5-trifluoromethyl-pyrimidin-2-ylamino]-*N*-methyl-*N*-(1-methyl-piperidin-4-yl)-benzamid;
(±)-(2S*,3R*)-3-(2-{4-[Methyl-(1-methyl-piperidin-4-yl)-carbamoyl]-phenylamino}-5-trifluoromethyl-pyrimidin-4-ylamino)-bicyclo[2.2.2]octane-2-carbonsäure- cyclopropylamid;
(±)-(2S*,3R*)-3-(2-{4-[Methyl-(1-methyl-piperidin-4-yl)-carbamoyl]-phenylamino}-5-trifluoromethyl-pyrimidin-4-ylamino)-bicyclo[2.2.2]octan-2-carbonsäureethylamid;
*N*-Methyl-4-{4-[3-(1-methyl-cyclopropylcarbamoyl)-cyclobutylamino]-5-trifluoromethyl-pyrimidin-2-ylamino}-*N-*(1-methyl-piperidin-4-yl)-benzamid;
4-[4-(3-Cyclopropylcarbamoyl-cyclobulylamino)-5-trifluoromethylpyrimidin-2-ylamino]-*N*-methyl-*N*-(1-methyl-piperidin-4-yl)-benzamid;
*N*-Methyl-4-[4-(3-methylcarbamoyl-cyclobutylamino)-5-trifluoromethylpyrimidin-2-ylamino]-*N*-(1-methyl-piperidin-4-yl)-benzamid;
*N*-Methyl-4-[4-((1R,2S)-2-methylcarbonoyl-cyclopentylamino)-5-trifluoromethyl-pyrimidin-2-ylamino]-*N*-(1-methyl-piperidin-4-yl)-benzamid;
(±)-4-[4-((1R*,2S*)-2-*tert*-Butylcarbomoyl-cyclopentylamino)-5-trifluoromethyl-pyrimidin-2-ylamino]-*N*-methyl-*N*-(1-methyl-piperidin-4-yl)-benzamid;
(±)-*N*-Methyl-4-{4-[(1R*,2S*)-2-(1-methyl-cyclopropylcarbamyol)-cyclopentylamino]-5-trifluoromethyl-pyrimidin-2-ylamino}-*N*-(1-methylpiperidin-4-yl)-benzamid;
*N*-Methyl-4-{4-[(1R,2S)-2-(1-methyl-cyclopropylcarbamoyl)-cyclopentylamino]-5-trifluoromethyl-pyrimidin-2-ylamino}-*N*-(1-methylpiperidin-4-yl)-benzamid;
(±)-4-{4-[(1R*,2S*)-2-(2-Hydroxy-1,1-dimethyl-ethylcarbamoyl)-cyclopentylamino]-5-trifluoromethyl-pyrimidin-2-ylamino}-*N*-methyl-*N*-(1-methyl-piperidin-4-yl)-benzamid;
(±)-4-{4-[(1R*,2S*)-2-(Azetidin-1-carbonyl)-cyclopentylamino]-5-trifluoromethyl-pyrimidin-2-ylaminol-N-methyl-N-(1-methyl-piperidin-4-yl)-benzamid;
(±)-*N*-Methyl-*N*-(1-methyl-piperidin-4-yl)-4-{4-[(1R*,2S*)-2-(piperidin-1-carbonyl)-cyclopentylamino]-5-trifluoromethyl-pyrimidin-2-ylamino}benzamid;
(±)-*N*-Methyl-*N*-(1-methyl-piperidin-4-yl)-4-{4-[(1R*,2S*)-2-(morpholine-4-carbonyl)-cyclopentylamino]-5-trifluoromethyl-pyrimidin-2-ylamino}benzamid;
(±)-*N*-Methyl-4-{4-[(1R*,2S*)-2-(4-methyl-piperazine-1-carbonyl)-cyclopentylamino]-5-trifluoromethyl-pyrimidin-2-ylamino}-*N*-(1-methylpiperidin-4-yl)-benzamid;
(±)-*N*-Methyl-*N*-(1-methyl-piperidin-4-yl)-4-{4-[(1R*,2S*)-2-(thiomorpholine-4-carbonyl)-cyclopentylamino]-5-trifluoromethylpyrimidin-2-ylamino}-benzamid;
(±)-(1S*,2R*)-2-(2-{4-[Methyl-(1-methyl-piperidin-4-yl)-carbamoyl]-phenylamino}-5-trifluoromethyl-pyrimidin-4-ylamino)-cyclopentanecarbonsäure methyl ester;
(±)-4-{4-[(1R*,2S*)-2-((2R,6S)-2,6-Dimethyl-morpholine-4-carbonyl)-cyclopentylamino]-5-trifluoromethyl-pyrimidin-2-ylamino}-*N*-methyl*-N*-(1-methyl-piperidin-4-yl)-benzamid;
4-{4-[(1R,3S)-3-(Cyclopropylmethyl-carbamoyl)-cyclopentylamino]-5-trifluoromethyl-pyrimidin-2-ylamino}-*N-*methyl-*N*-(1-methyl-piperidin-4-yl)-benzamid;
4-[4-((1R,3S)-3-Benzylcarbamoyl-cyclopentylamino)-5-trifluoromethylpyrimidin-2-ylamino]-*N*-methyl-*N*-(1-methyl-piperidin-4-yl)-benzamid;
4-[4-((1 R,3S)-3-Cyclopentylcarbamoyl-cyclopentylamino)-5-trifluoromethyl-pyrimidin-2-ylamino]-*N*-methyl-*N*-(1-methyl-piperidin-4-yl)-benzamid;
4-{4-[(1R,3S)-3-(Cyanomethyl-carbamoyl)-cylopentylamino]-5-trifluoromethyl-pyrimidin-2-ylamino}-*N*-methyl-*N*-(1-methyl-piperidin-4-yl)-benzamid;
4-{4-[(1R,3S)-3-(3-Dimethylamino-propylcarbamoyl)-cyclopentylamino]-5-trifluoromethyl-pyrimidin-2-ylamino}-*N*-methyl-*N*-(1-methyl-piperidin-4-yl)-benzamid;
4-{4-[(1R,3S)-3-(2-Dimethylamino-ethylcarbamoyl)cyclopentylamino]-5-trifluoromethyl-pyrimidin-2-ylamino}-*N*-methyl-*N*-(1-methyl-piperidin-4-yl)-benzamid;
4-{4-[(1R,2S)-2-(4-Dimethylamino-butylcarbamoyl)-cyclopentylamino]-5-trifluoromethyl-pyrimidin-2-ylamino}-*N*-methyl-*N*-(1-methyl-piperidin-4-yl)-benzamid;
*N*-Methyl-*N*-(1-methyl-piperidin-4-yl)-4-{4-[(1R,2S)-2-(3-pyrrolidin-1-yl-propylcarbamoyl)-cyclopentylamino]-5-trifluoromethyl-pyrimidin-2-ylamino}-benzamid;
*N*-Methyl-*N*-(1-melhyl-piperidin-4-yl)-4-(4-{(1R,2S)-2-[(1-methylpiperidin-4-ylmethyl)-carbamoyl]-cyclopentylamino}-5-trifluoromethylpyrimidin-2-ylamino)-benzamid;
*N*-Methyl-*N*-(1-methyl-piperidin-4-yl)-4-{4-[(1R,2S)-2-(1-methyl-piperidin-4-ylcarbamoyl)-cyclopentylamino]-5-trifluoromethyl-pyrimidin-2-ylamino}-benzamid;
4-{4-[(1R,2S)-2-(3-Dimethylamino-2,2-dimethyl-propylcarbamoyl)-cyclopentylamino]-5-trifluoromethyl-pyrimidin-2-ylamino}-*N*-methyl-*N*-(1-methyl-piperidin-4-yl)-benzamid;
*N*-Methyl-*N*-(1-methyl-piperidin-4-yl)-4-{4-[(1R,2S)-2-(3-pyrrolidin-1-yl-cyclobutylcarbamoyl)-cyclopentylamino]-5-trifluoromethyl-pyrimidin-2-ylamino}-benzamid;
*N*-Methyl-*N*-(1-methyl-piperidin-4-yl)-{4-[(1R,2S)-2-(4-morpholin-4-yl-cyclohexylcarbamoyl)-cyclopentylamino]-5-trifluoromethyl-pyrimidin-2-ylamino}-benzamid;
*N*-Methyl-4-(4-{(1R,2S)-2-[3-(4-methyl-piperazin-1-yl)-propylcarbamoyl]-cyclopentylamino}-5-trifluoromethyl-pyrimidin-2-ylamino)-*N*-(1-methylpiperidin-4-yl)-benzamid;
(±)-4-[4-((1R*,2S*)-2-Dimethylcarbamoyl-cyclopentylamino)-5-trifluoromethyl-pyrimidin-2-ylamino]-*N*-methyl-*N*-(1-methyl-piperidin-4-yl)-benzamid;
(±)-4-[4-((1R*,2S*)-2-Cyclobutylcarbamoyl-cyclopentylamino)-5-trifluoromethyl-pyrimidin-2-ylamino]-*N*-methyl-*N*-(1-methyl-piperidin-4-yl)-benzamid;
(±)-4-[4-((1R*,2S*)-2-Cyclopentylcarbamoyl-cyclopentylamino)-5-trifluoromethyl-pyrimidin-2-ylamino]-*N*-methyl-*N*-(1-methyl-piperidin-4-yl)-benzamid;
(±)-*N*-Methyl-4-{4-[(1R*,2S*)-2-(1-methyl-cyclopropylcarbamoyl)-cyclohexylamino]-5-trifluoromethyl-pyrimidin-2-ylamino}-*N*-(1-methylpiperidin-4-yl)-benzamid;
(±)-4-[4-((1R*,2S*)-2-Cyclopropylcarbamoyl-cyclohexylamino)-5-trifluoromethyl-pyrimidin-2-ylamino]-*N*-methyl-*N*-(1-methyl-piperidin-4-yl)-benzamid;
(±)-{4-[(1R*,2S*)-2-(2,2-Difluoro-ethylcarbamoyl)-cyclohexylamino]-5-trifluoromethyl-pyrimidin-2-ylamino}-*N*-methyl-*N*-(1-methyl-piperidin-4-yl)-benzamid;
(±)-4-[4-((1R*,2S*)-2-Dimethylcarbamoyl-cyclohexylamino)-5-trifluoromethyl-pyrimidin-2-ylamino]-*N*-methyl-*N*-(1-methyl-piperidin-4-yl)-benzamid;
(±)-4-[4-((1R*,S*)-2-Ethylcarbamoyl-cyclohexylamino)-5-trifluoromethylpyrimidin-2-ylamino]-*N*-methyl-*N*-(1-methyl-piperidin-4-yl)-benzamid;
(±)-*N*-Methyl-*N*-(1-methyl-piperidin-4-yl)-4-{4-[(1R*,2S*)-2-(piperidin-1-carbonyl)-cyclohexylamino]-5-trifluoromethyl-pyrimidin-2-ylamino}benzamid;
(±)-*N*-Methyl-*N*-(1-methyl-piperidin-4-yl)-4-{4-[(1R*,2S*)-2-(pyrrolidine-1-carbonyl)-cyclohexylamino]-5-trifluoromethyl-pyrimidin-2-ylamino}benzamid;
(±)-4-[4-((1R*,2S*)-2-Isopropylcarbamoyl-cyclohexylamino)-5-trifluoromethyl-pyrimidin-2-ylamino]-*N*-methyl-*N*-(1-methyl-piperidin-4-yl)-benzamid;
(±)-*N*-Methyl-4-[4-((1R*,2S*)-2-methylcarbamoyl-cyclohexylamino)-5-trifluoromethyl-pyridin-2-ylamino]-*N*-(1-methyl-piperidin-4-yl)-benzamid;
(±)-4-{4-[(1R*,2S*)-2-(2-Fluoro-ethylcarbannoyl)-cyclohexylamino]-5-trifluoromethyl-pyrimidin-2-ylamino}-*N*-methyl-*N*-(1-methyl-piperidin-4-yl)-bcnzamid;
*N*-Methyl-*N*-(1-methyl-piperidin-4-yl)-4-{4-[(1R,2S)-2-(tetrahydro-furan-3-ylcarbamoyl)-cyclopenlylamino]-5-trifluoromethyl-pyrimidin-2-ylamino}benzamid;
(±)-*N*-Methyl-*N*-(1-methyl-piperidin-4-yl)-4-{4-[(1R*,2S*)-2-(tetrahydropyran-4-ylcarbamoyl)-cyclopentylamino]-5-trifluoromethyl-pyrimidin-2-ylamino}-benzamid;
*N*-Methyl-*N*-(1-methyl-piperidin-4-yl)-4-{4-[(1R,2S)-2-(2,2,2-trifluoroethylcarbamoyl)-cyclopentylamino]-5-trifluoromethyl-pyrimidin-2-ylaminol-benzamid;
(±)-4-{4-[(1R*,2S*)-2-(2-Dimethylamino-ethylcarbamoyl)-cyclopentylamino]-5-trifluoromethyl-pyrimidin-2-ylamino}-*N*-methyl-*N*-(1-methyl-piperidin-4-yl)-benzamid;
(±)-*N*-Methyl-*N*-(1-methyl-piperidin-4-yl)-4-{4-[(1R*,2S*)-2-(2-pyrrolidin-1-yl-ethylcarbamoyl)-cyclopentylamino]-5-trifluoromethyl-pirimidin-2-ylamino}-benzamid;
4-[4-((1R,3S)-3-Isopropylcarbamoyl-cyclopentylamino)-5-trifluoromethylpyrimidin-2-ylamino]-*N*-methyl-*N*-(1-methyl-piperidin-4-yl)-benzamid;
4-[4-((1R,3S)-3-Carbamoyl-cyclopentylamino)-5-trifluoromethylpyrimidin-2-ylamino]-*N*-methyl-*N*-(1-methyl-piperidin-4-yl)-benzamid;
4-[4-((1S,3R)-3-Isopropylcarbamoyl-cyclopentylamino)-5-trifluoromethylpyrimidin-2-ylamino]-*N*-methyl-*N*-(1-methyl-piperidin-4-yl)-benzamid;
4-[4-((1S,3R)-3-Carbamoyl-cyclopentylamino)-5-trifluoromethylpyrimidin-2-ylamino]-*N*-methyl-*N*-(1-methyl-piperidin-4-yl)-benzamid;
(±)-*N*-Methyl-*N*-(1-methyl-piperidin-4-yl)-4-{4-[(1R*,2S*)-2-(2-morpholin-4-yl-ethylcarbamoyl)-cyclopentylamino]-5-trifluoromethyl-pyrimidin-2-ylamino}-benzamid;
(±)-4-[4-((1R*,2S*)-2-Carbamoyl-cyclobutylamino)-5-trifluoromethylpyrimidin-2-ylamino]-*N*-methyl-*N*-(1-methyl-piperidin-4-yl)-benzamid;
(±)-4-[4-((1R*6S*)-6-Carbamoyl-cyclohex-3-enylamino)-5-trifluoromethyl-pyrimidin-2-ylamino]-*N*-methyl-*N*-(1-methyl-piperidin-4-yl)-benzamid;
(±)-*N*-Methyl-4-[4-((1R*,2S*)-2-methylcarbamoyl-cyclopentylamino)-5-trifluoromethyl-pyrimidin-2-ylamino]-*N*-(1-methyl-piperidin-4-yl)-benzamid;
4-{4-[(1R,3S)-3-(Azetidin-1-carbonyl)-cyclopentylamino]-5-trifluoromethyl-pyrimidin-2-ylamino}-*N*-methyl-*N*-(1-methyl-piperidin-4-yl)-benzamid;
*N*-Methyl-*N*-(1-methyl-piperidin-4-yl)-4-{4-[(1R,3S)-3-(piperidin-1-carbonyl)-cyclopentylamino]-5-trifluoromethyl-pyrimidin-2-ylamino}benzamid;
4-{4-[(1R,3S)-3-(2-Methoxy-ethylcarbamoyl)-cyclopentylamino]-5-trifluoromethyl-pyrimidin-2-ylamino}-*N*-methyl-*N*-(1-methyl-piperidin-4-yl)-benzamid;
*N*-Methyl-*N*-(1-methyl-piperidin-4-yl)-4-{4-[(1R,3S)-3-(pyrrolidine-1-carbonylrcyclopentylamino]-5-trifluoromethyl-pyrimidin-2-ylamino}benzamid;
4-[4-((1R,3S)-3-Ethylcarbamoyl-cyclopentylamino)-5-trifluoromethylpyrimidin-2-ylamino]-*N*-methyl-*N*-(1-methyl-piperidin-4-yl)-benzamid;
(±)-(1S*,2R*)-2-(2-{4-[Methyl-(1-methyl-piperidin-4-yl)-carbamoyl]-phenylamino}-5-trifluoromethyl-pyrimidin-4-ylamino)-cyclopentanecarbonsäure;
(±)-4-{4-[(1R*,2S*)-2-(Cyanomethyl-carbamoyl)-cyclopentylamino]-5-trifluoromethyl-pyrimidin-2-ylamino}-*N*-methyl-*N*-(1-methyl-piperidin-4-yl)-benzamid;
(±)-4-{4-[(1R*,2S*)-2-(2-Cyano-ethylcarbamoyl)-cyclopentylamino]-5-trifluoromethyl-pyrimidin-2-ylamino}-*N*-methyl-*N*-(1-methyl-piperidin-4-yl)-benzamid;
(±)-{4-[(1R*,2S*)-2-(Carbamoylmethyl-carbamoyl)-cyclopentylamino]-5-trifluoromethyl-pyrimidin-2-ylamino}-*N*-methyl-*N*-(1-methyl-piperidin-4-yl)-benzamid;
*N*-Methyl-*N*-(1-methyl-piperidin-4-yl)-4-{4-[(1R,2S)-2-(3-oxo-piperazin-1-carbonyl)-cyclopentylamino]-5-trifluoromethyl-pyridin-2-ylamino}benzamid;
4-(4- {(1R,2S)-2-[(Carbamoyl-cyano-methyl)-carbamoyl]-cyclopentylamino}-5-trifluoromethyl-pyrimidin-2-ylamino)-*N*-methyl-*N*-(1-methyl-piperidin-4-yl)-benzamid;
4-{4-[(1R,2S)-2-((S)-1-Carbamoyl-3-methyl-butylcarbamoyl)-cyclopentylamino]-5-trifluoromethyl-pyrimidin-2-ylamino}-*N*-methyl-*N*-(1-methyl-piperidin-4-yl)-benzamid;
(±)-4-{4-[(1R*,2S*)-2-(Dimethylcarbamoylmethyl-carbamoyl)-cyclopentylamino]-5-trifluoromethyl-pyrimidin-2-ylamino}-*N*-methyl-*N*-(1-methyl-piperidin-4-yl)-benzamid;
(±)-*N*-Methyl-4-{4-[(1R*,2S*)-2-(methylcarbamoylmethyl-carbamoyl)-cyclopentylamino]-5-trifluoromethyl-pyrimidin-2-ylamino}-*N*-(1-methylpiperidin-4-yl)-benzamid;
(±)-4-{4-[(1R*,2S*)-2-((R)-1-Carbamoyl-3-methyl-butylcarbamoyl)-cyclopentylamino]-5-trifluoromethyl-pyrimidin-2-ylamino}-*N*-methyl-*N*-(1-methyl-piperidin-4-yl)-benzamid;
(±)-4-{4-[(1R*,2S*)-2-(2-Carbamoyl-ethylcarbamoyl)-cyclopentylamino]-5-trifluoromethyl-pyrimidin-2-ylamino}-*N*-methyl-*N*-(1-methyl-piperidin-4-yl)-benzamid;
(R)-1-[(1S,2R)-2-(2-{4-[Methyl-(1-methyl-piperidin-4-yl)-carbamoyl]-phenylamino}-5-trifluoromethyl-pyrimidin-4-ylamino)-cyclopentanecarbonyl]-pyrrolidine-2-carbonsäureamid;
(S)-1-[(1S,2R)-2-(2-{4-[Methyl-(1-methyl-piperidin-4-yl)-carbamoyl]-phenylamino}-5-trifluoromethyl-pyrimidin-4-ylamino)-cyclopentanecarbonyl]-pyrrolidine-2-carbonsäureamid;
4-{4-[(1R,2S)-2-((S)-Carbamoyl-ethylcarbamoyl)-cyclopentylamino]-5-trifluoromethyl-pyrimidin-2-ylamino}-*N*-methyl-*N*-(1-methyl-piperidin-4-yl)-benzamid;
4-{4-[(1R,2S)-2-((R)-1-Carbamoyl-ethylcarbamoyl)-cyclopentylamino]-5-trifluoromethyl-pyrimidin-2-ylamino}-*N*-methyl-*N*-(1-methyl-piperidin-4-yl)-benzamid;
(±)-4-{4-[(1R*,2S*)-2-(2-Amino-2-methyl-propylcarbamoyl)-cyclopentylamino]-5-trifluoromethyl-pyrimidin-2-ylamino}-*N*-methyl-*N*-(1-methyl-piperidin-4-yl)-benzamid;
(±)-*N*-Methyl-*N*-(1-methyl-piperidin-4-yl)-4-{4-[(1R*,2S*)-2-(thiazol-2-ylcarbamoyl)-cyclopentylamino]-5-trifluoromethyl-pyrimidin-2-ylamino}benzamid;
(±)-4-[4-((1R*,2S*)-2-Amino-cyclohexylamino)-5-trifluoromethylpyrimidin-2-ylamino]-*N*-methyl-*N*-(1-methyl-piperidin-4-yl)-benzamid;
(±)-4-[4-((1R*,2S*)-2-Acetylamino-cyclohexylamino)-5-trifluoromethylpyrimidin-2-ylamino]-*N*-methyl-*N*-(1-methyl-piperidin-4-yl)-benzamid;
(±)-4-[4-((1R*,2S*)-2-Methanesulfonylamino-cyclohexylamino)-5-trifluoromethyl-pyrimidin-2-ylamino]-*N*-methyl-*N*-(1-methyl-piperidin-4-yl)-benzamid;
(±)-4-[4-((1R*,2S*)-2-Isobutyrylamino-cyclohexylamino)-5-trifluoromethyl-pyrimidin-2-ylamino]-*N*-methyl-*N*-(1-methyl-piperidin-4-yl)-benzamid;
(±)-4-{4-[(1R*,2S*)-2-(Cyclopropanecarbonyl-amino)-cyclohexylamino]-5-trifluoromethyl-pyrimidin-2-ylamino}-*N*-methyl-*N*-(1-methyl-piperidin-4-yl)-benzamid;
(±)-*N*-Methyl-*N*-(1-methyl-piperidin-4-yl)-4-[4-((1R*,2S*)-2-propionylamino-cyclohexylamino)-5-trifuoromethyl-pyrimidin-2-ylamino]-benzamid;
(±)-4-[4-((1R*,2S*)-2-Ethanesulfonylamino-cyclohexylamino)-5-trifluoromethyl-pyrimidin-2-ylamino]-*N*-methyl-*N*-(1-methyl-piperidin-4-yl)-benzamid;
(±)-4-{4-[(1R*,2S*)-2-(3,3-Dimethyl-ureido)-cyclohexylamino]-5-trifluoromethyl-pyrimidin-2-ylamino}-*N*-methyl-*N*-(1-methyl-piperidin-4-yl)-benzamid;
(±)-(1R*,2S*,3R*,4S*)-3-(2-{4-[Methyl-(1-methyl-piperidin-4-yl)-carbamoyl]-phenylamino}-5-trifluoromethyl-pyrimidin-4-ylamino)-bicyclo[2.2.1]hept-5-en-2-carbonsäure;
4-[4-(Adamantan-2-ylamino)-5-trifluoromethyl-pyrimidin-2-ylamino]-*N-*methyl-*N*-(1-memyl-piperidin-4-yl)-benzamid;
(±)-(1R*,2R*,3S*,4S*)-3-(2-{4-[Methyl-(1-methyl-piperidin-4-yl)-carbamoyl]-phenylamino}-5-trifluoromethyl-pyrimidin-4-ylamino)-bicyclo[2.2.1]heptan-2-carbonsäure cyclopropylamid;
(±)-4-[4-((1R*,2S*)-2-Isobutyrylamino-cyclopentylamino)-5-trifluoromethyl-pyrimidin-2-ylamino]-*N*-methyl-*N*-(1-methyl-piperidin-4-yl)-benzamid;
(±)-*N*-Methyl-4-{4-[(1R*,2S*)-2-(3-methyl-butyrylamino)-cyclopentylamino]-5-trifluoromethyl-pyrimidin-2-ylamino}-*N*-(1-methylpiperidin-4-yl)-benzamid;
(±)-*N*-Methyl-*N*-(1-methyl-piperidin-4-yl)-4-[4-((1R*,2S*)-2-propionylamino-cyclopentylamino)-5-trifluoromethyl-pyrimidin-2-ylamino]-benzamid;
(±)-4-{4-[(1R*,2S*)-2-(2-Methoxy-acetylamino)-cyclopentylamino]-5-trifluoromethyl-pyrimidin-2-ylamino}-*N*-methyl-*N*-(1-methyl-piperidin-4-yl)-benzamid;
(±)-4-[4-((1R*,2S*)-2-Amino-cyclopentylamino)-5-trifluoromethylpyrimidin-2-ylanlinol-*N*-methyl-*N*-(1-methyl-piperidin-4-yl)-benzamid;
*N*-Methyl-*N*-(1-methyl-piperidin-4-yl)-4-(4-{(1R,2S)-2-[2-(1-methylpyrrolidin-2-yl)-ethylcarbamoyl]-cyclopentylamino}-5-trifluoromethylpyrimidin-2-ylamino)-benzamid;
4-{4-[(1R,2S)-2-(2-Difluoro-ethylcarbamoyl)-cyclopenlylamino]-5-trifluoromethyl-pyrimidin-2-ylamino}-*N*-methyl-*N*-(1-methyl-piperidin-4-yl)-benzamid;
4-{4-[(1R,2S)-2-(2-Fluoro-ethylcarbamoyl)-cyclopentylamino]-5-trifluoromethyl-pyrimidin-2-ylamino}-*N*-methyl-*N*-(1-methyl-piperidin-4-yl)-benzamid;
2-Methoxy-*N*-methyl-4-[4-((1R,2S)-2-methylcarbamoyl-cyclopentylamino)-5-trifluoromethyl-pyrimidin-2-ylamino]-*N*-(1-methyl-piperidin-4-yl)-benzamid;
4-[4-((1R,2S)-2-Isopropylcarbamoyl-cyclopentylamino)-5-trifluoromethylpyrimidin-2-ylamino]-2-methoxy-*N*-methyl-*N*-(1-methyl-piperidin-4-yl)-benzamid;
4-[4-((1R,2S)-2-Cyclopropylcarbamoyl-cyclopentylamino)-5-trifluoromethyl-pyrimidin-2-ylamino]-2-methoxy-*N*-methyl-*N*-(1-methylpiperidin-4-yl)-benzmid;
4-[4-((1R,2S)-2-Isopropylcarbamoyl-cyclopentylamino)-5-trifluoromethylpyrimidin-2-ylamino]-*N*-methyl-*N*-(1-methyl-piperidin-4-yl)-2-propoxybenzamid;
2-Ethoxy-4-[4-((1R,2S)-2-isopropylcarbamoyl-cyclopentylamino)-5-trifluormethyl-pyrimidin-2-ylamino]-*N*-methyl-*N*-(1-methyl-piperidin-4-yl)-benzamid;
2-Isopropoxy-4-[4-((1R,2S)-2-isopropylcarbamoyl-cyclopentylamino)-5-trifluoromethyl-pyrimidin-2-ylamino]-*N*-methyl-*N*-(1-methyl-piperidin-4-yl)-benzamid;
4-[4-((1R,2S)-2-Isopropylcarbamoyl-cyclopentylamino)-5-trifluoromethylpyrimidin-2-ylamino]-*N*-methyl-*N*-(1-methyl-piridin-4-yl)-2-prop-2-ynyloxy-benzamid;
(±)-4-[4-((1R*,2S*)-2-Isopropylcarbamoyl-cyclobutylamino)-5-trifluormethyl-pyrimidin-2-ylamino]-*N-*methyl-*N-*(1-methyl-piperidin-4-yl)-henzamid;
(±)-4-{5-Bromo-4-[(1R*,2S*)-2-(1-methyl-cyclopropylcarbamoyl)-cyclopentylamino]-pyrimidin-2-ylamino}-*N*-methyl-*N*-(1-methyl-piperidin-4-yl)-benzamid;
(±)-4-[5-Bromo-4-((1R*,2S*)-2-Isopropylcarbamoyl-cyclopentylamino)-pyrimidin-2-ylamino]-*N*-methyl-*N*-(1-methyl-piperidin-4-yl)-benzamid;
(±)-4-[5-Brom-4-[(1R*,2S*)2-cyclopropylcarbamoyl)-cyclopentylamino)-pyrimidin-2-ylamino]-*N*-methyl-*N*-(1-methyl-piperidin-4-yl)-benzamid;
(±)-4-{5-Bromo-4-[(1R*,2S*)-2-(2-cyano-ethylcarbamoyl)-cyclopentylamino]-pyrimidin-2-ylamino}-*N*-methyl-*N*-(1-methyl-piperidin-4-yl)-benzamid;
(±)-4-[5-Bromo-4-((1R*,2S*)-2-cyclopentylcarbamoyl-cyclopentylamino)-pyrimidin-2-ylamino]-*N*-methyl-*N*-(1-methyl-piperidin-4-yl)-benzamid;
4-{5-Bromo-4-[(1R,2S)-2-(pyrrolidine-1-carbonyl)-cyclopenlylamino]-pyrimidin-2-ylamino}-*N*-methyl-*N*-(1-methyl-piperidin-4-yl)-benzamid;
4-{4-[(1R,2S)-2-(Azetidin-1-carbonyl)-cyclopentylamino]-5-bromopyrimidin-2-ylamino}-*N*-methyl-*N*-(1-methyl-piperidin-yl)-benzamid;
(±)-4-[5-Bromo-4-((1R*,2S*)-2-cyclobutylcarbamoyl-cyclopentylamino)-pyrimidin-2-ylamino]-*N*-methyl-*N-*(1-methyl-piperidin-4-yl)-benzamid;
4-[4-((1R,2S)-2-Isopropylcarbamoyl-cyclopentylamino)-5-methylpyrimidin-2-ylamino]-*N*-methyl-*N*-(1-methyl-piperidin-4-yl)-benzamid;
4-[5-Bromo-4-((1R,2S)-2-isopropylcarbamoyl-cyclopentylamino)-pyrimidin-2-ylamino]-*N*-methyl-*N*-(1-methyl-piperidin-4-yl)-benzamid;
4-[5-Chloro-4-((1R,2S)-2-isopropylcarbamoyl-cyclopentylamino)-pyrimidin-2-ylamino]-*N*-methyl-*N*-(1-methyl-piperidin-4-yl)-benzamid.

Ein weiterer Aspekt der Erfindung sind ausgewählte Verbindungen der allgemeinen Formel (I), oder deren pharmazeutisch wirksamen Salze, zur Verwendung als Arzneimittel.

Ein weiterer Aspekt der Erfindung sind ausgewählte Verbindungen der allgemeinen Formel (1), oder deren pharmazeutisch wirksamen Salze, zur Herstellung eines Arzneimittels mit antiproliferativer Wirkung.

Ein weiterer Aspekt der Erfindung ist eine pharmazeutische Zubereitung, enthaltend als Wirkstoff eine oder mehrere ausgewählte Verbindungen der allgemeinen Formel (1), oder deren pharmazeutisch wirksamen Salze, gegebenenfalls in Kombination mit üblichen Hilfs- und/oder Trägerstoffen.

Ein weiterer Aspekt der Erfindung ist die Verwendung von ausgewählten Verbindungen der allgemeinen Formel (1) zur Herstellung eines Arzneimittels zur Behandlung und/oder Prävention von Krebs, Infektionen, Entzündungs- und Autoimmunerkrankungen.

Ein weiterer Aspekt der Erfindung ist eine pharmazeutische Präparation umfassend eine ausgewählte Verbindung der allgemeinen Formel (1), gegebenenfalls in Form ihrer Tautomeren, ihrer Racemate, ihrer Enantiomere, ihrer Diastereomere und ihrer Gemische, sowie gegebenenfalls ihrer pharmazeutisch wirksamen Salze und mindestens eine weitere zytostatische oder zytotoxische, von Formel (1) verschiedene Wirksubstanz.

### Definitionen

Wie hierin verwendet treffen folgenden Definitionen zu, falls nicht anders beschrieben.
m ist 0 oder 1;
n ist 0, 1, 2, 3 oder 4;
p ist 0, 1 oder 2;
worin
X ist N oder CH ;
R¹ ist C₃₋₁₀Cycloalkyl, substituiert mit R³ und gegebenenfalls mit einem oder mehreren R⁴ ;
R² ist ein Rest ausgewählt aus der Gruppe bestehend aus Wasserstoff, Halogen, -CN, -NO₂, C₁₋₄Alkyl, C₁₋₄Haloalkyl, C₃₋₁₀Cycloalkyl, C₄₋₁₆Cycloalkylakyl und C₇₋₁₆Arylalkyl ;
R³ ist ein geeigneter Rest ausgewählt aus der Gruppe bestehend aus -C(O)R^{c}, -C(O)OR^{c}, -C(O)NR^{c}R^{c}, -S(O)₂R^{c}, -N(R^{f})S(O)₂R^{c}, -N(R^{f})C(O)R^{c}, -N(R^{f})C(O)OR^{c}, und -N(R^{f})C(O)NR^{c}R^{c} ;
R⁴ ist ein Rest ausgewählt aus der Gruppe bestehend aus R^{a}, R^{b} und R^{a} substituiert mit einem oder mehreren, gleich oder verschiedenen R^{c} und/oder R^{b}, und
jedes R^{a} unabhängig voneinander ausgewählt ist aus der Gruppe bestehend aus C₁₋₆Akyl, C₃₋₁₀Cycloalkyl, C₄₋₁₆Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-12 gliedriges Heteroaryl und 6-18 gliedriges Heteroarylalkyl, und jedes R^{b} ein geeigneter Rest und jeweils unabhängig voneinander ausgewählt ist aus der Gruppe bestehend aus =O, -OR^{c}, C₁₋₃Haloalkyloxy, -OCF₃, =S, -SR^{c}, =NR^{c}, =NOR^{c}, -NR^{c}R^{c}, Halogen, -CF₃, -CN, -NC, -OCN, -SCN, -NO₂, -S(O)R^{c}, -S(O)₂R^{c}, - S(O)₂OR^{c}, -S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c}, -C(O)R^{c}, -C(O)OR^{c}, -C(O)NR^{c}R^{c}, -CN(R^{f})NR^{c}R^{c}, -CN(OH)R^{c}, -CN(OH)NR^{c}R^{c}, -OC(O)R^{c}, -OC(O)OR^{c}, -OC(O)NR^{c}R^{c}, -OCN(R^{f})NR^{c}R^{c}, -N(R^{f})C(O)R^{c}, -N(R^{f})C(S)R^{c}, -N(R^{f})S(O)₂R^{c}, -N(R^{f})C(O)OR^{c}, -N(R^{f})C(O)NR^{c}R^{c}, -[N(R^{f})C(O)]₂R^{c}, -N[C(O)]₂R^{c}, -N[C(O)]₂OR^{c}, -[N(R^{f})C(O)]₂OR^{c} und -N(R^{f})CN(R^{f})NR^{c}R^{c}, ;
jedes R^{c} ist unabhängig voneinander Wasserstoff oder ein gegebenenfalls mit einem oder mehreren, gleich oder verschiedenen R^{d} und/oder R^{e} substituierter Rest ausgewählt aus der Gruppe bestehend aus C₁₋₆Alkyl, C₃₋₁₀Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-12 gliedriges Heteroaryl und 6-18 gliedriges Heteroarylalkyl ;
jedes R^{d} ist unabhängig voneinander Wasserstoff oder ein gegebenenfalls mit einem oder mehreren, gleich oder verschiedenen R^{e} und/oder R^{f} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-12 gliedriges Heteroaryl und 6-18 gliedriges Heteroarylalkyl ;
jedes R^{e} ist ein geeigneter Rest und jeweils unabhängig voneinander ausgewählt aus der Gruppe bestehend aus =O, -OR^{f}, C₁₋₃Haloalkyloxy, -OCF₃, =S, -SR^{f}, =NR^{f}, =NOR^{f}, -NR^{f}R^{f}, Halogen, -CF₃, -CN, -NC, -OCN, -SCN, -NO₂, -S(O)R^{f}, -S(O)₂R^{f}, - S(O)₂OR^{f}, -S(O)NR^{f}R^{f}, -S(O)₂NR^{f}R^{f}, -OS(O)R^{f}, -OS(O)₂R^{f}, -OS(O)₂OR^{f}, -OS(O)₂NR^{f}R^{f}, -C(O)R^{f}, -C(O)OR^{f}, -C(O)NR^{f}R^{f}, -CN(R²)NR^{f}R^{f}, -CN(OH)R^{f}, -C(NOH)NR^{f}R^{f}, -OC(O)R^{f}, -OC(O)OR^{f}, -OC(O)NR^{f}R^{f}, -OCN(R^{g})NR^{f}R^{f}, -N(R^{g})C(O)R^{f}, -N(R^{g})C(S)R^{f}, -N(R^{g})S(O)₂R^{f}, -N(R^{d})C(O)OR^{f}, -N(R^{g})C(O)NR^{f}R^{f}, und -N(R^{g})CN(R^{f})NR^{f}R^{f} ;
jedes R^{f} ist unabhängig voneinander Wasserstoff oder ein gegebenenfalls mit einem oder mehreren, gleich oder verschiedenen R^{g} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-12 gliedriges Heteroaryl und 6-18 gliedriges Heteroarylalkyl ;
jedes R^{g} ist unabhängig voneinander Wasserstoff oder C₁₋₆Alkyl

Unter Alkyl-Substituenten sind jeweils gesättigte, ungesättigte, unverzweigte oder verzweigte aliphatische Kohlenwasserstoffreste (Alkylrest) zu verstehen und umfasst sowohl gesättigte Alkylreste als auch ungesättigte Alkenyl- und Alkinylreste. Alkenyl-Substituenten sind jeweils unverzweigt oder verzweigte, ungesättigte Alkylreste, die mindestens eine Doppelbindung aufweisen. Unter Alkinyl-Substituenten sind jeweils unverzweigt oder verzweigte, ungesättigte Alkylreste, die mindestens eine Dreifachbindung aufweisen, zu verstehen.

Heteroalkyl repräsentiert unverzweigt oder verzweigte aliphatische Kohlenwasserstoffketten, die 1 bis 3 Heteroatome enthalten, wobei jedes der verfügbaren Kohlenstoff- und Heteroatome in der Heteroalkylkette gegebenenfalls jeweils unabhängig voneinander substituiert sein kann und die Heteroatome unabhängig voneinander ausgewählt werden aus der Gruppe bestehend aus O, N, P, PO, PO₂, S, SO und SO₂ (z. B. Dimethylaminomethyl, Dimethylaminoethyl, Dimethylaminopropyl, Diethylaminomethyl, Diethylaminoethyl, Diethylaminopropyl, 2-Disopropylaminoethyl, Bis-2-methoxyethylamino, [2-(Dimethylamino-ethyl)-ethyl-amino]-methyl, 3-[2-(Dimethylamino-ethyl)-ethyl-amino]-propyl, Hydroxymethyl, 2-Hydroxyethyl, 3-Hydroxypropyl, Methoxy, Ethoxy, Propoxy, Methoxymethyl, 2-Methoxyethyl).
Halogenalkyl bezieht sich auf Alkylreste, in denen ein oder mehrere Wasserstoffatome durch Halogenatome ersetzt sind. Halogenalkyl umfasst sowohl gesättigte Alkylreste als auch ungesättigte Alkenyl- und Alkinylreste, wie beispielsweise -CF₃, -CHF₂, -CH₂F, -CF₂CF₃,-CHFCF₃, -CH₂CF₃, -CF₂CH₃, -CHFCH₃, -CF₂CF₂CF₃, -CF₂CH₂CH₃, -CF=CF₂, -CCl=CH₂, -CBr=CH₂, -CJ=CH₂, -C≡C-CF₃, -CHFCH₂CH₃ und -CHFCH₂CF₃. Halogen bezieht sich auf Fluor-, Chlor-, Brom- und/oder Jodatome.

Unter Cycloalkyl ist ein mono- oder polyzyklischer Ring zu verstehen, wobei das Ringsystem ein gesättigter Ring aber auch ein ungesättigter, nicht-aromatischer Ring beziehungsweise eine Spiroverbindung sein kann, welcher gegebenenfalls auch Doppelbindungen enthalten kann, wie zum Beispiel Cyclopropyl, Cyclopropenyl, Cyclobutyl, Cyclobutenyl, Cyclopentyl, Cyclopentenyl, Cyclohexyl, Cyclohexenyl, Cycloheptanyl, Cycloheptenyl, Norbornyl, Norbornenyl, Indanyl, Adamantyl, Bicyclo[2.2.3]octanyl, Spiroheptanyl und Spiro[4.2]heptanyl.

Cycloalkylalkyl umfasst vorstehend definiertes nicht-zyklisches Alkyl, in dem ein an einem Kohlenstoffatom gebundenes Wasserstoffatom, üblicherweise an einem terminalen C-Atom, durch eine vorstehend definierte Cycloalkylgruppe ersetzt ist.

Aryl bezieht sich auf monozyklische oder bizyklische Ringe mit 6-12 Kohlenstoffatomen wie beispielsweise Phenyl und Naphthyl.

Arylalkyl umfasst vorstehend definiertes nicht-zyklisches Alkyl, in dem ein an einem Kohlenstoffatom gebundenes Wasserstoffatom, üblicherweise an einem terminalen C-Atom, durch eine vorstehend definierte Arylgruppe ersetzt ist.

Unter Heteroaryl sind mono- oder polyzyklische Ringe zu verstehen, welche anstelle eines oder mehrere Kohlenstoffatome ein oder mehrere, gleich oder verschiedene Heteroatome enthalten, wie z.B. Stickstoff-, Schwefel- oder Sauerstoffatome. Beispielsweise genannt seien Furyl, Thienyl, Pyrrolyl, Oxazolyl, Thiazolyl, Isoxazolyl, Isothiazolyl, Pyrazolyl, Imidazolyl, Triazolyl, Tetrazolyl, Oxadiazolyl, Thiadiazolyl, Pyridyl, Pyrimidyl, Pyridazinyl, Pyrazinyl und Triazinyl. Beispiele für bizyklische Heteroarylreste sind Indolyl, Isoindolyl, Benzofuranyl, Benzothienyl, Benzoxazolyl, Benzothiazolyl, Benzisoxazolyl, Benzisothiazolyl, Benzimidazolyl, Indazolyl, Isoquinolinyl, Quinolinyl, Quinoxalinyl, Cinnolinyl, Phthalazinyl, Quinazolinyl und Benzotriazinyl, Indolizinyl, Oxazolopyridinyl, Imidazopyridinyl, Naphthyridinyl, Indolinyl, Isochromanyl, Chromanyl, Tetrahydroisochinolinyl, Isoindolinyl, Isobenzotetrahydrofuranyl, Isobenzotetrahydrothienyl, Isobenzothienyl, Benzoxazolyl, Pyridopyridinyl, Benzotetrahydrofuranyl, Benzotetrahydrothienyl, Purinyl, Benzodioxolyl, Triazinyl, Phenoxazinyl, Phenothiazinyl, Pteridinyl, Benzothiazolyl, Imidazopyridinyl, Imidazothiazolyl, Dihydrobenzisoxazinyl, Benzisoxazinyl, Benzoxazinyl, Dihydrobenzisothiazinyl, Benzopyranyl, Benzothiopyranyl, Coumarinyl, Isocoumarinyl, Chromonyl, Chromanonyl, Pyridinyl-N-oxid Tetrahydroquinolinyl, Dihydroquinolinyl, Dihydroquinolinonyl, Dihydroisoquinolinonyl, Dihydrocoumarinyl, Dihydroisocoumarinyl, Isoindolinonyl, Benzodioxanyl, Benzoxazolinonyl, Pyrrolyl-N-oxid, Pyrimidinyl-N-oxid, Pyridazinyl-N-oxid, Pyrazinyl-N-oxid, Quinolinyl-N-oxid, Indolyl-*N*-oxid, Indolinyl-N-oxid, Isoquinolyl-N-oxid, Quinazolinyl-*N*-oxid, Quinoxalinyl-N-oxid, Phthalazinyl-N-oxid, Imidazolyl-N-oxid, Isoxazolyl-N-oxid, Oxazolyl-N-oxid, Thiazolyl-N-oxid, Indolizinyl-*N*-oxid, Indazolyl-N-oxid, Benzothiazolyl-N-oxid, Benzimidazolyl-N-oxid, Pyrrolyl-N-oxid, Oxadiazolyl-N-oxid, Thiadiazolyl-N-oxid, Triazolyl-N-oxid, Tetrazolyl-N-oxid, Benzothiopyranyl-S-oxid und Benzothiopyranyl-S,S-dioxid.

Heteroarylalkyl umfasst vorstehend definiertes nicht-zyklisches Alkyl, in dem ein an einem Kohlenstoffatom gebundenes Wasserstoffatom, üblicherweise an einem terminalen C-Atom, durch eine vorstehend definierte Heteroarylgruppe ersetzt ist.

Heterocycloalkyl bezieht sich auf 3 - 12 Kohlenstoffatome umfassende gesättigte oder ungesättigte, nicht aromatische mono-, polyzyklische oder überbrückte polyzyklische Ringe oder Spiroverbindungen, welche anstelle eines oder mehrere Kohlenstoffatome Heteroatome, wie Stickstoff, Sauerstoff oder Schwefel, tragen. Beispiele für solche Heterocylylreste sind Tetrahydrofuranyl, Pyrrolidinyl, Pyrrolinyl, Imidazolidinyl, Imidazolinyl, Pyrazolidinyl, Pyrazolinyl, Piperidinyl, Piperazinyl, Indolinyl, Isoindolinyl, Morpholinyl, Thiomorpholinyl, Homomorpholinyl, Homopiperidinyl, Homopiperazinyl, Homothiomorpholinyl, Thiomorpholinyl-S-oxid, Thiomorpholinyl-S,S-dioxid, Tetrahydropyranyl, Tetrahydrothienyl, Homothiomorpholinyl-S,S-Dioxid, Oxazolidinonyl, Dihydropyrazolyl, Dihydropyrrolyl, Dihydropyrazinyl, Dihydropyridinyl, Dihydropyrimidinyl, Dihydrofuryl, Dihydropyranyl, Tetrahydrothienyl-S-oxid, Tetrahydrothienyl-S,S-dioxid, Homothiomorpholinyl-S-oxid, 2-Oxa-5-azabicyclo[2.2.1]heptan, 8-Oxa-3-aza-bicyclo[3.2.1]octan, 3,8-Diaza-bicyclo[3.2.1]octan, 2,5-Diaza-bicyclo[2.2.1]heptan, 3,8-Diaza-bicyclo[3.2.1]octan, 3,9-Diazabicyclo[4.2.1]nonan und 2,6-Diaza-bicyclo[3.2.2]nonan.

Heterocycloalkylalkyl bezieht sich auf vorstehend definiertes nicht-zyklisches Alkyl, in dem ein an einem Kohlenstoffatom gebundenes Wasserstoffatom, üblicherweise an einem terminalen C-Atom, durch eine vorstehend definierte Heterocycloalkylgruppe ersetzt ist.

Unter dem Ausdruck "substituiert" ist zu verstehen, dass ein Wasserstoffatom, welches direkt am betrachteten Atom gebunden ist, durch ein anderes Atom oder eine andere Atomgruppe ersetzt ist. Bivalente Substituenten wie etwa =O, =S, =NR, =NOR, =NNRR, =NN(R)C(O)NRR, =N₂ oder weitere erfordern den Austausch gegen zwei Wasserstoffatome, welche direkt am betrachteten Atom gebunden sind. Entsprechend können solche bivalente Substituenten nicht Substituenten an aromatischen Systemen sein.

### Abkürzungsliste

| eq | Äquivalent(e) | IR | Infrarotspektroskopie |
|---|---|---|---|
| Ac | Acetyl | Kat., kat | Katalysator, katalytisch |
| Bn | Benzyl | konz. | konzentriert |
| Boc | *t*-Butyloxycarbonyl | Kp., Sdp. | Koch- oder Siedepunkt |
| Bu | Butyl | LC | liquid chromatography |
| bzw. | beziehungsweise | LDA | Lithiumdiisopropylamid |
| cHex | Cyclohexan | min | Minute(n) |
| DC | Dünnschichtchromatographie | Me | Methyl |
| DCC | Dicyclohexylcarbodiimid | MeCN | Acetonitril |
| DCM | Dichlormethan | MS | Massenspektrometrie |
| DMAP | *N,N*-Dimethylaminopyridin | NMP | *N*-Methylpyrrolidon |
| DMF | *N,N*-Dimethylformamid | NMR | nuclear magnetic resonance |
| DMA | *N,N*-Dimethylacetamid | Ph | Phenyl |
| DMSO | Dimethylsulfoxid | Pr | Propyl |
| EE | Ethylacetat (Essigsäureethylester) | rac | racemisch |
| ESI | electron spray ionization | R_{f}(Rf) | Retentionsfaktor |
| Et | Ethyl | RP | Reversed Phase |
| EtOH | Ethanol | RT | Raumtemperatur oder Retentionszeit (HPLC) |
| h | Stunde(n) | *tert* | tertiär |
| Hex | Hexyl | THF | Tetrahydrofuran |
| HPLC | high performance liquid chromatography | TBTU | *O*-(Benzotriazol-1-yl)-*N,N,N',N'*-tetramethyl-uronium tetrafluorborat |
| Hünig-Base | *N*-Ethyl-diisopropylamin | UV | Ultraviolett |
| i | iso | | |

Die nachfolgenden Beispiele illustrieren die vorliegende Erfindung ohne sie jedoch in ihrem Umfang einzuschränken.

### Allgemeines

Alle Reaktionen werden, sofern nicht anders beschrieben, in kommerziell erhältlichen Apparaturen nach im chemischen Laboratorien gängigen Verfahren durchgeführt. Die benutzten Lösungsmittel werden in *pro analysi* Qualität gekauft und ohne weitere Reinigung eingesetzt. Alle Reagenzien werden ebenfalls direkt ohne weitere Reinigung in der Synthese verwendet.
Luft- und/oder feuchtigkeitsempfindliche Ausgangsstoffe werden vorzugsweise unter Schutzgas aufbewahrt und entsprechende Reaktionen und Manipulationen mit letzteren unter Schutzgas (Stickstoff oder Argon) durchgeführt.

### Chromatographie

Für die präparative Mitteldruck-Chromatographie (MPLC, Normalphase) wird Kieselgel der Firma Millipore (Granula Silica Si-60A 35-70 µm) oder C-18 RP-Kieselgel (RP-Phase) der Firma Macherey Nagel (Polygoprep 100-50 C 18) eingesetzt.
Die Dünnschichtchromatographie erfolgt auf DC-Fertigplatten Kieselgel 60 auf Glas (mit Fluoreszenzindikator F-254) der Fa. Merck.
Für die präparative HPLC werden Säulen der Firma Waters (XTerra Prep. MS C18, 5 µM, 30*100 mm bzw. XTerra Prep. MS C18, 5 µm, 50*100 mm OBD oder Symmetrie C18, 5 µm, 19*100 mm), die analytische HPLC (Reaktionskontrolle) wird mit Säulen der Firma Agilent (Zorbax SB-C8, 5 µm, 21,2*50mm) durchgeführt.
Für die chirale HPLC werden Säulen der Firma Daicel Chemical Industries, LTD. (Chiralpak AD-H, Chiralpak AS, Chiracel OD-RH, Chiracel OD-H oder Chiracel OJ-H in diversen Größen und 5 µm Material) verwendet.

### HPLC-Massenspektroskopie/UV-Spektrometrie

Die Retentionszeiten/MS-ESI⁺ zur Charakterisierung der Beispiele werden mit Hilfe einer HPLC-MS Anlage (high performance liquid chromatography mit Massendetektor) der Firma Agilent erzeugt.
Die Anlage ist so aufgebaut, dass anschließend an die Chromatographie (Säule: XTerra MS C18, 2.5 µm, 2.1*30 mm, Fa. Waters, Part.No. 186000592) ein Diodenarry-Detektor (G1315B von Fa. Agilent) und ein Massendetektor (1100 LS-MSD SL; G1946D; Fa. Agilent) in Reihe geschaltet sind.
Diese Anlage wird mit einem Fluss von 1.1 mL/min betrieben. Für einen Trennvorgang wird ein Gradient innerhalb von 3.1 min durchlaufen (Gradient Anfang: Wasser/MeCN 95/5, Gradient Ende: Wasser/MeCN 5/95; beiden Lösungsmitteln wird jeweils 0.1% HCOOH (Ameisensäure) beigemischt).

Soweit die Herstellung der Ausgangsverbindungen nicht beschrieben ist, sind diese kommerziell erhältlich oder analog zu bekannten Verbindungen oder hier beschriebenen Verfahren herstellbar. In der Literatur beschriebene Substanzen werden nach den publizierten Syntheseverfahren hergestellt.

### Herstellung der erfindungsgemäßen Verbindungen

Die Herstellung der erfindungsgemäßen Verbindungen kann nach den im Folgenden beschriebenen Syntheseverfahren erfolgen, wobei die Substituenten der allgemeinen Formeln die zuvor genannten Bedeutungen haben. Diese Verfahren sind als Erläuterung der Erfindung zu verstehen ohne selbige auf deren Gegenstand und den Umfang der beanspruchten Verbindungen auf diese Beispiele zu beschränken.

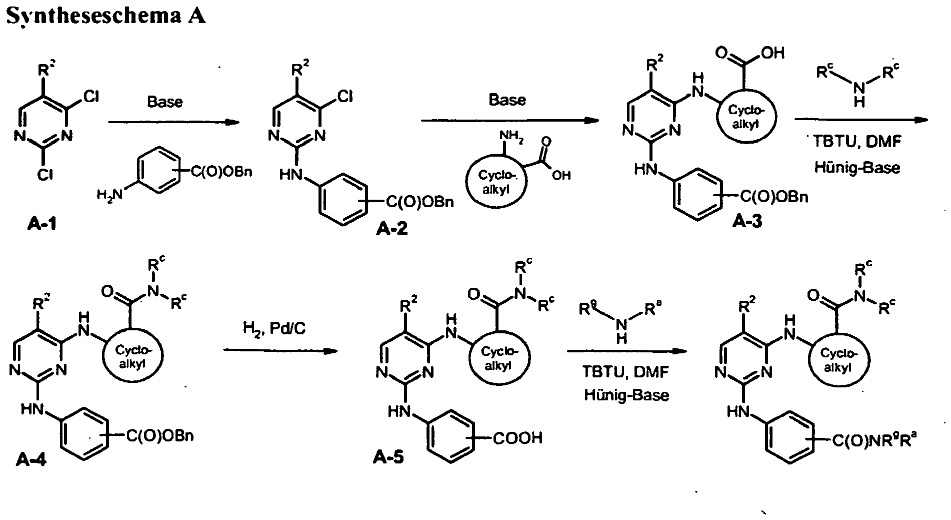

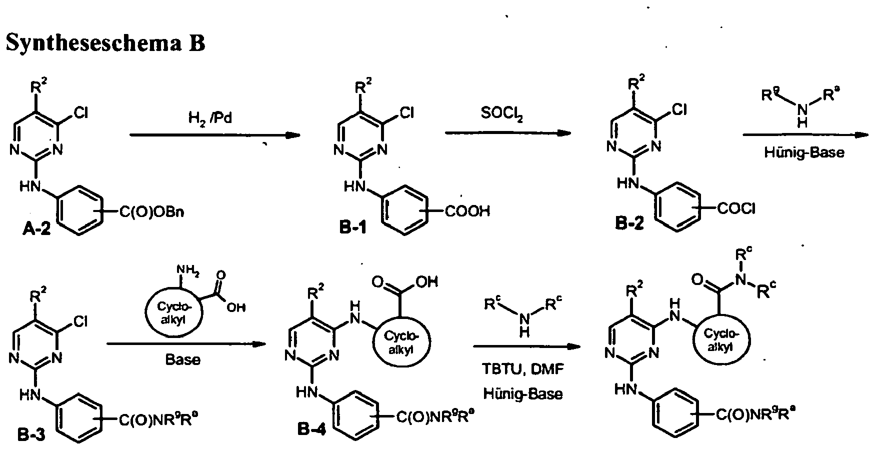

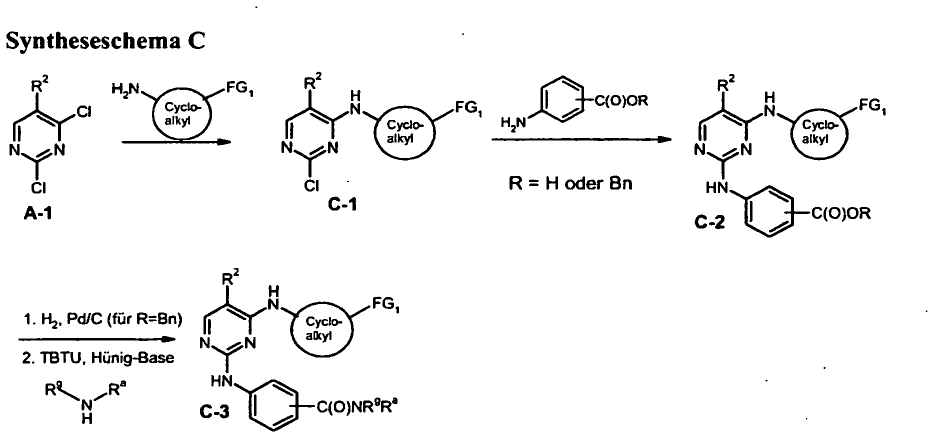

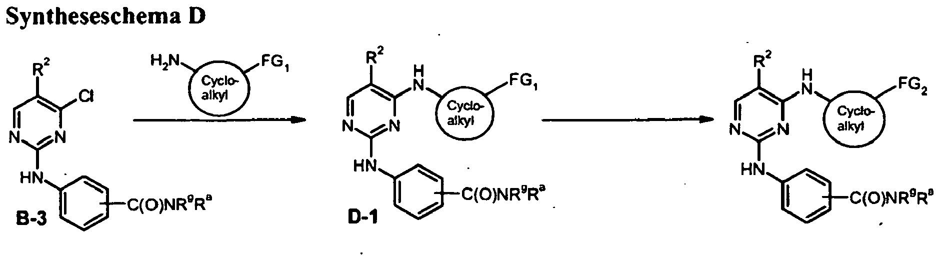

Optional ist nach dem Aufbau des Diaminopyrimidins noch eine Transformation einer oder mehrerer funktioneller Gruppen (FG₁ oder FG₂) möglich. Dies ist in den Beispielen beschrieben, sofern relevant.

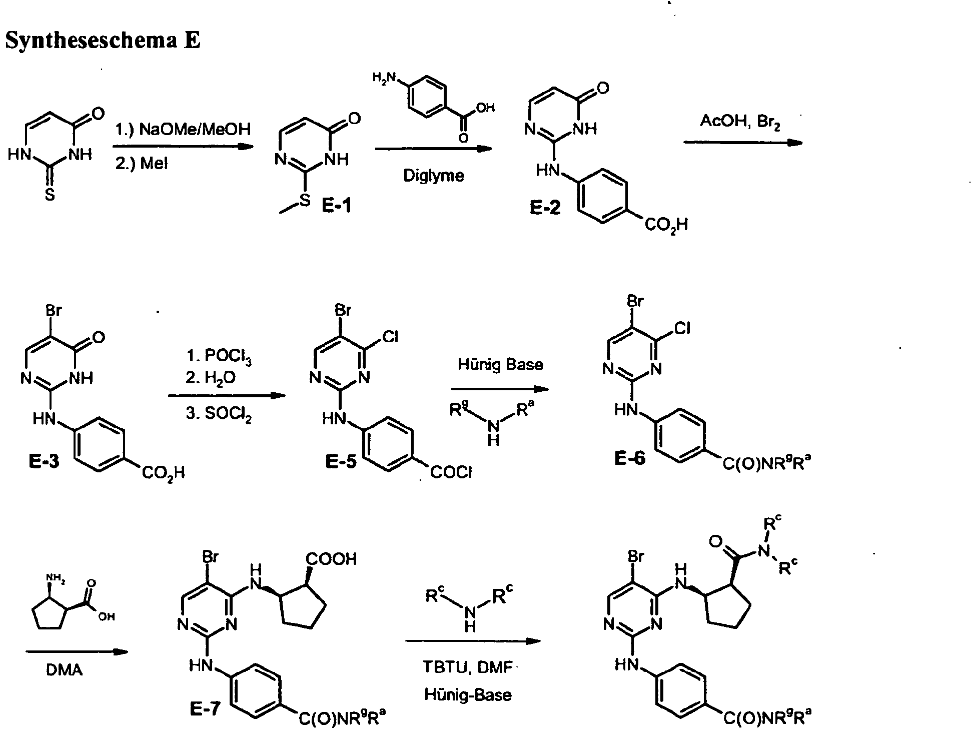

### A-1a) 2,4-Dichlor-5-trifluormethyl-pyrimidin

48 g (267 mmol) 5-Trifluormethyluracil werden unter Feuchtigkeitsausschluss in 210 mL Phosphoroxychlorid (POCl₃) suspendiert. Zu dieser Suspension werden 47.7 g (320 mmol, 1.2 eq) Diethylanilin so langsam zugetropft, dass die Temperatur zwischen 25 °C und 30 °C bleibt. Nach beendeter Zugabe wird noch 5-10 min im Wasserbad gerührt und der Ansatz für 5 - 6 h unter Feuchtigkeitsausschluss bei 80 - 90 °C erwärmt. Das erhaltene Gemisch wird in ca. 1200 g schwefelsaures Eiswasser eingerührt und die wässrige Phase sofort 3 mal mit jeweils 500 mL Ether oder *tert*-Butyl-methyl-ether extrahiert. Die vereinigten etherischen Extrakte werden 2 mal mit je 300 mL schwefelsaurem Eiswasser (ca. 0.1 M) sowie mit kalter Kochsalzlösung gewaschen und getrocknet. Man filtriert das Trocknungsmittel ab und entfernt das Lösungsmittel im Vakuum. Der Rückstand wird im Vakuum (10 mbar) über eine kurze Kolonne destilliert (Kopftemperatur: 65 - 70 °C), wobei man 35.3 g einer Flüssigkeit erhält, die unter Schutzgas abgefüllt und gelagert wird. DC: R_{f}= 0.83 (cHex:EE =3:1)

### 4-Aminobenzoesäurebenzylester

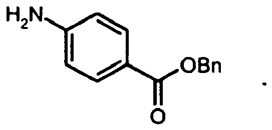

15.01 g (89.9 mmol) 4-Nitrobenzoesäure werden in 500 mL MeCN suspendiert und nachfolgend mit 15.03 g (108.7 mmol, 1.2 eq) Kaliumcarbonat versetzt. Unter Rühren werden 15.4 g (90.4 mmol, 1.01 eq) Benzylbromid zugetropft und die Reaktionsmischung für 5 h unter Rühren bei 60 °C erwärmt. Es wird mit 750 mL destilliertem Wasser versetzt, 4 mal mit je 250 mL EE extrahiert und nach Vereinigen der organischen Phasen über Natriumsulfat getrocknet. Nach Entfernen aller flüchtigen Bestandteile im Vakuum wird das Rohprodukt nacheinander 2 mal in Toluol suspendiert und im Vakuum eingeengt. Man erhält 20.6 g (80.1 mmol) 4-Nitrobenzoesäurebenzylester, welcher in der nächsten Stufe ohne weitere Reinigung eingesetzt wird.
20.6 g des 4-Nitrobenzoesäurebenzylesters werden in 350 mL Dioxan gelöst und diese Lösung mit 6.9 g (49.9 mmol, 0.61 eq) Raney-Nickel versetzt. Unter Rühren wird bei 5 bar H₂-Druck 16 h hydriert. Der Katalysator wird abfiltriert, im Vakuum alle flüchtigen Bestandteile entfernt. Man erhält 17 g 4-Aminobenzoesäurebenzylester.

### A-2a) 4-(4-Chlor-5-trifluormethyl-pyrimidin-2-ylamino)-benzoesäurebenzylester

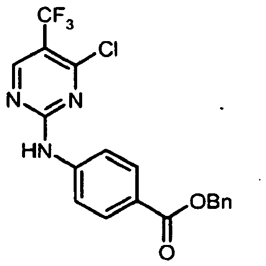

10 g (44 mmol) 4-Aminobenzoesäurebenzylester werden in 200 mL DMA gelöst, 8 mL Hünig-Base (0.97 eq) zugegeben und zu dieser Lösung 10.4 g (48.21 mmol) 2,4-Dichlor-5-trifluormethylpyrimidin, gelöst in 50 mL DMA, bei RT zugetropft. Die Reaktionsmischung wird bei 60 °C über Nacht gerührt, danach mit 300 mL DCM versetzt und mit Wasser (3 mal 300 mL) ausgeschüttelt. Die organische Phase wird getrocknet und das Lösungsmittel im Vakuum entfernt. Das Rohprodukt wird mit 100 mL MeOH versetzt, digeriert und 2 h stehen gelassen. Anschließend wird 10 min gerührt, der Niederschlag abfiltriert und mit MeOH gewaschen. Abschließend wird das Rohprodukt nochmals in MeOH suspendiert, abfiltriert, mit wenig MeOH gewaschen und im Vakuumtrockenschrank bei 60 °C getrocknet. Man erhält 8.5 g **A-2a.**
R_{f}= 0.71 (Kieselgel, cHex:EE 1:2)
MS-ESI⁺: 408 (M+H)⁺

### A-3a) 4-[4-((1R,2S)-2-Carboxy-cyclopentylamino)-5-trifluormethyl-pyrimidin-2-ylaminol-benzoesäurebenzylester

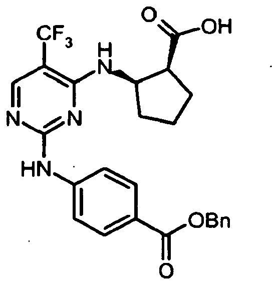

2.05 g (5 mmol, 1 eq) **A-2a** und 1 g (1S,2R)-2-Amino-1-cyclopentancarbonsäure Hydrochlorid (6 mmol, 1.2 eq) werden in 18 mL EtOH vorgelegt, 7.3 mL (42.5 mmol, 8.5 eq) Hünig-Base zugegeben und 4 h bei 70 °C gerührt. Die Reaktionsmischung wird in 275 mL Wasser eingerührt, vom Ungelösten abfiltriert, das Filtrat mit gesättigter, wässriger KHSO₄-Lösung auf pH 2 gestellt, 5 min gerührt und die entstehende Ausfällung abgesaugt. Das Rohprodukt wird mit Wasser gewaschen, im Vakuum getrocknet und 2.37 g **A-3a** erhalten.
MS-ESI⁺: 501 (M+H)⁺

Die Synthese mit (1R,2S)-2-Amino-1-cyclopentancarbonsäure bzw. (1R*,2S*)-(±)-2-Amino-1-cyctopentancarbonsäure wird analog durchgeführt. Die entsprechenden Produkte tragen die Benennung **A-2b** (chiral, Enantiomer zu **A-2a**) und **A-2c** (racemisch).

### Herstellung von (1S,2R)-2-Aminocyclopentancarbonsäure Hydrochlorid

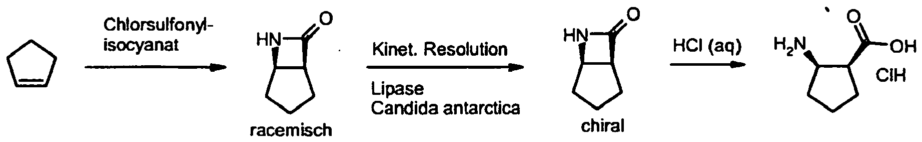

Die Synthese erfolgt gemäß Forro, E. and Fueloep, F. (2003) Lipase-Catalyzed Enantioselective Ring Opening of Unactivated Alicyclic-Fused β-Lactams in an Organic Solvent. Org. Lett. 5, 1209-1212.

### A-4a) 4-[4-((1R,2S)-2-Isopropylearbamoyl-cyclopentylamino)-5-trifluormethyl-pyrimidin-2-ylamino]-benzoesäurebenzylester

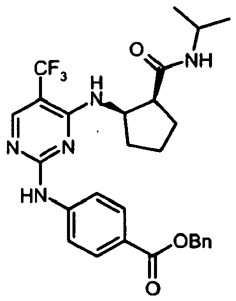

2.59 g (4.9 mmol) **A-3a**, 2.21 g (6.9 mmol, 1.4 eq) TBTU und 4.21 mL (24.6 mmol, 5 eq) Hünig-Base werden in 75 mL DMF gelöst und 20 min bei RT gerührt. Daraufhin werden 0.63 mL (7.38 mmol, 1.5 eq) Isopropylamin zugegeben und über Nacht bei RT gerührt. Es wird über basisches Aluminiumoxid abgesaugt, mit DMF gewaschen und die Mutterlauge in 400 mL Wasser eingerührt, weitere 30 min gerührt und die Ausfällung abgesaugt. Das Rohprodukt wird mit Wasser gewaschen und im Vakuum getrocknet. Zur Reinigung wird mit 50 mL MeCN 30 min bei 5 °C verrührt, abgesaugt, mit etwas kaltem MeCN gewaschen und der Rückstand im Vakuum getrocknet. Man erhält 2.13 g **A-4a**.
R_{f}= 0.53 (Kieselgel, cHx:EE 1:1)
MS-ESI⁺: 542 (M+H)⁺

Durch Verwendung von Ethylamin bzw. Cyclopropylamin werden analog die Verbindungen **A-4d** und **A-4e** hergestellt, die als Edukte in der Synthesesequenz für Beispiele 9 und 10 dienen.

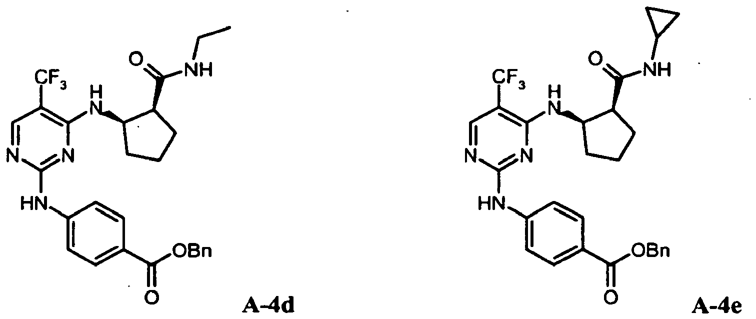

### A-5a) 4-[4-((1R,2S)-2-Isopropylcarbamoyl-cyclopentylamino)-5-trifluormethyl-pyrimidin-2-ylamino]-benzoesäure

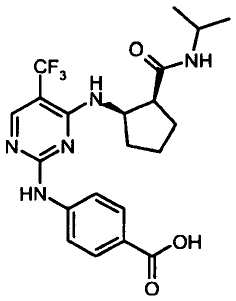

2.13 g (3.9 mmol) **A-4a** werden in 150 mL THF gelöst und 250 mg Palladiumhydroxid/C-Katalysator (20 Gew.% Pd auf Kohle) zugegeben. 16 h wird bei einem H₂-Druck von 6 bar unter Rühren bei RT hydriert. Danach werden 30 mL) MeOH zugegeben, der Katalysator über Kieselgur abfiltriert, mit MeOH nachgewaschen und das Filtrat eingeengt. Der Rückstand wird mit 45 mL EtOH aufgekocht, langsam auf 5 °C abgekühlt, 1 h weiter gerührt und dann abgesaugt und mit kaltem EtOH nachgewaschen. Man erhält 2.46 g **A-5a.**
R_{f}= 0.46 (Kieselgel, CH₂Cl₂:MeOH:AcOH 5:1:0.1)
MS-ESI⁺: 452 (M+H)⁺

Die Synthesen der enantiomeren Verbindung **A-5b** sowie des Racemats **A-5c** erfolgen analog.

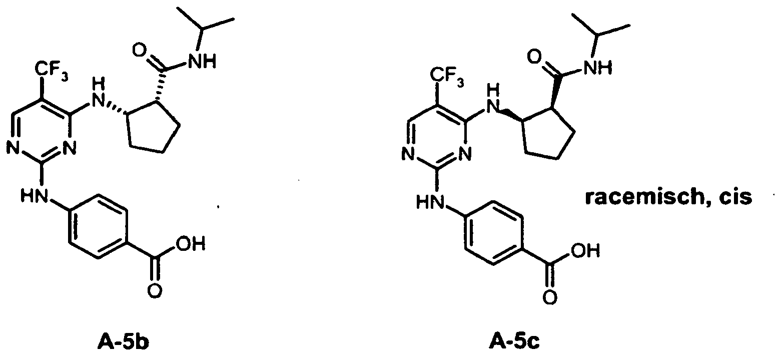

### A-5d) 4-[4-((1R,2S)-2-Isopropylcarbamoyl-cyclobutylamino)-5-trifluormethyl-pyrimidin-2-ylamino]-benzoesäure

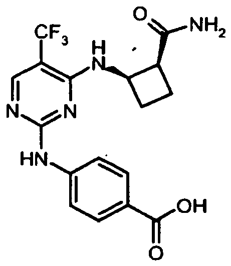

250 mg (2.8 mmol, 1 eq) **B-1a** werden in 1 mL DMA gelöst und 0.74 mL (4.3 mmol, 5.5 eq) Hünig-Base zugegeben. Nun werden 461 mg (2.4 mmol, 3 eq) cis-2-Aminocyclobutancarbonsäureamid zugefügt und die Reaktionsmischung auf 70 °C erwärmt. Nach 1 h ist der Umsatz vollständig. Die Reaktionsmischung wird mit RP-Gel versetzt, die flüchtigen Bestandteile im Vakuum entfernt und das Produkt säulenchromatographisch über eine RP-Phase gereinigt und isoliert (Wasser/MeCN 78/22 (+0.2% HCOOH) auf Wasser/MeCN 58/42 in 12 min). Entsprechende Produktfraktionen werden vereinigt, mittels Gefriertrocknung vom Lösungsmittel befreit und 50 mg **A-5d** erhalten.

### Methyl-(1,2,2,6,6-pentamethyl-piperidin-4-yl)-amin (Edukt Synthese Beispiel 1)

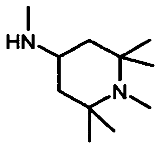

2 g (11.8 mmol, 1 eq) 1,2,2,6,6-Pentamethyl-4-piperidon werden in 10 mL THF gelöst, 3.99 g (59.1 mmol, 5 eq) Methylamin Hydrochlorid zugegeben und die Reaktionsmischung 1 h bei RT gerührt. Nachfolgend werden 4.85 g (59.1 mmol, 5 eq) Natriumacetat und 2.78 g (11.82 mmol, 1 eq) Natriumtrisacetoxyborhydrid zugegeben und die Reaktionsmischung bei RT 16 h gerührt. Reaktionskontrolle mittels Dünnschichtchromatographie zeigt vollständigen Umsatz. Es wird vom Ungelösten abfiltriert, das Filtrat mit Kieselgel versetzt und im Vakuum die flüchtigen Bestandteile entfernt. Durch säulenchromatographische Reinigung (Normalphase, Kieselgel, DCM/MeOH/NH₃(aq) 7/3/0.3) erhält man 373 mg des Amins.
R_{f}= 0.47 (Kieselgel, CH₂Cl₂/MeOH/NH₃ 6/4/0.4)

### (1-Ethyl-piperidin-4-yl)-methyl-amin (Edukt Synthese Beispiel 5)

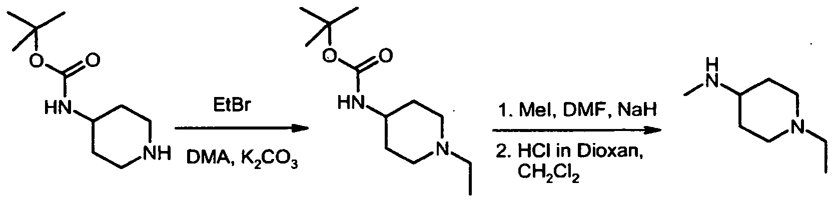

10 g 4-(Boc-Amino)piperidin, 4 mL Ethylbromid und 10 g Kaliumcarbonat werden in 75 mL DMA 2 h bei 120 °C gerührt. Die Reaktionsmischung wird in 500 mL Wasser eingerührt, 3 mal mit je 150 mL EE ausgeschüttelt, die organische Phase über MgSO₄ getrocknet, eingeengt und der Rückstand in 300 mL Diethylether gelöst. Es wird mit 20 mL 4 M Salzsäure in 1,4-Dioxan unter Eiskühlung versetzt, 15 min bei 0° C nachgerührt und der Niederschlag abgesaugt, mit Diethylether nachgewaschen und im Vakuum getrocknet. Man erhält 11.5 g (1-Ethyl-piperidin-4-yl)-carbaminsäure-tert-butylester Hydrochlorid, welches ohne weitere Reinigung eingesetzt wird.
1 g (3.8 mmol, 1 eq) (1-Ethyl-piperidin-4-yl)-carbaminsäure-tert-butylester Hydrochlorid wird in 25 mL DMF gelöst, 378 mg (9.4 mmol, 2.5 eq, 60%ige Dispersion in Öl) Natriumhydrid zugegeben und nach Zugabe von 246 µL (3.95 mmol, 1.1 eq) Methyliodid wird 30 min bei RT gerührt. Das Reaktionsgemisch in 150 mL Wasser eingerührt, mit gesättigter wässriger NaHCO₃-Lösung auf pH 8 eingestellt und 3 mal mit je 50 mL EE extrahiert. Die organische Phase wird über MgSO₄ getrocknet, das Lösungsmittel im Vakuum entfernt und 300 mg (1-Ethyl-piperidin-4-yl)-methyl-carbaminsäure-*tert-*butylester erhalten. Dieser wird in 5 mL DCM vorgelegt, 928 µL Salzsäure (4 M in 1,4-Dioxan, 3 eq) zugegeben und 4 h bei RT gerührt. Nachdem der Umsatz nur ca. 30% beträgt wird 1 mL Trifluoressigsäure zugegeben und 1 h bei RT gerührt. Es wird im Vakuum eingeengt und das rohe (1-Ethyl-piperidin-4-yl)-methyl-amin ohne weitere Reinigung zur Amidkupplung eingesetzt.

### 4-Isopropylamino-piperidin-1-carbonsäure-tert-butylester

### (Edukt Synthese von Beispiel 6)

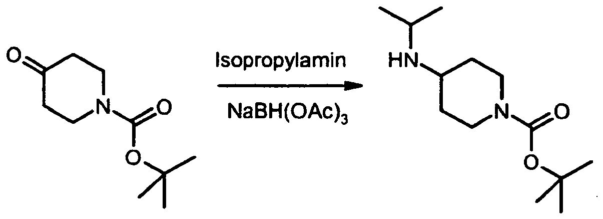

500 mg (2.51 mmol, 1 eq) 1-Boc-4-piperidinon werden in 7 mL 1,2-Dichlorethan vorgelegt, 214 µL (2.51 mmol, 1 eq) Isopropylamin zugegeben und 20 min bei RT gerührt. Nach Zugabe von 145 µL (2.51 mmol, 1 eq) Eisessig werden portionsweise 745 mg (3.5 mmol, 1.4 eq) Natriumtrisacetoxyborhydrid zugegeben und über Nacht bei RT gerührt. Die Reaktionsmischung wird mit 25 mL gesättigter, wässriger NaHCO₃-Lösung versetzt, nach beendeter Gasentwicklung 3 mal mit je 20 mL DCM extrahiert, die vereinigten organischen Phasen über MgSO₄ getrocknet, im Vakuum das Lösungsmittel entfernt und 527 mg des Produktamins erhalten, welches ohne weitere Reinigung eingesetzt wird.

### (±)-Cis-2-aminocyclobutancarbonsäureamid

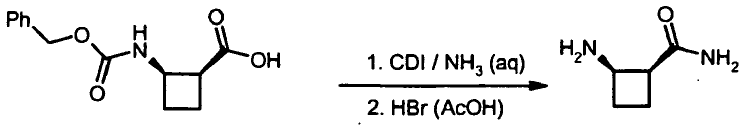

5 g (20.1 mmol, 1 eq) cis-2-(Benzyloxycarbonylamino)-cyclobutancarbonsäure werden in 10 mL THF gelöst und 3.9 g (24.1 mmol, 1.2 eq) Carbonyldiimidazol (CDI) zugegeben. Die klare Lösung wird 40 min bei RT gerührt und nachfolgend 26.6 mL wässrige Ammoniaklösung (1.4 mol, 70 eq, 28-30%ig) zugesetzt. 30 min nach Zugabe wird die Reaktionsmischung auf 500 mL Wasser gegossen und 3 mal mit je 150 mL EE extrahiert. Nach Vereinen der organischen Phasen, Trocknen über MgSO₄ und Entfernen aller flüchtigen Bestandteile im Vakuum erhält man 4.05 g des N-Z-Cis-2-aminocyclobutancarbonsäureamids.
MS-ESI⁺: 249 (M+H)⁺

Zur Entfernung der Schutzgruppe werden 2 g (8.06 mmol, 1 eq) des Produkts bei 0 °C in einer Lösung von 100 mL Bromwasserstoff in Eisessig (33%) suspendiert und 2 h bei 0 °C gerührt. Die Lösung wir in ca. 500 mL Diethylether eingerührt, der Niederschlag abgesaugt und 2 h in Diethylether verrührt. Der Niederschlag wird mit THF gewaschen und 1.27 g Cis-2-aminocyclobutancarbonsäureamids als Hydrobromid erhalten. MS-ESI⁺: 115 (M+H)⁺

### (±)-Cis-2-aminocyclobutancarbonsäureisopropylamid

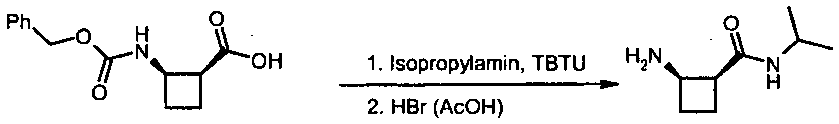

1 g (4 mmol, 1 eq) cis-2-(Benzyloxycarbonylamino)-cyclobutancarbonsäure werden in 2 mL THF gelöst und 3.22 g (10 mmol, 2.5 eq) TBTU sowie 3.43 mL (20 mmol, 5 eq) Hünig-Base zugegeben. Die Suspension wird 30 min bei RT gerührt und dann 513 µL (6.02 mmol, 1.5 eq) Isopropylamin zugetropft. Nach 16 h Rühren bei RT ist der Umsatz vollständig und die Reaktionsmischung wird in 200 mL Wasser eingerührt. Es wird 3 mal mit je 50 mL EE extrahiert. Nach Vereinen der organischen Phasen, Trocknen über MgSO₄ und Entfernen aller flüchtigen Bestandteile im Vakuum erhält man 1.1 g des *N-Z*-Cis-2-aminocyclobutancarbonsäureisopropylamids.
R_{f} = 0.57 (Kieselgel, CH₂Cl₂/MeONH₃ 9/1/0.1)
MS-ESI⁺: 291 (M+H)⁺

Zum Entfernen der Schutzgruppe werden 1.1 g (3.79 mmol, 1 eq) des Produkts bei 0 °C in einer Lösung von 100 mL Bromwasserstoff in Eisessig (33%) suspendiert und 2 h bei 0 °C gerührt. Die Lösung wir in ca. 250 mL Diethylether eingerührt, der Niederschlag abgesaugt und 2 h in Diethylether verrührt. Nach Waschen des Niederschlags mit THF werden 644 mg Cis-2-aminocyclobutancarbonsäureisopropylamids als Hydrobromid erhalten. MS-ESI⁺: 157 (M+H)⁺

### B-1a) 4-(4-Chlor-5-trifluormethyl-pyrimidin-2-ylamino)-benzoesäure

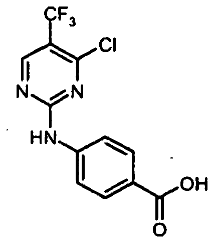

2 g (4.91 mmol) **A-2a** werden in 88 mL Dioxan gelöst, 220 mg Palladiumhydroxid (1.57 mmol, 0.32 eq) zugegeben und 16 h bei 3 bar H₂-Druck und RT gerührt. Die Reaktionsmischung wird über Celite® filtriert, mit THF nachgewaschen, das Filtrat im Vakuum vom Lösungsmittel befreit und 1.31 g **B-1a** erhalten, die ohne weitere Reinigung eingesetzt werden.
MS-ESI+: 318 (M+H)⁺

### B-2a) 4-(4-Chlor-5-trifluormethyl-pyrimidin-2-ylamino)-benzoylchlorid

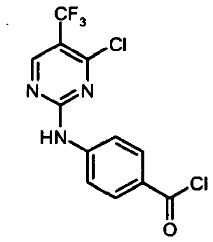

1.31 g (4.13 mmol) **B-1a** werden in 100 mL Toluol suspendiert, 360 µL (4.96 mmol, 1.2 eq) Thionylchlorid unter Rühren vorsichtig zugegeben und die Lösung 1 h unter Rückfluss erwärmt. Alle flüchtigen Bestandteile werden nach Abkühlen auf RT im Vakuum entfernt und der Rückstand **B-2a** ohne weitere Reinigung weiter umgesetzt.

### B-3a) 4-(4-Chlor-5-trifluormethyl-pyrimidin-2-ylamino)-N-methyl-N-(1-methyl-piperidin-4-yl)-benzamid

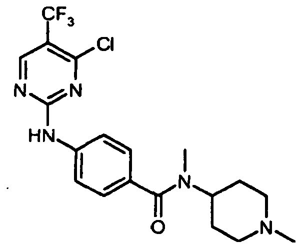

1.36 g (4.05 mmol) **B-2a** werden in 10 mL THF gelöst und mit 1.04 mL (6.1 mmol, 1.5 eq) Hünig-Base versetzt. Nach Zugabe von 589 µL (4.1 mmol, 1 eq) 1-Methyl-4-(methylamino)-piperidin wird die Lösung 1 h bei RT gerührt. Das Reaktionsgemisch wird in ca. 100 mL destilliertes Wasser gegossen, 30 min gerührt und die wässrige Phase 3 mal mit je 100 mL EE extrahiert. Nach Trocknen der organischen Phase über MgSO₄, Filtration und Entfernen der flüchtigen Bestandteile im Vakuum werden 1.64 g **B-3a** erhalten.
R_{f}= 0.30 (Kieselgel, CH₂Cl₂/MeOH/NH₃ 5/1/0.1)
MS-ESI+: 428 (M+H)⁺

### B-4c) (±)-(1S*,2R*)-2-(2-{4-[Methyl-(1-methyl-piperidin-4-yl)-carbamoyl]-phenylamino}-5-trifluormethyl-pyrimidin-4-ylamino)-cyclopentancarbonsäure

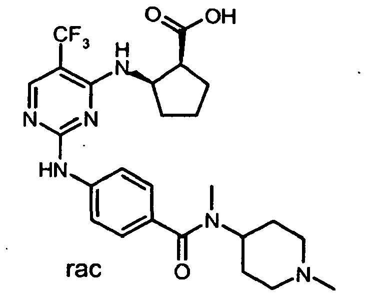

1 g (2.34 mmol) **B-3a** werden in 2.9 mL DMA gelöst und anschließend 1.2 mL (7.01 mmol, 3 eq) Hünig-Base zugegeben. Nach Zugabe von 465 mg (2.81 mmol, 1.2 eq) cis-2-Amino-1-cyclopentancarbonsäure (racemisch) wird ca. 30 min bei 120 °C gerührt.

Die Reaktionsmischung wird mit RP-Gel versetzt, die flüchtigen Bestandteile im Vakuum entfernt, das Produkt säulenchromatographisch über eine RP-Phase gereinigt und isoliert (Wasser/MeCN 85/15 (+0.2% HCOOH) auf Wasser/MeCN 72/28 in 10 min). Entsprechende Produktfraktionen werden vereinigt, mittels Gefriertrocknung vom Lösungsmittel befreit und 578 mg **B-4c** erhalten.
MS-ESI⁺: 521 (M+H)⁺

Die Herstellung von **B-4a** erfolgt analog unter Verwendung von EtOH als Lösungsmittel und (1S,2R)-2-Aminocyclopentancarbonsäure als Ausgangsmaterial.

### B-4a) (1S,2R)-2-(2-{4-[Methyl-(1-methyl-piperidin-4-yl)-carbamoyl]-phenylamino}-5-trifluormethyl-pyrimidin-4-ylamino)-cyclopentancarbonsäure

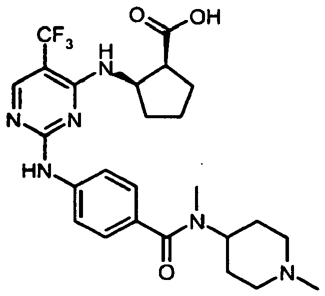

MS-ESI⁺ :521 (M+H)⁺

### B-4d) (1S,3R)-3-(2-{4-[Methyl-(1-methyl-piperidin-4-yl)-carbamoyl]-phenylamino}-5-trifluoromethyl-pyrimidin-4-ylamino)-cyclopentancarbonsäure

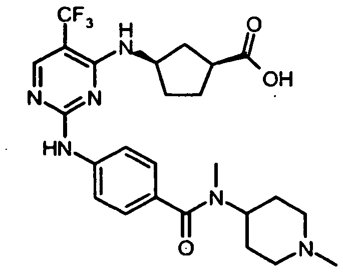

200 mg (0.46 mmol, 1 eq) **B-3a** werden in 750 µL DMA gelöst und 160 µL (0.92 mmol, 2 eq) Hünig-Base zugegeben. Nun werden 72 mg (0.56 mmol, 1.2 eq) (1S,3R)-3-Aminocyclopentancarbonsäure zugegeben und die Reaktionsmischung unter Rühren 40 min lang auf 120 °C erwärmt. Die Reaktionsmischung wird mit RP-Gel versetzt, die flüchtigen Bestandteile im Vakuum entfernt, das Produkt säulenchromatographisch über eine RP-Phase gereinigt und isoliert (von Wasser/MeCN 85/15 (+0.2% HCOOH) auf Wasser/MeCN 76/24 in 20 min). Entsprechende Produktfraktionen werden vereinigt, mittels Gefriertrocknung vom Lösungsmittel befreit und 150 mg **B-4d** erhalten.

In analoger Weise und unter Verwendung von (1R,3S)-3-Aminocyclopentancarbonsäure kann die enantiomere Verbindung **B-4e** hergestellt werden.

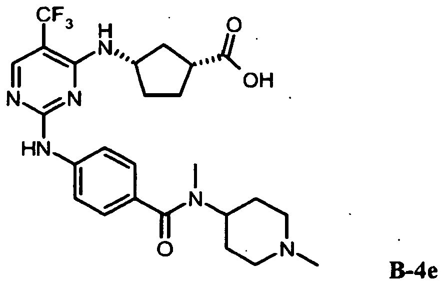

### 3-Amino-bicyclo[2.2.2]octan-2-carbonsäure

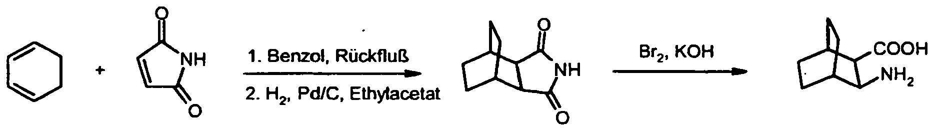

9.7 g (99.9 mmol, 1 eq) Maleinimid werden in 100 mL Benzol suspendiert und nachfolgend 8 g (99.9 mmol, 1 eq) Cyclohexadien, suspendiert in 20 mL Benzol, bei 5 °C langsam zugegeben. Die Reaktionsmischung wird langsam auf Rückflusstemperatur erwärmt und 3 h unter Rückfluss gerührt. Nach Abkühlen auf 0 °C wird der Niederschlag abfiltriert und im Vakuum getrocknet. Man erhält 14.7 g des Cycloadditionsproduktes. MS-ESI⁺: 178 (M+H)⁺
12.2 g (68.6 mmol, 1 eq) des Cycloadditionsproduktes werden in 240 mL EE gelöst und 1.2 g Palladium auf Aktivkohle (20% w/w Pd, 11.3 mmol, 0.16 eq) zugegeben. Die Reaktionsmischung wird unter Wasserstoffatmosphäre (5 bar) bei RT gerührt bis kein Wasserstoff mehr aufgenommen wird (20 h). Nun wird ein Gemisch aus MeOH und DCM (1/1, 50 mL) zugegeben, der Katalysator abfiltriert und die flüchtigen Bestandteile im Vakuum entfernt. Man erhält 12.1 g Produktes, welches ohne weitere Reinigung umgesetzt wird.
Bei 0 °C werden 3.7 mL (72.2 mmol, 1 eq) Brom zu einer Lösung von 36.6 g (652.2 mmol, 9 eq) Kaliumhydroxid in Wasser getropft. Die Lösung wird unter weiterer Kühlung auf 0 °C mit 13 g (72.7 mmol, 1 eq) des Hydrierungsproduktes versetzt. Nach Aufwärmen auf RT wird 2.5 h auf 60 °C erwärmt. Die Reaktionsmischung wird auf RT abgekühlt, mit wässriger Salzsäure angesäuert und im Vakuum alle flüchtigen Bestandteile entfernt. Der Rückstand wird mit kaltem Wasser zerrieben, der Niederschlag abfiltriert, das Filtrat zur Trockene eingeengt, mit heißem 1-Butanol ausgekocht, abermals vom Ungelösten abfiltriert und mit heißem 1-Butanol nachgewaschen. Das Filtrat wird im Vakuum eingeengt und Umkristallisieren aus EtOH ergibt 2.4 g der Titelverbindung.
MS-ESI⁺: 170 (M+H)⁺

### B-4f) 3-(2-{4-[Methyl-(1-methyl-piperidin-4-yl)-carbamoyl]-phenylamino}-5-trifluormethyl-pyrimidin-4-ylamino)-bicyclo[2.2.2]octan-2-carbonsäure

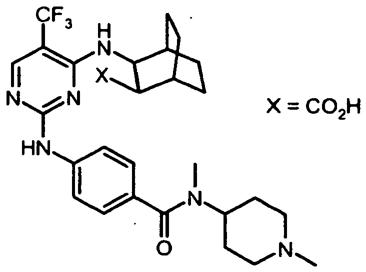

Die Herstellung von **B-4f** erfolgt analog zu **B-4a** mit EtOH als Lösungsmittel durch Reaktion von 3-Amino-bicyclo[2.2.2]octan-2-carbonsäure mit **B-3a.**
MS-ESI⁺: 561 (M+H)⁺

### B-4g) 3-(2-{4-[Methyl-(1-methyl-piperidin-4-yl)-carbamoyl]-phenylamino}-5-trifluormethyl-pyrimidin-4-ylamino)-cyclobutancarbonsäure

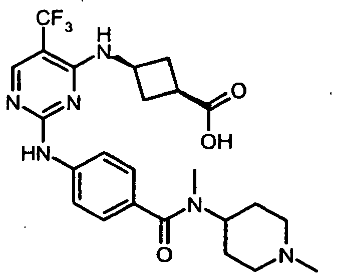

Die Herstellung von **B-4g** erfolgt analog zu **B-4a** mit EtOH als Lösungsmittel durch Reaktion von cis-3-Amino-cyclobutancarbonsäure mit **B-3a.**

### B-4h) (±)-(1S*,2R*)-2-(2-{4-[Methyl-(1-methyl-piperidin-4-yl)-carbamoyl]-phenylamino}-5-trifluormethyl-pyrimidin-4-ylamino)-cyclohexancarbonsäure

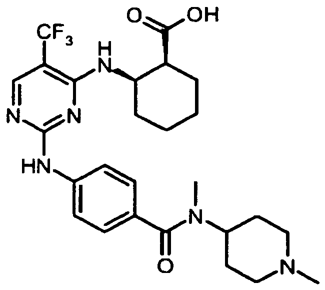

Die Herstellung von **B-4h** erfolgt analog zu **B-4a** mit 1-Butanol als Lösungsmittel durch Reaktion von (±)-cis-2-Amino-cyclohexancarbonsäure mit **B-3a.**

### B-4i) (±)-(1S*,6R*)-6-(2-{4-[Methyl-(1-methyl-piperidin-4-yl)-carbamoyl]-phenylamino}-5-trifluoromethyl-pyrimidin-4-ylamino)-cyclohex-3-encarbonsäure

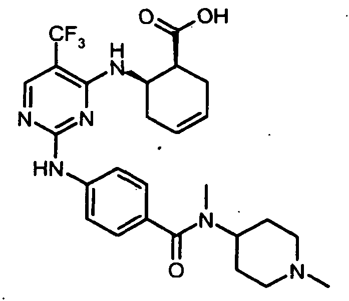

Die Herstellung von **B-4i** erfolgt analog zu **B-4a** mit 1-Butanol als Lösungsmittel durch Reaktion von (±)-cis-2-Amino-cyclohex-3-encarbonsäure (Fa. BioBlocks) mit **B-3a.**

### cis-(±)-2-Amino-cyclopentancarbonsäure-isopropylamid

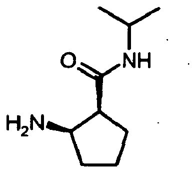

55 mg (0.43 mmol) cis-(±)-2-Amino-cyclopentancarbonsäure werden in 900 µL (25 eq) Isopropylamin suspendiert und 205 mg (0.064 mmol, 1.5 eq) TBTU und 550 µL DMF zugegeben. Es wird 16 h gerührt und die Reaktionsmischung wird in DCM/MeOH/NH₃(aq) 9/1/0.1 aufgenommen und mit 7 mL Kieselgel versetzt. Nach Entfernen aller flüchtigen Bestandteile im Vakuum wird chromatographiert (Kieselgel DCM/MeOWNH₃ 9/1/0.1). Man erhält 63 mg der Titelverbindung.
R_{f}= 0.33 (Kieselgel, DCM/MeOH/NH₃ 85/15/1.5)

In Analogie zu dieser Vorschrift wird unter Verwendung von (1 S,2R)-2-Aminocyclopentancarbonsäure die chirale Verbindung (1S,2R)-2-Aminocyclopentancarbonsäureisopropylamid hergestellt. Weiters werden auf diese Weise eine Vielzahl von Amiden ausgehend von 2-Aminocyclopentancarbonsäure (racemisch oder chiral) hergestellt.

### Allgemeine Vorschrift zur Synthese von Verbindungen des Typs C-1

Ein entsprechend R²-substituiertes 2,4-Dichlorpyrimidin **A-1** (käuflich bzw. hergestellt durch Chlorierung des entsprechenden Uracils wie für **A-1a** beispielhaft beschrieben) wird in THF (Dioxan, DMA, NMP, Aceton oder DCM) gelöst (ca. 2 - 5 mL/mmol), 1 - 1.6 eq Hünig-Base (Triethylamin, Kaliumcarbonat oder eine andere geeignete Base) zugegeben und die Reaktionsmischung temperiert (-78 °C bei sehr reaktiven Pyrimidinen, RT oder erhöhte Temperatur bei eher reaktionsarmen Pyrimidinen). Nun werden ca. 0.75 - 1 eq des Amins, gelöst in dem entsprechenden Lösungsmittel (siehe oben), zugegeben und die Reaktionsmischung über einen bestimmten Zeitraum bei der entsprechenden Temperatur gerührt bzw. aufgetaut oder erwärmt, abhängig von der Reaktivität des verwendeten Pyrimidins. Nach beendeter Reaktion (Reaktionskontrolle mittels HPLC oder DC) wird die Reaktionsmischung mit Kieselgel versetzt und alle flüchtigen Bestandteile im Vakuum entfernt. Säulenchromatographische Reinigung liefert die gewünschten Substitutionsprodukte. In Abhängigkeit vom Rest R² des Pyrimidins entstehen beide möglichen Regioisomeren in verschiedenen Verhältnissen. Sie sind in der Regel chromatographisch separierbar.

### C-1c) (±)-(1S*,2R*)-2-(2-Chlor-5-trifluormethyl-pyrimidin-4-ylamino)-cyclopentancarbonsäureisopropylamid

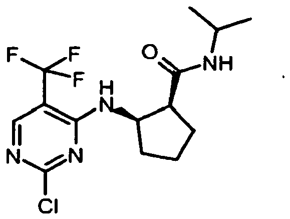

2 g (9.2 mmol) A-1a und 1.8 mL (11.2 mmol, 1.2 eq) Hünig-Base werden in 60 mL THF gelöst, auf -78 C° gekühlt, danach wird cis-(±)-2-Aminocyclopentancarbonsäureisopropylamid, gelöst in 60 mL THF, bei -78 °C langsam zugetropft. Man lässt die Reaktionsmischung über Nacht unter Rühren auf RT auftauen. Dann wird mit 40 mL Kieselgel versetzt und alle flüchtigen Bestandteile im Vakuum entfernt. Säulenchromatographisch werden beide regioisomeren Produkte getrennt, wobei das gewünschte Regioisomer das zuerst eluierende Produkt ist (Kieselgel, cHex/EE von 85/15 auf 80/20 innerhalb von 30 min). Es werden 590 mg **C-1c** isoliert sowie 690 mg des regioisomeren Produktes **C-1c'**.
**R_{f}(C-1c)** = 0.21 (Kieselgel, cHex/EE 3/1), [**R_{f}**(**C-1c'**) = 0.10]
MS-ESI+: 351 (M+H)⁺
UVₘₐₓ = 246 nm

### C-1a (1S,2R)-2-(2-Chlor-5-trifluormethyl-pyrimidin-4-ylamino)-cyclopentancarbonsäureisopropylamid

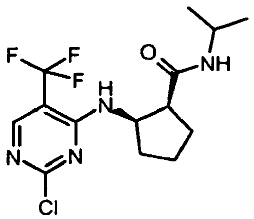

Die Herstellung der chiralen Verbindung **C-1a** erfolgt ausgehend von (1S,2R)-2-Aminocyclopentancarbonsäureisopropylamid in Analogie.

### C-1d (1S,2R)-2-(2-Chlor-5-methyl-pyrimidin-4-ylamino)-cyclopentancarbonsäureisopropylamid

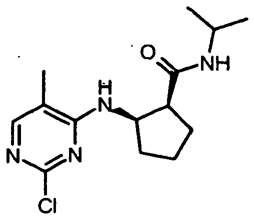

1 g (6.1 mmol, 1 eq) 5-Methyl-2,4-Dichlorpyrimidin (analog zu **A-1a** hergestellt) werden in 3 mL DMA gelöst und nachfolgend 5.3 mL (30.5 mmol, 5 eq) Hünig-Base zugetropft.
Es werden 1.03 g (6.1 mmol, 1 eq) (1S, 2R)-2-Aminocyclopentancarbonsäureisopropylamid zugegeben und die Reaktionsmischung 1 h bei 70 °C gerührt. HPLC Kontrolle zeigt vollständigen Umsatz und lediglich die Bildung eines Regioisomers. Die Reaktionsmischung wird mit RP-Gel versetzt, die flüchtigen Bestandteile im Vakuum entfernt, das Produkt säulenchromatographisch über eine RP-Phase gereinigt und isoliert (von Wasser/MeCN (+ je 0.2% HCOOH) von 82/18 auf 60/40 in 15 min). Entsprechende Produktfraktionen werden vereinigt, mittels Gefriertrocknung vom Lösungsmittel befreit und 956 mg **C-1d** erhalten.
R_{f} = 0.15 (Kieselgel, cHex:EE 1:1)
MS-ESI+: 297/299 (M+H)⁺

### C-1e (1S,2R)-2-(2-Chlor-5-brom-pyrimidin-4-ylamino)-cyclopentancarbonsäureisopropylamid

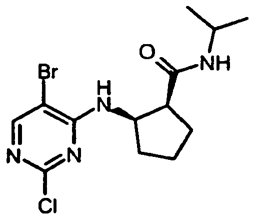

Die Synthese von **C-1e** erfolgt analog zur Herstellung von **C-1d** unter Verwendung von 1-Butanol (0.7 M) als Lösungsmittel bei 70 °C und Rühren über 2 h. Die Isolierung erfolgt durch Einengen im Vakuum und Waschen des Niederschlags mit MeOH.
MS-ESI+: 361/363 (M+H)⁺

### C-1f (1S,2R)-2-(2,5-Dichlor-pyrimidin-4-ylamino)-cyclopentancarbonsäureisopropylamid

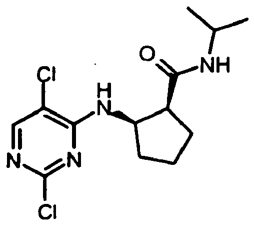

Die Synthese von **C-1f** erfolgt analog zur Herstellung von **C-1d** unter Verwendung von DCM (0.3 M) als Lösungsmittel. Die Ausgangsverbindungen werden bei 0 °C zusammen gegeben und die Reaktionsmischung 6 h bei RT gerührt.
R_{f} = 0.63 (Kieselgel, EE)

### C-2a 4-[4-((1R,2S)-2-Isopropylcarbamoyl-cyclopentylamino)-5-trifluormethyl-pyrimidin-2-ylamino]-2-ethoxy-benzoesäure

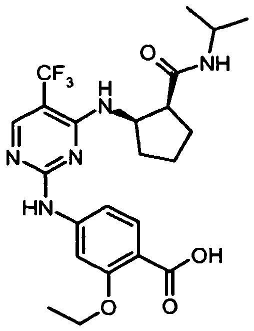

120 mg (0.34 mmol, 1 eq) **C-1a** werden mit 102 mg (0.56 mmol, 1.7 eq) 2-Ethoxy-4-aminobenzoesäure in 500 µL DMA (wasserfrei) suspendiert, mit 221 µL dioxanischer HCl (0.89 mmol, 2.6 eq, 4 M) versetzt und bei 70 °C 2.5 h geschüttelt. Das Reaktionsgemisch wird in 10 mL Wasser gegossen, mit konzentrierter wässriger Salzsäure angesäuert und der Niederschlag abfiltiert. Nach Trocknen im Vakuum werden 143 mg **C-2a** erhalten und ohne weitere Reinigung eingesetzt.

In Analogie erfolgt die Umsetzung mit anderen 2-Alkoxy-substituierten Benzoesäuren (Synthese der Ausgangsverbindungen für die Herstellung der Beispiele **131, 133** und **134).** Für die Herstellung von **Beispiel 135** wird 2-Chlor-4-amino-benzoesäure verwendet.

### 2-Ethoxy-4-amino-benzoesäure

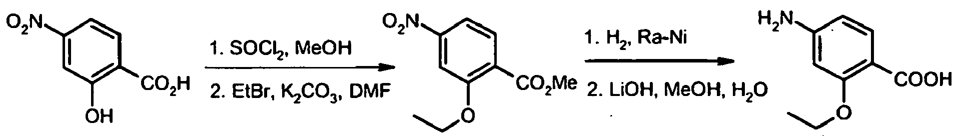

4.05 g (21.7 mmol, 1 eq) 2-Hydroxy-4-nitro-benzoesäure werden in 40 mL MeOH gelöst und langsam 1.8 mL (24.8 mmol, 1.14 eq) Thionylchlorid zugetropft. Es wird 2 h bei 50 °C und weitere 2 h unter Rückfluss erwärmt und gerührt. Die flüchtigen Bestandteile werden nach dem Abkühlen im Vakuum entfernt und 4.36 g des rohen 2-Hydroxy-4-nitrobenzoesäuremethylesters erhalten, welche ohne weitere Reinigung umgesetzt werden. 1 g (5.1 mmol, 1 eq) des Methylesters werden in 25 mL DMF gelöst, 2.1 g (15.3 mmol, 3 eq) Kaliumcarbonat zugegeben und nachfolgend 1.4 mL (18.8 mmol, 3.7 eq) Bromethan zugetropft. Die Reaktionsmischung wird 16 h bei RT gerührt, dann in 100 mL Wasser gegossen und der pH Wert mit konzentrierter wässriger Salzsäure auf pH 3 eingestellt. Es wird 2 mal mit je 100 mL EE extrahiert, über MgSO₄ getrocknet und nach Entfernen aller flüchtigen Bestandteile im Vakuum werden 1.28 g 2-Ethoxy-4-nitro-benzoesäuremethylester erhalten. 1.28 g (5.06 mmol, 1 eq) der Nitroverbindung werden in 50 mL MeOH gelöst, eine Spatelspitze Raney-Nickel wird zugegeben und die Reaktionsmischung im Autoklaven bei einem Wasserstoffdruck von 4 bar 16 h gerührt. Der Katalysator wird über Celite® abfiltriert und das Filtrat im Vakuum von allen flüchtigen Bestandteilen befreit. Man erhält 0.99 g des Anilins [MS-ESI+: 351 (M+H)⁺].
Diese Menge wird in 5 mL MeOH gelöst und eine Lösung von 0.35 g (14.6 mmol, 2.9 eq) Lithiumhydroxid in 10 mL Wasser zugegeben. Nach 16 h Rühren bei RT wird die Reaktionsmischung im Vakuum konzentriert, der Rückstand in 10 mL Wasser aufgenommen und mit wässriger Salzsäure angesäuert. Der Niederschlag wird abfiltriert, mit Wasser gewaschen und im Vakuum getrocknet. Man erhält 392 mg 2-Ethoxy-4-aminobenzoesäure.

### C-2d 4-[4-((1R,2S)-2-Isopropylcarbamoyl-cyclopentylamino)-5-methyl-pyrimidin-2-yl-amino]-benzoesäure

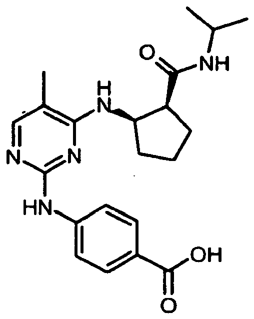

956 mg (3.2 mmol, 1 eq) **C-1d** werden in 5.4 mL 1-Butanol gelöst und diese Lösung mit 446 mg (3.2 mmol, 1 eq) 4-Amino-benzoesäure versetzt. Nach Zugabe von 105 µL (0.42 mmol, 0.13 eq) dioxanischer Salzsäure (4 M) wird 2 h unter Rückfluss und Rühren erhitzt. Nach dem Abkühlen wird der entstandene Niederschlag abfiltriert und mit 2 mL kaltem 1-Butanol gewaschen. Nach Trocknen im Vakuum werden 1.15 g **C-2d** erhalten und ohne weitere Reinigung eingesetzt.
MS-ESI+: 398 (M+H)⁺

### C-2e 4-[4-((1R,2S)-2-Isopropylcarbamoyl-cyclopentylamino)-5-chlor-pyrimidin-2-yl-amino]-benzoesäure

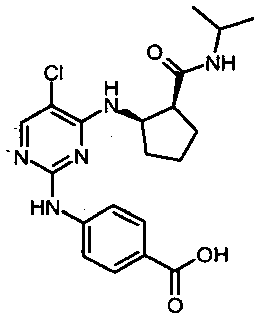

Die Synthese von **C-2e** erfolgt analog der Herstellung von **C-2d.**

### D-1a) (±)-4-[4-((1R*,2S*)-2-Amino-cyclohexylaminol-5-trifluormethyl-pyrimidin-2-ylamino]-N-methyl-N-(1-methyl-piperidin-4-yl)-benzamid

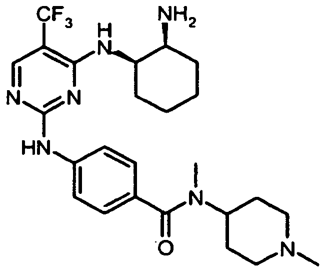

500 mg (1.17 mmol, 1 eq) **B-3a** werden in 5 mL 1-Butanol gelöst und mit 760 µL (4.44 mmol, 3.8 eq) Hünig-Base versetzt. Nun werden 165 µL (1.40 mmol, 1.2 eq) cis-1,2-Diaminocyclohexan zugegeben und die Reaktionsmischung 15 min in der Mikrowelle auf 150 °C erwärmt. Im Vakuum werden alle flüchtigen Bestandteile entfernt und der Rückstand in DCM aufgenommen. Nun wird 2 mal mit verdünnter wässriger Ammoniumchlorid Lösung gewaschen, die organische Phase über MgSO₄ getrocknet, im Vakuum das Lösungsmittel entfernt und 477 mg **D-1a** erhalten.
MS-ESI+: 506 (M+H)⁺

### D-1b) (±)- 4-[4-((1R*,2S*)-2-Amino-cyclopentylamino)-5-trifluormethyl-pyrimidin-2-ylamino]-N-methyl-N-(1-methyl-piperidin-4-yl)-benzamid

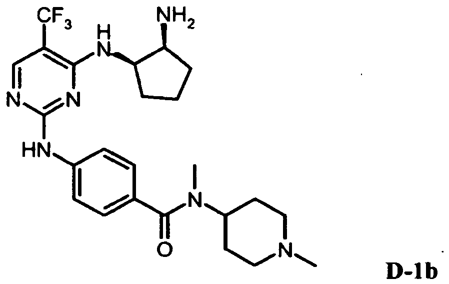

364 mg (0.85 mmol, 1 eq) **B-3a** wird in 1 mL DMA gelöst und anschließend 177 mg (1.02 mmol, 1.2 eq) cis-1,2-Diaminocyclopentan Dihydrochlorid zugegeben. Die Reaktionsmischung wird mit 1.5 mL) (8.76 mmol, 10.3 eq) Hünig-Base versetzt und in der Mikrowelle für 20 min unter Rühren auf 150 °C erwärmt. Nachfolgend werden alle flüchtigen Bestandteile im Vakuum entfernt und das Rohprodukt (418 mg) ohne Reinigung weiter umgesetzt.

### E-1) 2-Methylsulfanyl-1H-pyrimidin-4-on

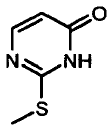

20 g (153 mmol) 2-Thiouracil werden in 250 mL MeOH suspendiert und anschließend 8.7 g (152.9 mmol, 1 eq) Natriummethanolat zugegeben. Die Lösung wird 5 min bei RT gerührt und danach 12.4 mL (198.8 mmol, 1.3 eq) Methyliodid zugetropft. Die Reaktionsmischung wird über Nacht gerührt, danach auf Wasser gegossen und 3 x mit je ca. 150 mL Chloroform extrahiert. Die vereinigten organischen Phasen werden über MgSO₄ getrocknet, das Lösungsmittel im Vakuum entfernt und 16 g **E-1** erhalten.

### E-2) 4-(6-Oxo-1,6-dihydro-pyrimidin-2-ylamino)-benzoesäure

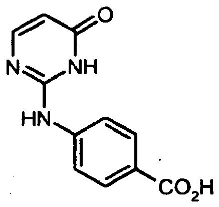

4.1 g (28.8 mmol) E-1 werden in 10 mL Diglyme (Diethylenglycoldimethylether) gelöst und diese Lösung mit 4.79 g (34.6 mmol, 1.2 eq) 4-Aminobenzoesäure versetzt. Die Reaktionsmischung wird für 16 h unter Rückfluss erhitzt. Nach Abkühlen auf RT wird der Niederschlag abgesaugt, mit wenig Diglyme, dann mit Diethylether nachgewaschen und im Vakuum getrocknet. Man erhält 5.27 g **E-2.**
MS-ESI⁺: 232 (M+H)⁺

### E-3) 4-(5-Brom-6-oxo-1,6-dihydro-pyrimidin-2-ylamino)-benzoesäure

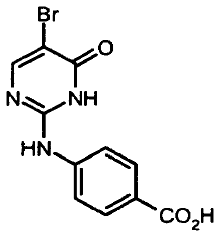

9 g (38.9 mmol) **E-2** werden in 10 mL Essigsäure vorgelegt und dazu eine Lösung von 2.1 mL (40.9 mmol, 1.05 eq) Brom in 50 mL Essigsäure getropft und ca. 1 h bei RT gerührt. Die Reaktionsmischung wird in 800 mL Wasser eingerührt, die Ausfällung abgesaugt und der erhaltene Niederschlag mit Wasser gewaschen und im Vakuum getrocknet. Man erhält 11.5 g **E-3.**
R_{f}= 0.27 (Kieselgel, EE:MeOH 7:3)
MS-ESI⁺: 309/311 (M+H)⁺

### E-5) 4-(4-Chlor-5-brom-pyrimidin-2-ylamino)-benzoylchlorid bzw.

### E-4) 4-(4-Chlor-5-brom-pyrimidin-2-ylamino)-benzoesäure

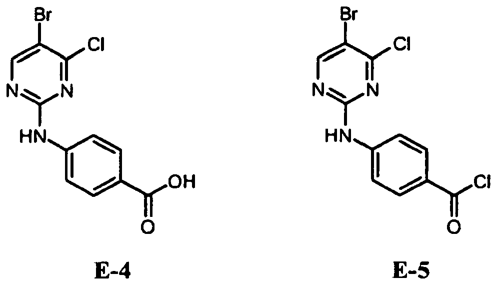

5 g (16.1 mmol) **E-3** werden in 70 mL Phosphoroxychlorid suspendiert und 1 h unter Rühren unter Rückfluss erwärmt. Die Reaktionsmischung wird in 600 mL Wasser/Eis unter starkem Rühren eingetropft, weitere 30 min gerührt und die rohe Säure **E-4** abfiltriert Diese wird im Vakuum getrocknet und ohne Reinigung weiter eingesetzt.
Zur Herstellung des Säurechlorids werden 2.7 g (8.2 mmol) der rohen Säure in 20 mL) Toluol gelöst und 715 µL (9.9 mmol, 1.2 eq) Thionylchlorid zugegeben. Die Reaktionsmischung wird 1 h bei Rückflusstemperatur gerührt und dann im Vakuum eingeengt. Nach Trocknung im Vakuum erhält man 2.9 g **E-5.**

### E-6) 4-(5-Bromo-4-chloro-pyrimidin-2-ylamino)-N-methyl-N-(1-methyl-piperidin-4-yl)-benzamid

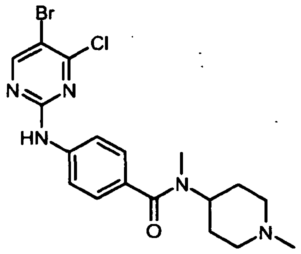

500 mg (1.44 mmol, 1 eq) **E-5** werden in 20 mL THF gelöst und mit 370 µL (2.16 mmol, 1.5 eq) Hünig-Base versetzt, gefolgt von 209 µL (1.44 mmol, 1 eq) 1-Methyl-4-(methylamino)-piperidin. Die Reaktionsmischung wird 16 h bei RT gerührt und danach in 250 mL Wasser eingegossen. Es wird 4 mal mit je 100 mL EE extrahiert. Die vereinigten organischen Phasen werden über MgSO₄ getrocknet und das Lösungsmittel im Vakuum entfernt. Man erhält 418 mg **E-6.**
R_{f}= 0.64 (Kieselgel, DCM:MeOH:NH₃ 5:1:0.1)
MS-ESI⁺: 440/442 (M+H)⁺

### E-7a) (1S,2R)-2-(5-Bromo-2-{4-[methyl-(1-methyl-piperidin-4-yl)-carbamoyl]-phenylamino}-pyrimidin-4-ylamino)-cyclopentancarbonsäure

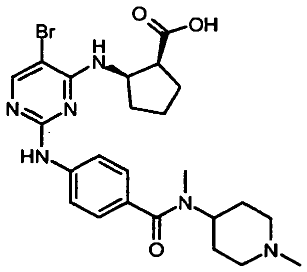

1.91 g (4.35 mmol, 1 eq) E-6 werden in 5 mL DMA suspendiert und mit 1.5 mL (8.7 mmol, 2 eq) Hünig-Base versetzt. Zu der Lösung werden 865 mg (5.22 mmol, 1.2 eq) (1S, 2R)-2-Aminocyclopentancarbonsäure gegeben und die Reaktionsmischung 120 min bei 120°C (CEM Mikrowelle, 100 W) gerührt. Das Reaktionsgemisch wird eingeengt, in ca. 200 mL Wasser verrührt und 3 mal mit je 100 mL EE extrahiert. Die vereinigten organischen Phasen werden über MgSO₄ getrocknet und im Vakuum eingeengt. Anschließend wird mit RP-Gel versetzt, die flüchtigen Bestandteile im Vakuum entfernt, das Produkt säulenchromatographisch über eine RP-Phase gereinigt und isoliert (Wasser/MeCN (+ je 0.2% HCOOH) von 92/8 auf 79/21 in 20 min). Entsprechende Produkt fraktionen werden vereinigt und mittels Gefriertrocknung vom Lösungsmittel befreit. Man erhält 1.26 g **E-7a.**
MS-ESI⁺: 531/533 (M+H)⁺

### E-7b) (±)-(1S*,2R*)-2-(5-Bromo-2-{4-[methyl-(1-methyl-piperidin-4-yl)-carbamoyl]-phenylamino}-pyrimidin-4-ylamino)-cyclopentancarbonsäure

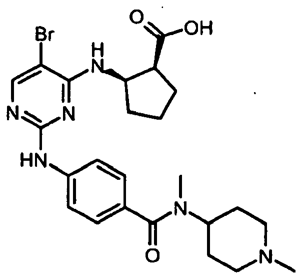

**E-7b** wird analog zu **E-7a** unter Verwendung von racemischer cis-2-Aminocyclopentancarbonsäure hergestellt.

### Beispiel 1

### 4-[4-((1R,2S)-2-Isopropylcarbamoyl-cyclopentylamino)-5-trifluormethyl-pyrimidin-2-ylamino]-N-methyl-N-(1,2,2,6,6-pentamethyl-piperidin-4-yl)-benzamid

100 mg (0.22 mmol) **A-5a** werden in 2 mL DMF gelöst, 190 µL (1.11 mmol, 5 eq) Hünig-Base und 112 mg (0.35 5 mmol, 1.6 eq) TBTU zugegeben. Die Reaktionsmischung wird 30 min bei RT gerührt und nachfolgend werden 88 mg (0.38 mmol, 1.7 eq, Gehalt 80%) Methyl-(1,2,2,6,6-pentamethyl-piperidin-4-yl)-amin zugetropft. Es wird 2 Tage bei RT gerührt, die Reaktionsmischung über basisches Aluminiumoxid filtriert, anschließend mit RP-Gel versetzt, die flüchtigen Bestandteile im Vakuum entfernt und das Produkt säulenchromatographisch über eine RP-Phase gereinigt und isoliert (Wasser/MeCN (+ je 0.2% HCOOH) von 80/20 auf 55/45 in 15 min). Entsprechende Produktfraktionen werden vereinigt, mit konzentrierter Salzsäure versetzt, mittels Gefriertrocknung vom Lösungsmittel befreit und 61 mg der Verbindung **1** als Hydrochlorid erhalten.

In analoger Weise werden die **Beispiele 3, 5** und **8** hergestellt. Im Fall der racemischen **Beispiele 2** und **7** wird **A-5c** und im Fall des **Beispiels 4** wird **A-5b** anstelle von **A-5a** verwendet. Die Herstellung der **Beispiele 9-10** erfolgt ebenfalls analog nach dem allgemeinen Syntheseschema A unter Verwendung der durch Hydrogenolyse aus **A-4b** und **A-4c** (analog des für **A-5a** beschriebenen Verfahrens) erhaltenen Benzoesäuren, **Beispiel 84** unter Verwendung von **A-5d.**

### Beispiel 6

### N-Isopropyl-4-[4-((1R,2S)-2-isopropylcarbamoyl-cyclopentylamino)-5-trifluoromethylpyrimidin-2-ylamino]-N-(1-methyl-piperidin-4-yl)-benzamid

Die Synthese erfolgt wie für **Beispiel 1** beschrieben durch eine Amidkupplung ausgehend von **A-5a.** Dabei wird 4-Isopropylamino-piperidin-1-carbonsäure-*tert*-butylester als Aminkomponente verwendet. Das Kupplungsprodukt wird dann wie folgt von der Schutzgruppe befreit und methyliert.
98 mg des Kupplungsproduktes aus **A-5a** und 4-Isopropylamino-piperidin-1-carbonsäuretert-butylester werden in 2 mL DCM und 2 mL Trifluoressigsäure 2 h bei RT gerührt.
10 mL Wasser werden zugegeben und mit Natriumcarbonat auf pH 10 eingestellt. Es wird 3 mal mit je 15mL DCM ausgeschüttelt, die organische Phase über MgSO₄ getrocknet, die flüchtigen Bestandteile im Vakuum entfernt und das rohe Produkt (50 mg) gleich weiter umgesetzt. Dazu werden 50 mg (0.08 mmol, 1 eq) des Produkts in 1 mL DMA vorgelegt, 13 µL Formaldehyd (37% ig in Wasser, 0.16 mmol, 2 eq) zugegeben und 20 min bei RT gerührt. 5 µL Eisessig werden zugetropft, dann portionsweise 92 mg (0.43 mmol, 5 eq) Natriumtrisacetoxyborhydrid zugegeben und über Nacht bei RT gerührt. Es wird mit 20 mL Wasser versetzt, 10 mL gesättigte wässrige NaHCO₃-Lösung langsam zugegeben, 3 mal mit je 10 mL DCM extrahiert und über MgSO₄ getrocknet. Anschließend mit RP-Gel versetzt, die flüchtigen Bestandteile im Vakuum entfernt und das Produkt säulenchromatographisch über eine RP-Phase gereinigt und isoliert (Wasser/MeCN 82/18 (+ je 0.2% HCOOH) auf 60/40 in 15 min). Entsprechende Produktfraktionen werden vereinigt, mit konzentrierter Salzsäure versetzt, mittels Gefriertrocknung vom Lösungsmittel befreit und 7 mg der Verbindung 6 als Hydrochlorid erhalten.

### Beispiel 12

### 4-{4-[(1R*,25*)-2-((R)-2-Hydroxy-1-methyl-ethylcarbamoyl)-cyclopentylamino]-5-trifluormethyl-pyrimidin-2-ylamino}-N-methyl-N-(1-methyl-piperidin-4-yl)-benzamid

80 mg (0.15 mmol, 1 eq) **B-4c** werden in 3 mL THF gelöst und mit 264 µL (1.54 mmol, 10 eq) Hünig-Base versetzt. Zu dieser Lösung werden 69 mg (0.22 mmol, 1.5 eq) TBTU gegeben und 40 min. bei RT gerührt. Die Suspension wird mit einigen Tropfen DMF versetzt, wobei alle ungelösten Bestandteile in Lösung gehen. Nun werden 17 mg (0.23 mmol, 1.5 eq) D-Alaninol zugegeben und 16 h bei RT gerührt. Danach wird die Reaktionsmischung mit 10 mL RP-Gel versetzt und alle flüchtigen Bestandteile im Vakuum entfernt. Es wird chromatographisch über eine RP-Phase gereinigt (MeCN/Wasser 15/85 + (je 0.2% HCOOH) auf 30/70 in 15 min). Nach Vereinigung der Produktfraktionen, Zugabe von Salzsäure (4 M in Dioxan) und Gefriertrocknung erhält man 85 mg des Hydrochlorids von **12.**

In analoger Weise unter Verwendung der entsprechenden Carbonsäurederivate **B-4** und Aminkomponenten werden **Beispiele 13 - 83, 85 -108, 125, 126** und **127** hergestellt, wobei gegebenenfalls anstelle von TBTU O-(7-Azabenzotriazol-1-yl)-*N,N,N',N'-*tetramethyluroniumhexafluorphösphat als Kupplungsreagens verwendet werden kann.

**Beispiele 128 - 130** werden nach dem allgemeinen Syntheseschema A hergestellt, wobei die Synthesesequenz mit der Umsetzung von **A-1a** mit 4-Amino-3-methoxy-benzoesäurebenzylester begonnen wird.

### Beispiel 110

### (±)-4-[4-((1R*,2S*)-2-Acetylamino-cyclohexylamino)-5-trifluormethyl-pyrimidin-2-ylamino]-N-methyl-N-(1-methyl-piperidin-4-yl)-benzamid

50 mg (0.1 mmol, 1 eq) **D-1a (Beispiel 109)** werden in 0.5 mL NMP gelöst, nachfolgend 17 µL (0.12 mmol, 1.2 eq) Triethylamin zugegeben und zu dieser Lösung 9 µL (0.12 mmol, 1.2 eq) Acetylchlorid getropft. Die Reaktionsmischung wird 16 h bei RT gerührt, mit RP-Gel versetzt und alle flüchtigen Bestandteile im Vakuum entfernt. Es wird chromatographisch über eine RP-Phase gereinigt. Man erhält 30 mg der Verbindung **110.**

In Analogie werden die **Beispiele 112-114** ausgehend von **D-1a** und den entsprechenden Carbonsäurechloriden hergestellt. Die **Beispiele 120-123** werden analog ausgehend von **D-1b (Beispiel 124)** durch Acylierung unter den für **Beispiel 110** beschriebenen Bedingungen hergestellt. Diese Synthesevorschrift findet ebenso Verwendung für die Synthese der Verbindungen **111** und **115,** wobei hier mit den entsprechenden Sulfonsäurechloriden umgesetzt wird. Im Falle von **Beispiel 116,** ein Harnstoffderivat, wird **D-1a** unter den für **Beispiel 110** beschriebenen Bedingungen mit *N,N-*Dimethylcarbamoylchlorid umgesetzt.

### Beispiel 117

Die Herstellung von **117** erfolgt analog zur Herstellung von **B-4a** ausgehend von **B-3a** und 3-endo-Aminobicyclo(2,2,1)-hept-5-en-2-endo-carbonsäure unter Verwendung von 1 Butanol als Lösungsmittel.

### Beispiel 118

Die Herstellung von **118** erfolgt analog zur Herstellung von **B-4a** ausgehend von **B-3a** und 2-Adamantanamin-hydrochlorid.

### Beispiel 119

Die Synthese erfolgt analog **Beispiel 12** unter Verwendung von **Beispiel 117** als Edukt. Nach erfolgter Amidkupplung wird die ungesättigte Zwischenverbindung unter Rühren bei RT durch Umsetzung mit Ameisensäure (11 eq) in Anwesenheit katalytischer Mengen an Pd/C in THF hydriert und auf diese Weise **Beispiel 119** erhalten.

### Beispiel 132

### 2-Ethoxy-4-[4-((1R,2S)-2-isopropylcarbamoyl-cyclopentylamino)-5-trifluormethylpyrimidin-2-ylaminol-N-methyl-N-1-methyl-piperidin-4-yl)-benzamid

46 mg (0.09 mmol, 1 eq) **C-2a** werden in 1.2 mL DMF gelöst und nachfolgend 81 µL (0.47 mmol, 5 eq) Hünig-Base sowie 42 mg (0.13 mmol, 1.4 eq) TBTU zugegeben. Es wird 5 min bei RT gerührt und anschließend 21 µL (0.14 mmol, 1.5 eq) 1-Methyl-4-(methylamino)-piperidin zugetropft. Die Reaktionsmischung wird 2 h bei RT gerührt und dann direkt chromatographisch über eine RP-Phase gereinigt. (Wasser/MeCN (+ je 0.2% HCOOH) von 83/17 auf 65/35% in 10 min). Nach Vereinigung der Produktfraktionen, Zugabe von 500 µL Salzsäure (4 M in Dioxan) und Gefriertrocknung erhält man 47 mg des Hydrochlorids von **132.**

Die **Beispiele 131** und **133-135** werden analog durch Amidkupplung aus den entsprechenden Säurederivaten **C-2** hergestellt, welche aus **C-1a** und den entsprechend substituierten 4-Aminobenzoesäuren durch nukleophile aromatische Substitution erhalten werden (beispielhaft für die Synthese von **C-2a** beschrieben).

### Beispiel 136

### (±)-4-[4-((1R*,2S*)-2-Isopropylcarbamoyl-cyclobutylamino)-5-trifluormethyl-pyrimidin-2-ylamino]-N-methyl-N-(1-methyl-piperidin-4-yl)-benzamid

150 mg (0.35 mmol, 1 eq) **B-3a** werden in 1.3 mL EtOH gelöst und 150 µL (0.88 mmol, 2.5 eq) Hünig-Base versetzt. Die Reaktionsmischung wird nach Zugabe von 100 mg (0.42 mmol, 1.2 eq) (±)-Cis-2-aminocyclobutancarbonsäureisopropylamid auf 70 °C erwärmt und 16 h bei dieser Temperatur gerührt. Die Reaktionsmischung wird danach mit 10 mL RP-Gel versetzt und alle flüchtigen Bestandteile im Vakuum entfernt. Es wird chromatographisch über eine RP-Phase gereinigt (MeCN/Wasser von 10/90 auf 30/70 in 15 min). Nach Vereinigung der Produktfraktionen, Zugabe von 100 µL dioxanischer Salzsäure (4 M) und Gefriertrocknung erhält man 86 mg des Hydrochlorids von **136.**

### Beispiel 139

### (±)-4-[5-Brom-4-[(1R*,2S*)-2-cyclopropylcarbamoyl)-cyclopentylamino)-pyrimidin-2-ylamino]-N-methyl-N(1-methyl-piperidin-4-yl)-benzamid

55 mg (0.10 mmol, 1 eq) **E-7b** werden in 1 mL DMF gelöst, 88 µL (0.5 mmol, 5 eq) Hünig-Base und 46 mg (0.14 mmol, 1.4 eq) TBTU zugegeben. Die Reaktionsmischung wird 10 min bei RT gerührt und nachfolgend werden 11 mg (0.15 mmol, 1.5 eq) Cyclopropylamin zugetropft. Es wird 2 Tage bei RT gerührt, die Reaktionsmischung über basisches Aluminiumoxid filtriert, anschließend mit RP-Gel versetzt, die flüchtigen Bestandteile im Vakuum entfernt und das Produkt säulenchromatographisch über eine RP-Phase gereinigt und isoliert (Wasser/MeCN (+ je 0.2% HCOOH) von 88/12% auf 75/25 in 10 min). Entsprechende Produktfraktionen werden vereinigt, mit 100 µL dioxanischer Salzsäure (4 M) versetzt, mittels Gefriertrocknung vom Lösungsmittel befreit und 25 mg der Verbindung **139** als Hydrochlorid erhalten.

Analog werden die Beispiele **137, 138, 140 - 144** und **146** hergestellt, wobei im Fall von **142, 143** und **146** die chirale Ausgangsverbindung **E-7a** eingesetzt wird.

### Beispiel 145/147

Die Synthese von **145** bzw. **147** erfolgt analog der Herstellung von **Beispiel 1** durch Amidkupplung von **C-2d** bzw. **C-2e** mit 1-Methyl-4-(methylamino)-piperidin.

### Beispiele 1-147

| **Bsp. Nr.** | **Struktur** | **Synthese- methode** | **HPLC RT [min]** | **MS (ESI⁺) [M+H]⁺** | **UVₘₐₓ [nm]** |
|---|---|---|---|---|---|
| 1 | 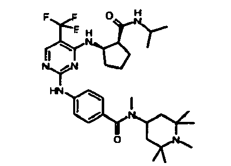 | A | 1.52 | 618 | 278 |
| 2 | 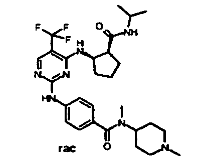 | A | 1.42 | 562 | 278 |
| 3 | 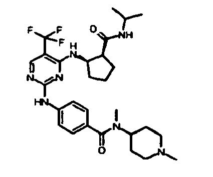 | A | 1.41 | 562 | 278 |
| 4 | 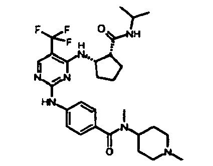 | A | 1.45 | 562 | 278 |
| 5 | 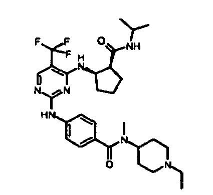 | A | 1.44 | 576 | 2178 |
| 6 | 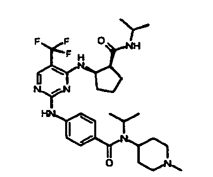 | A | 1.58 | 590 | 274 |
| 7 | 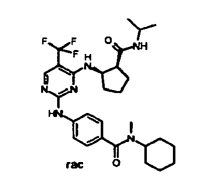 | A | 2.17 | 547 | 277 |
| 8 | 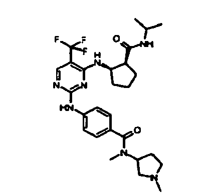 | A | 1.46 | 548 | 278 |
| 9 | 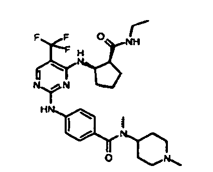 | A | 1.36 | 548 | 276 |
| 10 | 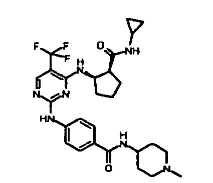 | A | | 546 | 284 |
| 12 | 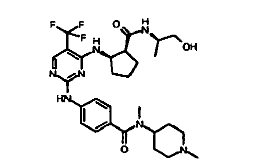 | B | 1.26 | 578 | 274 |
| 13 | 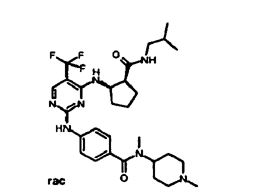 | B | 1.51 | 576 | 278 |
| 14 | 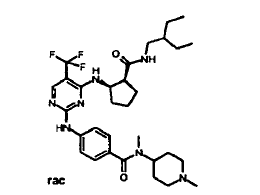 | B | 1.68 | 604 | 278 |
| 15 | 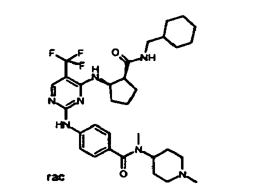 | B | 1.68 | 616 | 278 |
| 16 | 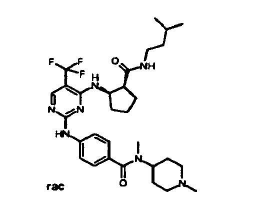 | B | 1.60 | 590 | 278 |
| 17 | 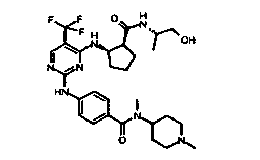 | B | | 578 | 274 |
| 18 | 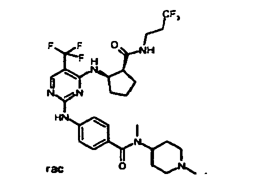 | B | 1.52 | 616 | 278 |
| 19 | 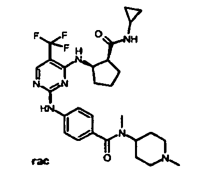 | B | 1.37 | 560 | 278 |
| 20 | 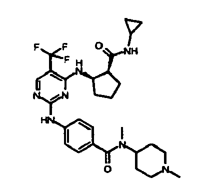 | B | 1.36 | 560 | 278 |
| 21 | 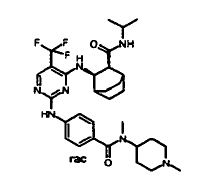 | B | 1.59 | 602 | 278 |
| 22 | 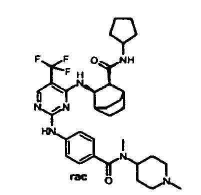 | B | 1.69 | 628 | 278 |
| 23 | 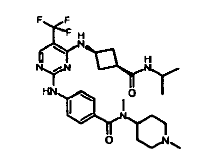 | B | | 548 | 278 |
| 24 | 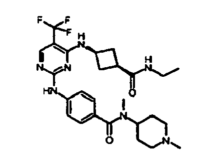 | B | | 534 | 274 |
| 25 | 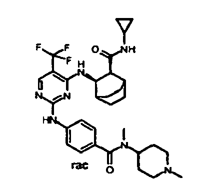 | B | 1.54 | 600 | 278 |
| 26 | 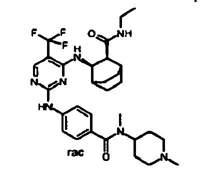 | B | 1.53 | 588 | 278 |
| 27 | 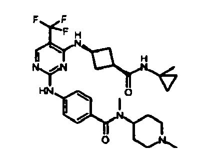 | B | 1.33 | 560 | 278 |
| 28 | 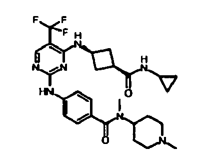 | B | 1.43 | 546 | 274 |
| 29 | 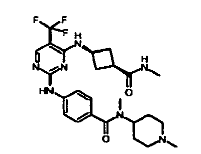 | B | 1.05 | 520 | 274 |
| 30 | 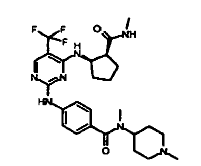 | B | | 534 | |
| 31 | 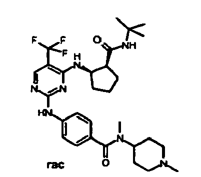 | B | | 576 | 278 |
| 32 | 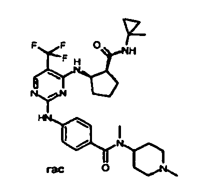 | B | | 574 | 278 |
| 33 | 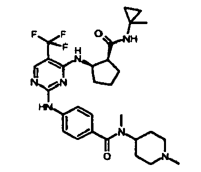 | B | 1.38 | 574 | 278 |
| 34 | 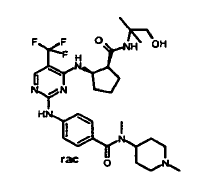 | B | | 592 | 274 |
| 35 | 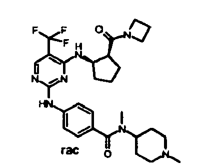 | B | 1.33 | 560 | 274 |
| 36 | 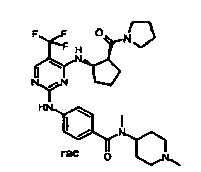 | B | 1.41 | 574 | 278 |
| 37 | 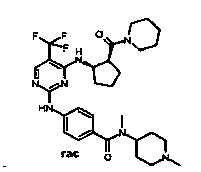 | B | 1.52 | 588 | 278 |
| 38 | 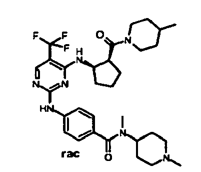 | B | 1.62 | 602 | 278 |
| 39 | 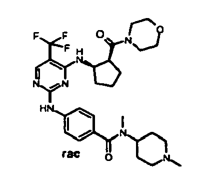 | B | 1.38 | 590 | 278 |
| 40 | 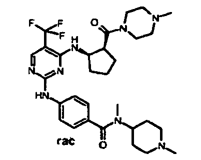 | B | 1.17 | 603 | 274 |
| 41 | 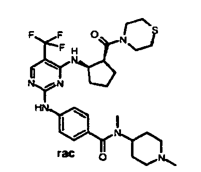 | B | 1.49 | 606 | 279 |
| 42 | 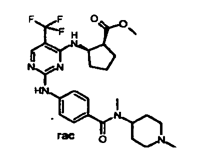 | B | 1.44 | 535 | 279 |
| 43 | 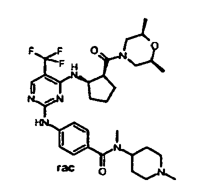 | B | 1.48 | 618 | 278 |
| 44 | 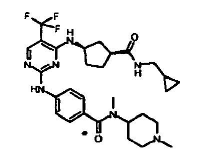 | B | 1.36 | 574 | 274 |
| 45 | 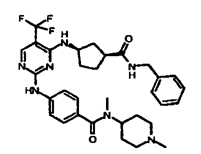 | B | 1.57 | 610 | 278 |
| 46 | 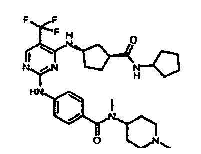 | B | 1.51 | 587 | 274 |
| 47 | 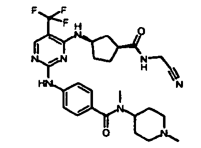 | B | 1.28 | 559 | 274 |
| 48 | 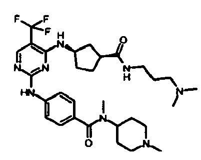 | B | | 605 | 274 |
| 49 | 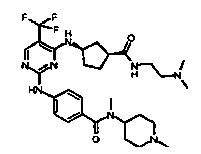 | B | | 591 | 270 |
| 50 | 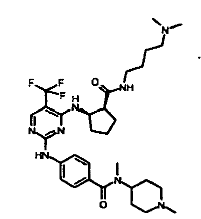 | B | | 619 | 278 |
| 51 | 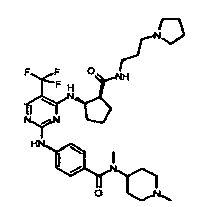 | B | | 631 | |
| 52 | 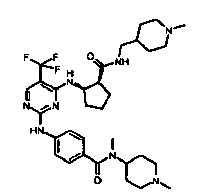 | B | | 631 | |
| 53 | 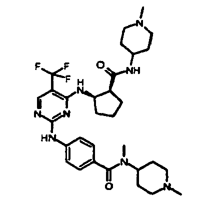 | B | | 617 | |
| 54 | 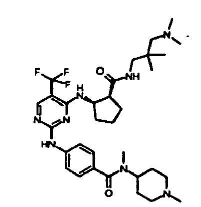 | B | | 633 | 271 |
| 55 | 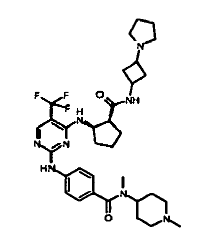 | B | | 643 | 276 |
| 56 | 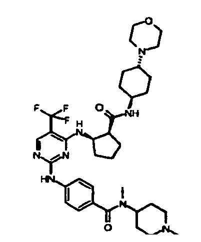 | B | | 687 | 275 |
| 57 | 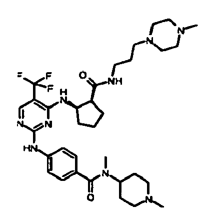 | B | | 660 | 275 |
| 58 | 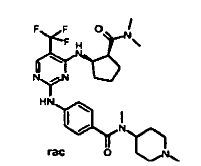 | B | 1.34 | 549 | 278 |
| 59 | 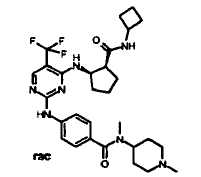 | B | 1.47 | 574 | 278 |
| 60 | 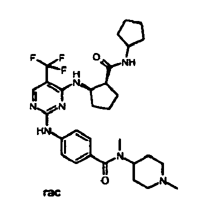 | B | 1.53 | 588 | 278 |
| 61 | 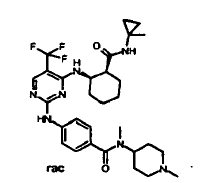 | B | 1.51 | 588 | 250 |
| 62 | 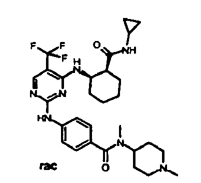 | B | 1.45 | 574 | 278 |
| 63 | 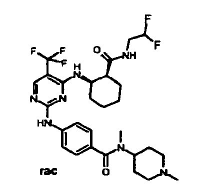 | B | 1.51 | 598 | 278 |
| 64 | 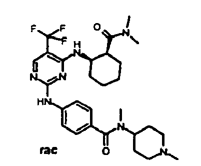 | B | 1.43 | 562 | 278 |
| 65 | 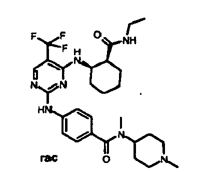 | B | 1.45 | 562 | 274 |
| 66 | 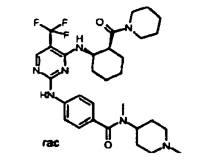 | B | 1.59 | 602 | 278 |
| 67 | 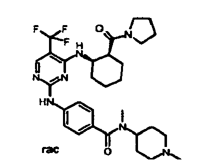 | B | 1.49 | 588 | 278 |
| 68 | 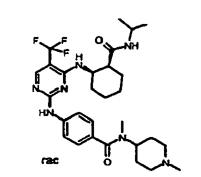 | B | 1.52 | 576 | 278 |
| 69 | 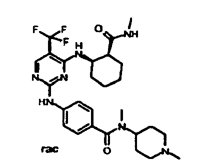 | B | 1.39 | 548 | 274 |
| 70 | 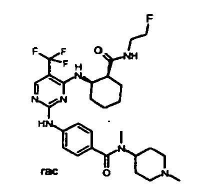 | B | 1.40 | 580 | 278 |
| 71 | 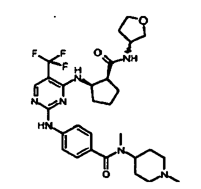 | B | 1.33 | 590 | 274 |
| 72 | 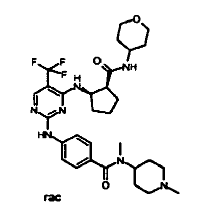 | B | | 604 | 278 |
| 73 | 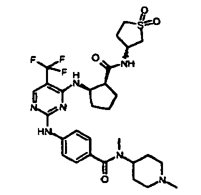 | B | | 638 | 278 |
| 74 | 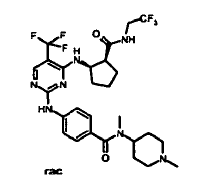 | B | 1.49 | 602 | 278 |
| 75 | 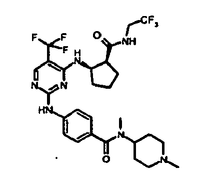 | B | 1.49 | 602 | 278 |
| 76 | 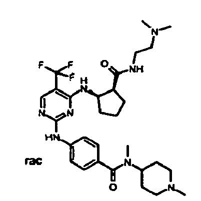 | B | | 591 | 274 |
| 77 | 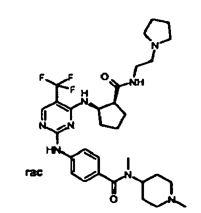 | B | | 617 | 278 |
| 78 | 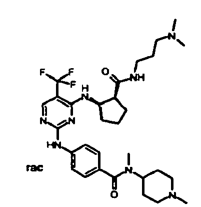 | B | | 605 | 278 |
| 79 | 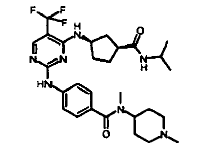 | B | 1.40 | 562 | 274 |
| 80 | 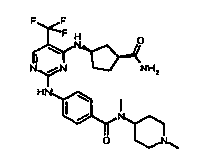 | B | | 520 | 270 |
| 81 | 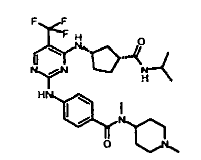 | B | 1.37 | 562 | 274 |
| 82 | 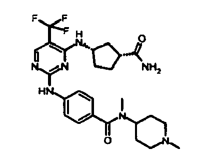 | B | | 520 | 270 |
| 83 | 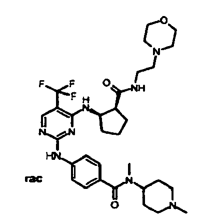 | B | | 633 | 298 |
| 84 | 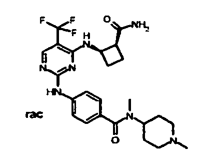 | A | | 506 | 274 |
| 85 | 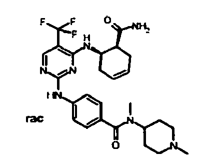 | B | 1.20 | 532 | 298 |
| 86 | 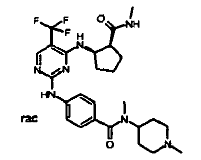 | B | | 535 | 278 |
| 87 | 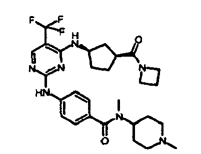 | B | | 560 | |
| 88 | 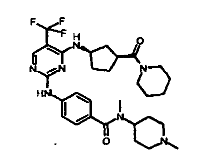 | B | 1.49 | 588 | 274 |
| 89 | 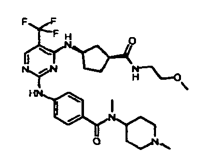 | B | 1.29 | 578 | 274 |
| 90 | 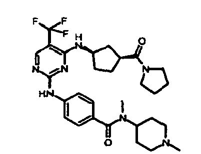 | B | 1.33 | 574 | 278 |
| 91 | 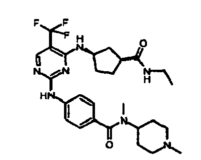 | B | | 548 | 274 |
| 92 (B-4c) | 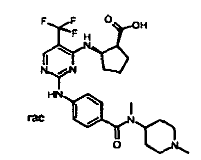 | B | 1.30 | 521 | 278 |
| 93 | 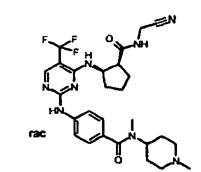 | B | 1.31 | 559 | 278 |
| 94. | 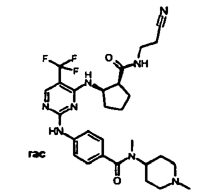 | B | 1.33 | 573 | 278 |
| 95 | 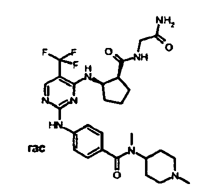 | B | | 577 | 298 |
| 96 | 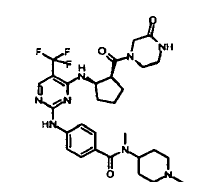 | B | | 603 | 298 |
| 97 | 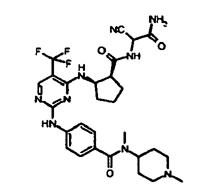 | B | 1.28 | 602 | 298 |
| 98 | 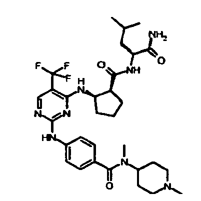 | B | 1.46 | 633 | 298 |
| 99 | 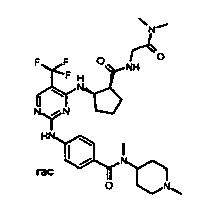 | B | 1.32 | 605 | 298 |
| 100 | 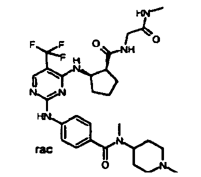 | B | 1.22 | 591 | 298 |
| 101 | 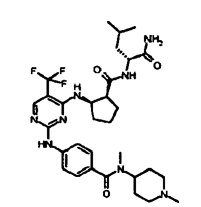 | B | 1.46 | 633 | 298 |
| 102 | 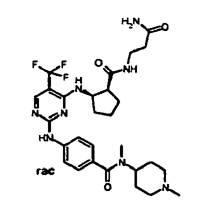 | B | 1.21 | 591 | 298 |
| 103 | 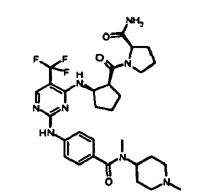 | B | | 617 | 298 |
| 104 | 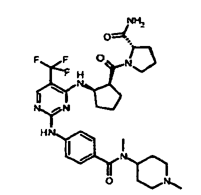 | B | | 617 | 298 |
| 105 | 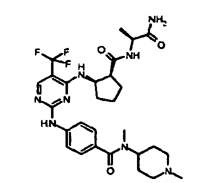 | B | | 591 | 298 |
| 106 | 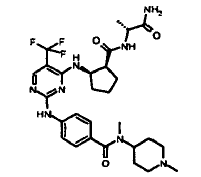 | B | | 591 | 298 |
| 107 | 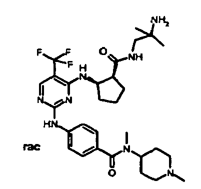 | B | | 591 | 302 |
| 108 | 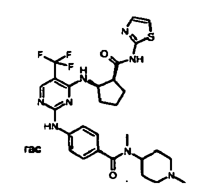 | B | 1.53 | 603 | 278 |
| 109 (D-1a) | 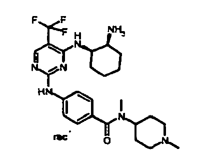 | D | | 506 | 280 |
| 110 | 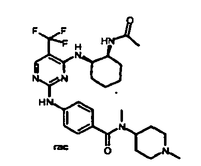 | D | | | 272 |
| 111 | 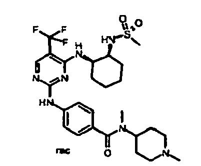 | D | 1.35 | 584 | 274 |
| 112 | 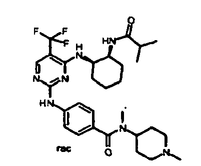 | D | 1.45 | 576 | 276 |
| 113 | 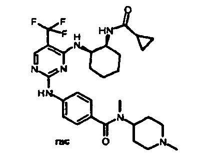 | D | 1.43 | 574 | 275 |
| 114 | 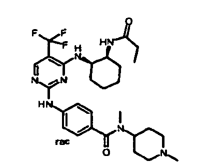 | D | 1.35 | 562 | 274 |
| 115 | 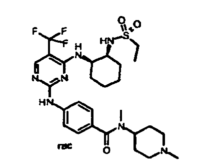 | D | 1.38 | 598 | 276 |
| 116 | 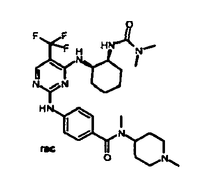 | D | 1.43 | 577 | 276 |
| 117 | 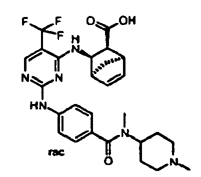 | B | 1.38 | 545 | 274 |
| 118 | 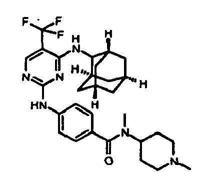 | B | 1.82 | 543 | 281 |
| 119 | 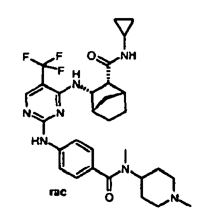 | B | 1.51 | 586 | 273 |
| 120 | 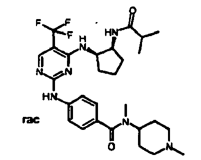 | D | 1.42 | 562 | 275 |
| 121 | 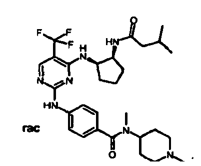 | D | 1.47 | 576 | 278 |
| 122 | 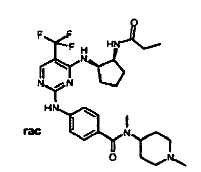 | D | 1.27 | 548 | 272 |
| 123 | 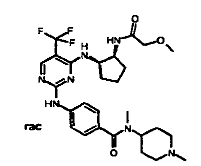 | D | 1.29 | 564 | 275 |
| 124 (D-1a) | 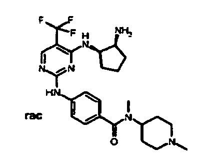 | D | | 492 | 277 |
| 125 | 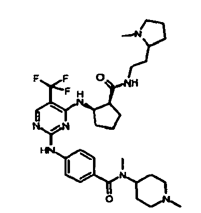 | B | | 631 | 277 |
| 126 | 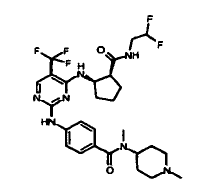 | B | 1.42 | 584 | 277 |
| 127 | 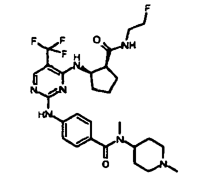 | B | 1.30 | 566 | 270 |
| 128 | 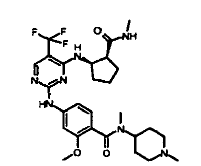 | A | 1.52 | 564 | 270 |
| 129 | 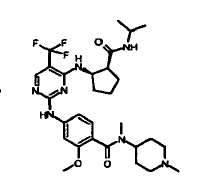 | A | 1.70 | 592 | 274/300 |
| 130 | 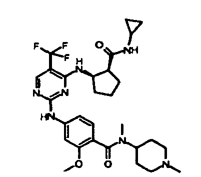 | A | 1.59 | 590 | 272/300 |
| 131 | 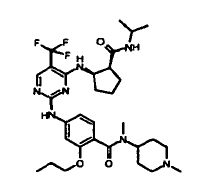 | C | 1.56 | 620 | 238 |
| 132 | 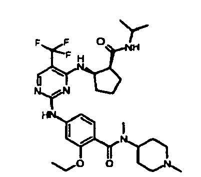 | C | 1.42 | 606 | 226 |
| 133 | 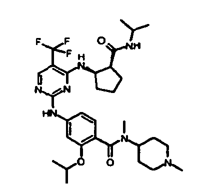 | C | 1.53 | 620 | 226 |
| 134 | 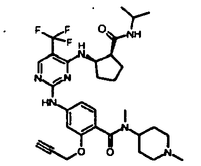 | C | 1.48 | 616 | 230 |
| 135 | 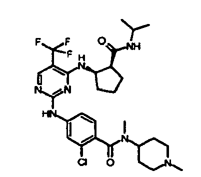 | C | 1.54 | 596 | 222/274 |
| 136 | 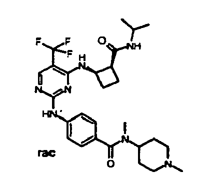 | D | 1.39 | 548 | 278 |
| 137 | 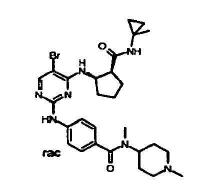 | E | 1.22 | 584/586 | 278 |
| 138 | 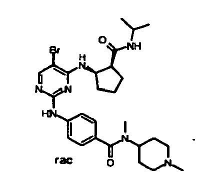 | E | 1.28 | 572/574 | 278 |
| 139 | 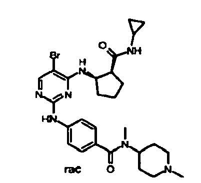 | E | | 570/572 | 274 |
| 140 | 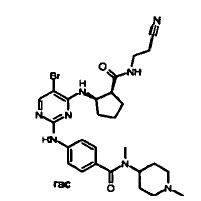 | E | | 583/585 | 275 |
| 141 | 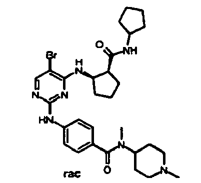 | E | 1.35 | 598/600 | 278 |
| 142 | 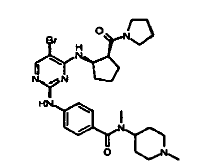 | E | | 584/586 | 218/278 |
| 143 | 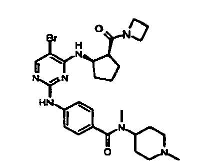 | E | | 570/572 | 214/276 |
| 144 | 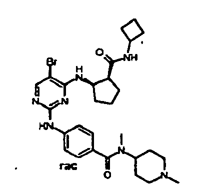 | E | 1.27 | 584/586 | 278 |
| 145 | 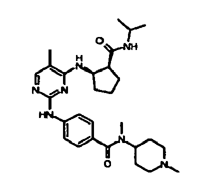 | C | 1.19 | 508 | 270 |
| 146 | 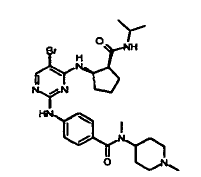 | E | | 572/574 | |
| 147 | 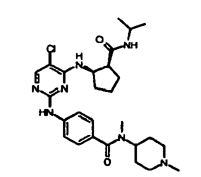 | C | | 528/530 | |

Die nachfolgenden Beispiele beschreiben die biologische Wirkung der erfindungsgemäßen Verbindungen ohne die Erfindung auf diese Beispiele einzuschränken.

Wie durch DNA-Färbung mit darauf folgender FACS oder Cellomics Array Scan Analysen gezeigt werden konnte, ist die durch die erfindungsgemäßen Verbindungen bewirkte Proliferationsinhibition vor allem durch Fehler in der Chromosomensegregation vermittelt. Wegen der Anhäufung von Fehlsegregationen kommt es zu einer massiven Polyploidie, die letztlich in einer Proliferationshemmung oder sogar Apoptose münden kann. Auf Grund ihrer biologischen Eigenschaften eignen sich die erfindungsgemäßen Verbindungen der allgemeinen Formel (1), deren Isomere und deren physiologisch verträgliche Salze sowie Polymorphe zur Behandlung von Erkrankungen, die durch exzessive oder abnormale Zellproliferation charakterisiert sind.

### Beispiel Aurora-B Kinase-Assay

Es wurde ein radioaktiver Enzymhemmungs-Assay entwickelt, der E.coli-exprimiertes rckombinantes N-terminal mit einem GST-tag ausgestattetes *Xenopus laevis* Aurora B Wildtyp Protein (Aminosäuren 60-361) im Komplex mit *Xenopus laevis* INCENP (Aminosäuren 790-847) verwendet, das aus Bakterien gewonnen und gereinigt wird. Äquivalent kann auch eine *Xenopus laevis* Aurora B Mutante (G96V) im Komplex mit *Nenopus laevis* INCENP⁷⁹⁰⁻⁸⁴⁷ verwendet werden.

### Epression und Reinigung

Die kodierende Sequenz für Aurora-B⁶⁰⁻³⁶¹ aus *Xenopus laevis* wird in eine modifizierte Version von pGEX-6T (Amersham Biotech) via BamHI und SalI Schnittstellen kloniert. Der Vektor enthält zwei Klonierungskassetten, die durch eine ribosomale Bindestelle getrennt sind, was bi-zistronische Expression erlaubt. In dieser Konfiguration wird *Nenopus laevis* Aurora B von der ersten Kassette, das *Xenopus laevis* INCENP⁷⁹⁰⁻⁸⁴⁷ von der zweiten Kassette exprimiert. Der resultierende Vektor ist pAUB-IN⁸⁴⁷ .

Zunächst wird der E.coli Stamm BL21 (DE3) mit pUBS520 Helfer Plasmid und pAUB-IN⁸⁴⁷ ko-transformiert, woraufhin Protein Expression mittels 0.3 mM IPTG bei einer OD₆₀₀ von 0.45-0.7 induziert wird. Die Expression wird dann für ca. 12-16 Stunden bei 23-25 °C unter Schütteln fortgesetzt.
Die Bakterien werden danach abzentrifugiert und das Pellet in Lysispuffer (50 mM Tris/Cl pH 7.6, 300 mM NaCl, 1 mM DTT, 1 mM EDTA, 5 % Glycerol, Roche Complete Protease Inhibitor Tabletten) mithilfe von Ultraschall aufgeschlossen, wobei 20-30 mL Lysispuffer pro Liter E.coli Kultur eingesetzt werden. Der Aufschluss wird durch Zentrifugation (12000 rpm, 45-60 min, JA20 Rotor) von Debris befreit. Der Überstand wird mit 300 µL äquilibrierter GST Sepharose Fast Flow (Amersham Biosciences) pro Liter E.coli Kultur für 4-5 h bei 4 °C inkubiert. Danach wird das Säulenmaterial mit 30 Volumen Lysispuffer gewaschen und anschließend mit 30 Volumen Spaltungspuffer (50 mM Tris/Cl pH 7.6, 150 mM NaCl, 1 mM DTT, 1 mM EDTA) äquilibriert. Um das GST-tag von Aurora B abzuspalten werden 10 Einheiten Prescission Protease (Amersham Biosciences) pro Milligram Substrat eingesetzt und für 16 h bei 4 °C inkubiert. Der Überstand, der das Spaltprodukt enthält, wird auf eine mit lonenaustauschpuffer (50 mM Tris/Cl pH 7.6, 150 mM NaCl, 1 mM DTT, 1 mM EDTA) äquilibrierte 6 mL Resource Q Säule (Amersham Biosciences) geladen. Der Aurora B/INCENP Komplex wird im Durchfluss aufgefangen, welcher ankonzentriert und auf eine Superdex 200 size-exclusion chromatography (SEC) Säule geladen wird, die mit SEC-Puffer (10 mM Tris/Cl pH 7.6, 150 mM NaCl, 1 mM DTT, 1 mM EDTA) äquilibriert ist. Fraktionen, die den AuroraB /INCENP-Komplex enthalten, werden gesammelt und mittels Vivaspin Konzentratoren (MW Ausschluß 3000-5000 Da) auf eine finale Konzentration von 12 mg/mL konzentriert. Aliquots (z.B. 240 ng/pL) für Kinase-Assays werden aus dieser Stammlösung in Einfrierpuffer (50 mM Tris/Cl pH 8.0, 150 mM NaCl, 0.1 mM EDTA, 0.03% Brij-35, 10% Glycerol, 1 mM DTT) überführt und bei -80°C aufbewahrt.

### Kinase-Assay

Testsubstanzen werden in eine Polypropylenplatte (96 Vertiefungen, Greiner #655 201) vorgelegt um einen Konzentrationsrahmen von 10 µM - 0.0001 µM abzudecken. Die Endkonzentration von DMSO im Assay ist 5%. Zu vorgelegten 10 µL Testsubstanz in 25% DMSO werden 30 µL Protein-Mix (50 mM Tris/Cl pH 7.5, 25 mM MgCl₂, 25 mM NaCl, 166 µM ATP, 10 ng Xenopus laevis Aurora B/INCENP Komplex in Einfrierpuffer) pipettiert und 15 min bei RT inkubiert. Danach werden 10 µL Peptid-Mix (100 mM Tris/Cl pH 7.5, 50 mM Mach, 50 mM NaCl, 5 mM NaF, 5 mM DTT, 1 µCi gamma-P33-ATP [Amersham], 50 µM Substratpeptid [Biotin-EPLERRLSLVPDS oder Multimere davon, oder Biotin-EPLERRLSLVPKM oder Multimere davon, bzw. Biotin-LRRWSLGLRRWSLGLRRWSLGLRRWSLG]) zugegeben. Die Reaktion wird 75 min inkubiert (RT) und durch Zugabe von 180 µL 6.4% Trichloressigsäure gestoppt und für 20 min auf Eis inkubiert. Eine Multiscreen Filtrationsplatte (Millipore, MAIP NOB10) wird mit zunächst 100 µL 70% EtOH und danach mit 180 µL Trichloressigsäure äquilibriert und die Flüssigkeiten mit einem geeigneten Absauggerät entfernt. Danach wird die gestoppte Kinasereaktion aufgebracht. Nach 5 Waschschritten mit jeweils 180 µL 1 % Trichloressigsäure wird der untere Teil der Platte getrocknet (10-20 min. bei 55 °C) und 25 µL Scintillations-Cocktail (Microscint, Packard # 6013611) zugegeben. Inkorporiertes gamma-Phosphat wird mithilfe eines Wallac 1450 Microbeta Flüssigscintillations Zählers quantifiziert. Proben ohne Testsubstanz oder ohne Substratpeptid dienen als Kontrollen. IC₅₀ Werte werden mittels Graph Pad Prism Software ermittelt.

Die anti-proliferative Wirkung der erfindungsgemäßen Verbindungen wird im Proliferationstest an kultivierten humanen Tumorzellen und/oder in einer Zell Zyklus Analyse, beispielsweise an NCI-H460 Tumorzellen, bestimmt. Die Verbindungen 1 - 147 zeigen in beiden Testmethoden eine gute bis sehr gute Wirksamkeit, d.h. beispielsweise einen EC₅₀-Wert im NCI-H460-Proliferationstest kleiner 5 µmol/L, in der Regel kleiner 1 µmol/L.

### Messung der Inhibierung der Proliferation an kultivierten humanen Tumorzellen

Zur Messung der Proliferation an kultivierten humanen Tumorzellen werden Zellen der Lungen Tumorzell-Linie NCI-H460 (erhalten von American Type Culture Collection (ATGC)) in RPMI 1640 Medium (Gibco) und 10% fötalem Rinderserum (Gibco) kultiviert und in der log-Wachstumsphase geerntet. Anschließend werden die NCI-H460 Zellen in 96-well Flachbodenplatten (Falcon) mit einer Dichte von 1000 Zellen pro well in RPMI 1640 Medium eingebracht und über Nacht in einem Inkubator (bei 37 °C und 5 % CO₂) inkubiert. Die Wirksubstanzen werden in verschiedenen Konzentrationen (gelöst in DMSO; DMSO-Endkonzentration: 0.1%) zu den Zellen zugegeben. Nach 72 h Inkubation werden zu jedem well 20 µL AlamarBlue Reagenz (AccuMed International) zugesetzt, und die Zellen für weitere 5-7 h inkubiert. Nach Inkubation wird der Farbumsatz des AlamarBlue Reagenz in einem Wallac Microbeta Fluoreszenzspektrophotometer bestimmt. EC₅₀ Werte werden mittels Standard Levenburg Marquard Algorithmen (GraphPadPrizm) kalkuliert.

Zellzyklusanalysen werden beispielsweise mittels FACS Analysen (Fluorescence Activated Cell Sorter) oder mittels Cellomics Array Scan (CellCycle Analysis) durchgeführt

### FACS - Analyse

Propidium Iodid (PI) bindet stöchiometrisch an doppelsträngige DNA, und ist damit geeignet den Anteil an Zellen in der G1, S, und G2/M Phase des Zellzykluses auf der Basis des zellulären DNA Gehaltes zu bestimmen. Zellen in der G0 und G1 Phase haben einen diploiden DNA Gehalt (2N), während Zellen in der G2 oder Mitose einen 4N DNA Gehalt haben.

Für eine PI-Färbung werden beispielsweise 1.75x 10⁶ NCI-H460 Zellen auf eine 75 cm² Zellkulturflasche ausgesät, nach 24 h wird entweder 0.1 % DMSO als Kontrolle zugesetzt, bzw. die Substanz in verschiedenen Konzentrationen (in 0.1 % DMSO). Die Zellen werden für 42 h mit der Substanz bzw. mit DMSO inkubiert. Dann werden die Zellen mit Trypsin abgelöst und zentrifugiert. Das Zellpellet wird mit gepufferter Kochsalzlösung (PBS) gewaschen und die Zellen anschließend mit 80% EtOH bei -20 °C für mindestens 2 h fixiert. Nach einem weiteren Waschschritt mit PBS werden die Zellen mit Triton-X100 (Sigma; 0.25% in PBS) für 5 min auf Eis perrneabilisiert und anschließen mit einer Lösung aus Propidium Iodid (Sigma; 10µg/mL)und RNAse (Serva; 1 mg/mL)im Verhältnis 9/1 für mindestens 20 min im Dunkeln inkubiert.

Die DNA Messung erfolgt in einem Becton Dickinson FACS Analyzer, mit einem Argonlaser (500 mW, Emission 488 nm), Datenerhebung und Datenauswertung erfolgt mit dem DNA Cell Quest Programm (BD).

### Cellomics Array Scan

NCI-H460 Zellen werden in 96-well Flachbodenplatten (Falcon) in RPMI 1640 Medium (Gibco) mit 10% fötalem Rinderserum (Gibco) in einer Dichte von 2000 Zellen pro well ausgesät und über Nacht in einem Inkubator (bei 37 °C und 5 % CO₂) inkubiert. Die Wirksubstanzen werden in verschiedenen Konzentrationen (gelöst in DMSO; DMSO-Endkonzentration: 0.1%) zu den Zellen zugegeben. Nach 42 h Inkubation wird das Medium abgesaugt, die Zellen 10 min mit 4% Formaldehydlösung und Triton X-100 (1/200 in PBS) bei RT fixiert und gleichzeitig permeabilisiert, anschließend 2 mal mit einer 0.3% BSA Lösung (Calbiochem) gewaschen. Anschließend erfolgt die Färbung der DNA durch Zugabe von 50 µL/well 4',6-Diarnidino-2-phenylindol (DAPI; Molecular Pröbes) in einher. Endkonzentration von 300 nM für 1 h bei RT im Dunkeln. Danach wird 2 mal sorgfältig mit PBS gewaschen, die Platten mit schwarzer Klebefolie abgeklebt und im Cellomics ArrayScan mittels CellCycle BioApplication Programm analysiert und via Spotfire visualisiert und ausgewertet.

Die Substanzen der vorliegenden Erfindung sind Aurora Kinaseinhibitoren. Auf Grund ihrer biologischen Eigenschaften eignen sich die neuen Verbindungen der allgemeinen Formel (1), deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma), Entzündung und Autoimmunerkrankungen (z.B. Colitis, Arthritis, Alzheimer Erkrankung, Glomerulonephritis und Wundheilung), bakterielle, fungale und/oder parasitäre Infektionen, Leukämien, Lymphoma und solide Tumore (zB. Karzinome und Sarkome), Hauterkrankungen (z.B. Psoriasis), auf Hyperplasie beruhende Krankheiten die durch einen Anstieg der Zellzahl (z.B. Fibroblasten, Hepatozyten, Knochen und Knochenmarkzellen, Knorpel oder glatte Muskulaturzellen oder Epithelialzellen (z.B. endometrische Hyperplasie)) gekennzeichnet sind; Knochenerkrankungen und kardiovaskuläre Erkrankungen (z.B. Restenose und Hypertrophie).

Beispielsweise können folgende Krebserkrankungen mit erfindungsgemäßen Verbindungen behandelt werden, ohne jedoch darauf beschränkt zu sein: Himtumore wie beispielsweise Akustikusneurinom, Astrozytome wie pilozytisches Astrozytome, fibrilläres Astrozytom, protoplasmatisches Astrozytom, gemistozytäres Astrozytom, anaplastisches Astrozytom und Glioblastome, Himlymphome, Hirnmetastasen, Hypophysentumor wie Prolaktinom, HGH (human growth hormon) produzierender Tumor und ACTH produzierender Tumor (adrenocorticotropes Hormon), Kraniopharyngiome, Medulloblastome, Meningeome und Oligodendrogliome; Nerventumore (Neoplasmen) wie zum Beispiel Tumore des vegetativen Nervensystems wie Neuroblastoma sympathicum, Ganglioneurom, Paragangliom (Phäochromozytom, Chromaffinom) und Glomuscaroticum-Tumor, Tumore am peripheren Nervensystem wie Amputationsneurorh, Neurofibrom, Neurinom (Neurilemmom, Schwannom) und malignes Schwannom, sowie Tumore am zentralen Nervensystem als Hirn- und Rückenmarkstumoren; Darmkrebs wie zum Beispiel Rektumkarzinom, Kolonkarzinom, Analkarzinom, Dünndarmtumore und Zwölffingerdarmtumore; Lidtumore wie Basaliom oder Basalzellkarzinom; Bauchspeicheldrüsenkrebs oder Pankreaskarzinom; Blasenkrebs oder Blasenkarzinom; Lungenkrebs (Bronchialkarzinom) wie beispielsweise kleinzellige Bronchialkarzinome (Haferzellkarzinome) und nicht-kleinzellige Bronchialkarzinome wie Plattenepithelkarzinome, Adenokarzinome und großzellige Bronchialkarzinome; Brustkrebs wie zum Beispiel Mammakarzinom wie infiltrierend duktales Karzinom, Kolloidkarzinom, lobulär invasives Karzinom, tubuläres Karzinom, adenozystisches Karzinom und papilläres Karzinom; Non-Hodgkin-Lymphomen (NHL) wie beispielsweise Burkitt-Lymphom, niedrigmaligne Non-Hodgkin-Lymphomen (NHL) und Mucosis fungoides; Gebärrnutterkrebs oder Endometriumkarzinom bzw. Corpuskarzinom; CUP-Syndrom (Cancer of Unknown Primary); Eierstockskrebs oder Ovarial-Karzinom wie mucinöse, endometriale oder seröse Krebs; Gallenblasenkrebs; Gallengangskrebs wie beispielsweise Klatskin-Tumor; Hodenkrebs wie zum Beispiel Seminome und Nicht-Seminome; Lymphom (Lymphosarkom) wie zum Beispiel malignes Lymphom, Morbus Hodgkin, Non-Hodgkin-Lymphomen (NHL) wie chronisch lymphatische Leukämie, Haarzell-Leukämie, Immunozytom, Plasmozytom (multiples Myelom), Immunoblastom, Burkitt-Lymphom, T-Zonen-Mycosis fungoides, großzellig-anaplastisches Lymphoblastom und Lymphoblastom; Kehlkopfkrebs wie zum Beispiel Stimmbandtumoren, supraglottisch, glottisch und subglottisch Kehlkopftumore; Knochenkrebs wie zum Beispiel Osteochondrom, Chondrom, Chondroblastom, Chondromyxoidfibrom, Osteom, Osteoid-Osteom, Osteoblastom, eosinophiles Granulom, Riesenzell-Tumor, Chondrosarkom, Osteosarkom, Ewing-Sarkom, Retikulo-Sarkom, Plasmozytom, Riesenzell-Tumor, fibröse Dysplasie, juvenile Knochenzyste und aneurysmatische Knochenzyste; Kopf-Hals-Tumoren wie zum Beispiel Tumoren der Lippen, Zunge, Mundboden, Mundhöhle, Zahnfleisch, Gaumen, Speicheldrüsen, Rachen, Nasenhöhlen, Nasennebenhöhlen, Kehlkopf und Mittelohr; Leberkrebs wie zum Beispiel das Leberzellkarzinom oder hepatozelluläres Karzinom (HCC); Leukämien wie zum Beispiel akute Leukämien wie akute lymphatische/lymphoblastische Leukämie (ALL), akute myeloische Leukämie (AML); chronische Leukämien wie chronisch lymphatische Leukämie (CLL), chronisch myeloische Leukämie (CML); Magenkrebs oder Magenkarzinom wie zum Beispiel papilläres, tubuläres und muzinöses Adenokarzinom, Siegelringzellkarzinom adenosquamöses Karzinom, kleinzelliges Karzinom und undifferenziertes Karzinom; Melanome wie zum Beispiel oberflächlich spreitendes, knotiges, lenitgo-maligna und akral-lentiginöse Melanom; Nierenkrebs wie zum Beispiel Nierenzellkarzinom oder Hypernephrom oder Grawitz-Tumor; Speiseröhrenkrebs oder Ösophaguskarzinom; Peniskrebs; Prostatakrebs; Rachenkrebs oder Pharynxkarzinome wie zum Beispiel Nasopharynxkarzinome, Oropharynxkarzinome und Hypopharynxkarzinome; Retinoblastom; Scheidenkrebs oder Vaginalkarzinorn; Plattenepithelkarzinome, Adenokarzinome, in situ Karzinome, maligne Melanome und Sarkome; Schilddrüsen-Karzinome wie zum Beispiel papilläre, follikuläre und medulläre Schilddrüsen-Karzinom, sowie anaplastische Karzinome; Spinaliom, Stachelzellkarzinom und Plattenepithelkarzinom der Haut; Thymome, Urethrakrebs und Vulvakrebs.

Die neuen Verbindungen können zur Prävention, Kurz- oder Langzeitbehandlung der vorstehend erwähnten Krankheiten gegebenenfalls auch in Kombination mit Radiotherapie oder mit anderen "State-of-art" Verbindungen, wie z.B. zytostatische oder zytotoxische Substanzen, Zellproliferationshemmer, anti-angiogene Substanzen, Steroide oder Antikörper, verwendet werden.

Die Verbindungen der allgemeinen Formel (1) können allein oder in Kombination mit anderen erfindungsgemäßen Wirkstoffen, gegebenenfalls auch in Kombination mit weiteren pharmakologisch aktiven Wirkstoffen, zur Anwendung gelangen.

Chemotherapeutica welche in Kombination mit den erfindungsgemäßen Verbindungen verabreicht werden können, umfassen ohne darauf eingeschränkt zu sein, Hormone, Hormonanaloge und Antihormone (z.B. Tamoxifen, Toremifen, Raloxifen, Fulvestrant, Megestrol Acetat, Flutamid, Nilutamid, Bicalutamid, Aminoglutethimid, Cyproteron Acetat. Finasterid, Buserelin Acetat, Fludrocortinson, Fluoxymesteron, hlcdroxyprogesteron, Octreotid), Aromatase Inhibitoren (z.B. Anastrozol, Letrozol, Liarozol, Vorozol, Exemestan, Atamestan,), LHRH Agonisten und Antagonisten (z.B. Goserelin Acetat, Luprolid), Inhibitoren von Wachstumsfaktoren (growth factors wie zum Beispiel "platelet derived growth factor" und "hepatocyte growth factor", Inhibitoren sind z. B. "growth factor"-Antikörper, "growth factor receptor"-Antikörper und Tyrosinekinase Inhibitoren, wie zum Beispiel Gefitinib, Imatinib, Lapatinib Cetuximab (Erbitux®) und Trastuzuniab); Antimetabolite (z.B. Antifolate wie Methotrexat, Raltitrexed, Pyrimidinanaloge wie 5-Fluorouracil, Capecitabin und Gemcitabin, Purin- und Adenosinanaloge wie Mercaptopurin, Thioguanin, Cladribin und Pentostatin, Cytarabin, Fludarabin); Antitumor-Antibiotica (z.B. Anthracycline wie Doxorubicin, Daunorubicin, Epirubicin und Idarubicin, Mitomycin-C, Bleomycin, Dactinomycin, Plicamycin, Strcptozocin); Platinderivative (z.B. Cisplatin, Oxaliplatin, Carboplatin); Alkylicrungsagentien (z.B. Estramustin, Meclorethamin, Melphalan, Chlorambucil, Busulphan, Dacarbazin, Cyclophosphamid, Ifosfamid, Temozolomid, Nitrosohamstoffe wie zum Beispiel Carmustin und Lomustin, Thiotepa); antimitotische Agentien (z.B. Vinca-Alkaloide wie zum Beispiel Vinblastin, Vindesin, Vinorelbin und Vincristin; und Taxane wie Paclitaxel, Docetaxel); Topoisomerase Inhibitoren (z.B. Epipodophyllotoxine wie zum Beispiel Etoposid und Etopophos, Teniposid, Amsacrin, Topotecan, Irinotecan, Mitoxantron) und verschiedene Chemotherapeutica wie Amifostin, Anagrelid, Clodronat, Filgrastin, Interferon alpha, Leucovorin, Rituximab, Procarbazin, Levamisol, Mesna, Mitotan, Pamidronat und Porfimer.

Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0.1 - 90 Gew.%, bevorzugt 0.5 - 50 Gew.% der Gesamtzusammensetzung liegen, das heißt in Mengen die ausreichend sind, um den unten angegebenen Dosierungsbereich zu erreichen. Die genannten Dosen können, falls erforderlich, mehrmals täglich gegeben werden.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoate, oder Stabilisatoren, wie Alkalisalzen der Ethylendiamintetraessigsäure, gegebenenfalls unter Verwendung von Emulgiermitteln und/oder Dispergiermitteln, wobei beispielsweise bei der Verwendung von Wasser als Verdünnungsmittel gegebenenfalls organische Lösemittel als Lösevermittler bzw. Hilfslösemittel eingesetzt werden können, hergestellt und in Injektionsflaschen oder Ampullen oder lnfusionsflaschen abgefüllt.
Die eine oder mehrere Wirkstoffe beziehungsweise Wirkstoffkombinationen enthaltenden Kapseln können beispielsweise hergestellt werden indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. EtOH oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dikalziumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen können die Wirkstoffe außer den oben genannten Hilfsstoffen mit verschiedenen Geschmacksaufbesserem oder Farbstoffen versetzt werden.

Für den Fall der parenteralen Anwendung können Lösungen der Wirkstoffe unter Verwendung geeigneter flüssiger Trägermaterialien eingesetzt werden.

Die Dosierung für die intravenöse Anwendung liegt bei 1 - 1000 mg pro Stunde, vorzugsweise zwischen 5 - 500 mg pro Stunde.

Trotzdem kann es gegebenenfalls notwendig sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Formulierungsbeispiele illustrieren die vorliegende Erfindung ohne sie jedoch in ihrem Umfang zu beschränken.

### Pharmazeutische Formulierungsbeispiele

| A) | Tabletten | pro Tablette |
|---|---|---|
| | Wirkstoff nach Formel (1) | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der fein gemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feucht granuliert und getrocknet. Das Granulat, der Rest der Maisstärke und das Magnesiumstearat werden gesiebt und miteinander vermischt. Das Gemisch wird zu Tabletten geeigneter Form und Größe verpresst.

| B) | Tabletten | pro Tablette |
|---|---|---|
| | Wirkstoff nach Formel (1) | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natriumcarboxymethylstärke | 23 mg |
| | Magnesiumstearat | 2 mg |
| | | 400 mg |

Der fein gemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpresst das Gemisch zu Tabletten geeigneter Größe.

| C) | Ampullenlösung | |
|---|---|---|
| | Wirkstoff nach Formel (1) | 50 mg |
| | Natriumchlorid | 50 mg |
| | Aqua pro inj. | 5 ml |

Der Wirkstoff wird bei Eigen-pH oder gegebenenfalls bei pH 5.5 - 6.5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (1), 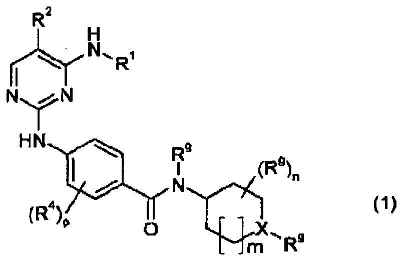 ausgewählt von der Gruppe bestehend aus
4-[4-((1R,2S)-2-Isopropylcarbamoyl-cyclopentylamino)-5-trifluormethylpyrimidin-2-ylamino]-Nmethyl-*N*-(1,2,2,6,6-pentamethyl-piperidin-4-yl)-benzamid;
(±)-4-[4-((1R*,S*)-2-Isopropylcarbamoyl-cyclopentylamino)-5-trifluommethyl-pyrimidin-2-ylamino]-*N*-methyl-N-(1-methyl-piperidin-4-yl)-benzamid;
4-[4-((1R,2S)-2-Isopropylcarbmnoyl-cyclopentylamino)-5-trifluoromethylpyrimidin-2-ylamino]-*N*-methyl-*N*-(1-methyl-piperidin-4-yl)-benzamid;
4-[4-((1S,2R)-2-Isopropylcarbamoyl-cyclopentylamino)-5-trifluoromethylpyrimidin-2-ylamino]-*N*-methyl-*N*-(1-methyl-piperidin-4-yl)-benzamid;
*N*-(1-Ethyl-piperidin-4-yl)-[4-[4-((1R,2S)-2-isopropylcarbamoylcyclopentylamino)-5-trifluoromethyl-pyrimidin-2-ylamino]-*N*-methylbenzamid;
*N*-Isopropyl-4-[4-((1R,2S)-2-isopropylcarbamoyl-cyclopcnlylamino)-5-trifluoromethyl-pyrimidin-2-ylamino]-*N*-(1-methyl-piperidin-4-yl) benzamid;
(*)-*N*-Cyclohexyl-4-[4-((1R*,2S*)-2-isopmpyicarbamoyl - cyclopentylamino)-5-trifluommethyl-pyrimidin-2-ylamino]-*N*-methylbenzamid;
4-[4-((1R,2S)-2-Isopropylcarbamoyl-cyclopentylamino)-5-trilluoromethylpyrimidin-2-ylamino]-*N*-methyl-*N*-(1-methyl-pyrrolidin-3-yl)benzamid;
4-[4-((1R,2S)-2-Ethylcarbamoyl-cyclopentylamino)-5-trifluoromethyl-Pyrimidin-2-ylamino]-*N*-methyl-*N*-(1-methyl-piperidin-4-yl)-benzamid;
4-[4-((1R,2S)-2-Cyclopropylcarbamoyl-cyclopentylamino)-5-trifluoromethyl-pyrimidin-2-ylaminno]-*N*-(1-methyl-piperidin-4-yl)-benzamid;,
4-{4-[(1R*,2S*)-2-((R)-2-Hydroxy-1-methyl-ethylcarbamoyl)-cyclopentylamino]-5-trifluormethyl-pyrirnidin-2-ylamino}-*N*-methyl-*N*-(1-methyl-piperidin-4-yl)-benzamid;
(±)-4-[4-((1R*,2S*)-2-Isobutylcarbamoyl-cyclopentylamino)-5-trifluoromethyl-pyrimidin-2-ylaminol-*N*-methyl-*N*-(1-methyl-piperidin-4-yl)-benzamid;
(±)-4-{4-[(1R*,2S*)-2-(2-Ethyl-butylcarbamoyl)-cyclopentylamino]-5-trifluorornethyl-pyrirnidin-2-ylamirto}-*N*-methyl-*N*-(1-methyl-piperidin-4-yl)-benzamid;
(±)-4-{4-[(1R*,2S*)-2-(Cyclohexylmethyl-carbamoyl)-cyclopentylamino]-5-trifluoromethyl-pyrimidin-2-ylamino]-*N*-methyl-*N*-(1-methyl-piperidin-4-yl)-benzamid;
4-(4-[(1R,2S)-2-(S)-2-Hydroxy-1-methyl-ethylcarbamoyl)-cyclopentylaminoJ-5-t,rifluoromethyl-pyrimidin-2-ylamino}-*N*-methyl-*N*-(1-methyl-piperidin-4-yl)-benzamid;
(±)-*N*-Methyl-*N*-(1-methyl-piperidin-4-yl)-4-{5-trifluoromethyl-4-[(1R*,2S*)-2-(3,3,3-trifluoro-propylcarbamoyl)-cyclopentylamino]-pyrimidin-2-ylamino]-benzamid;
(±)-4-[4-((1R*,2S*)-2-Cyclopropylcarbamoyl-yclopentylamino)-5-triftuoromethyl-pyrimidin-2-ylamin]-*N*-methyl-*N*-(1-methyl-piperidin-4-yl)-benzamid;
4-[4-{(1R,25)-2-Cyclopropylcarbamoyl-cyclopentylamino)-5-trifluoromethyl-pyrimidin-2-ylamino]-*N*-methyl-*N*-{1-methyl-piperidin-4-yl)-benzamid;
(±)- (2S*,3R*)-3-(2-{4-[Methyl-(1-methyl-piperidin-4-yl)-carbamoyl]-phenylamino}-5-trifluoromethyl-pyrimidin-4-ylamino)-bicyclo[2.2.2]octan-2-carbonsäure- isopropylamid;
(±)- (2S*,3R*)-3-(2-{4-[Methyl-(1-methyl-piperidin-4-yl)-carbamoyl]phenylamino)-5-trifluoromethyl-pyrimidin-4-ylamino)bicyclo[2.2.2]octan-2-carbonsaure- cyclopentylamid;
4-[4-(3-Isopropylcarbamoyl-cyclobutylamino)-5-trifluoromethyl-pyrimidin-2-ylamino]-N-methyl-*N*-(1-methyl-piperidin-4-yl)-benzamid;
4-[4-(3-Ethylcarbamoyl-cyclobutylamino)-5-trifluoromethyl-pyrimidin-2-ylamino]-*N*-methyl-*N*-(1-methyl-piperidin-4-yl)-benzamid;
(±)-(2S*,3R*)-3-(2-{4-[Methyl-(1-methyl-piperidin-4-yl)-carbamoyl]-phenylamino}-5-trifluorometlhyl-pyrimidin-4-ylamino)bicyclo[2.2.2]octane-2-carbonsäure-cyclopropylamid;
(±)-(2S* ,3R*)-3-(2-{ 4-[Methyl-(1-methyl-piperidin-4-yl)-carbamoyl]-phenylaminol-5-trifluoromethyl-pyrimidin-4-ylamino)-bicyclo[2.2.2]octan-2-carbonsäureethylamid;
*N*-Meihyl-4-{4-[3-(1-methyl-cyclopropylcarbamoyl)-cyclobutylamino]-5-trifluoromethyl-pyrimidin-2-ylamino}-*N*-(1-methyl-piperidin-4-yl)-benzamid;
4-[4-(3-Cyclopropylcarbamoyl-cyclobutylamino)-5-trifluoromethylpyrimidin-2-ylamino]-*N*-methyl-*N*-(1-methyl-piperidin-4-yl)-benzamid;
*N*-Methyl-4-[4-(3-methylcarbamoyl-cyclobutylamino)-5-trifluoromethylpyrimidin-2-ylammo]-*N*-(1-methyl-piperidin-4-yl)-benzamid;
*N*-Methyl-4-[4-((1R,2S)-2-methylcarbamoyl-cyclopentylamino)-5-triflufloromethyl-pyrimidin-2-ylamino]-*N*-(1-methyl-piyridin-4-yl)-benzamid;
(±)-4-[4-({1R*,2S*)-2-*tert*-Butylcarbamoyl-cyclopentylamino)-5-trifluoromethyl-pyrimidin-2-ylamino]-*N*-methyl-*N*-methyl-piperidin-1-yl)-benzamid;
(±)-*N*-Methyl-4-{4-[(1R*,2S*)-2-(1-methyl-cyclopropylcarbamoyl)-cyclopentylamino]-5-trifluoromethyl-pyrirnidin-2-ylamino}-*N*-(1-methylpiperidin-4-yl)-benzamid;
*N*-Methyl-4-{4-[(1R,2S)-2-(1-methyl-cyclopropylcarbamoyl)-cyclopentylamino]-5-trifluoromcthyl-pyrimidin-2-ylamino}-*N*-(1-methylpiperidin-4-yl)-benzamid;
(±)-4-{4-[(1R*,2S*)-2-(2-Hydroxyl-1,1-dimethyl-ethylcarbamoyl}cyclopentylamino]-5-trifluoromethyl-pyrimidin-2-ylamino}-*N*-methyl-*N*-(1-methyl-piperidin-4-yl)-benzamid;
(±)-4-{4-[(1R*,2S*)-2-(Azetidin-1-carbonyl)-cyclopentylamino]-5-trifluoromethyl-pyrimidin-2-ylamino}-*N*-methyl-*N*-(1-methyl-piperidin-4-yl)-benzamid;
(±)-*N*-Methyl-*N*-(1-methyl-piperidin-4-yl)-4-{4-[(1R*,2S*)-2-(piperidin-1-carbonyl)-cyclopenlylamino]-5-trifluoromethyl-pyrimidin-2-ylamino}benzamid;
(±)-*N*-Methyl-N-(1-methyl-piperidin-4-yl)-4-{4-[(1R*,25*)-2-(morpholine-4-carbonyl)-cyclopentylamino]-5-trifluoromethyl-pyrimidin-2-ylamino}benzamid;
(±)-*N*-Methyl-4-{4-[(1R*,2S*)-2-(4-methyl-piperazine-1-carbonyl)-cyclopentylamino]-5-trifluoromethyl-pyrimidin-2-ylamino)-*N*-(1-methylpiperidin-4-yl)-benzamid;
(+)-*N*-Me+yl-*N*-(1-methyl-pipiridin-4-yl)-4-(4-[(1R*,2S*)-2-(thiomorpholine-4-carbonyl)-cyclopentylamino]-5-trifluoromethylpyrimidin-2-ylamino} -benzamid;
(±)-(1S*,2R*)-2-(2-{4-[Methyl-(1-methyl-piperidin-4-yl)-carbamoyl]-phenylamino}-5-trifluoromethyl-pyrimidin-4-ylamino)-cyclopentanecarbonsäure methyl ester,
(±)-4-{4-[(1R*,2S*)-2-((2R,6S)-2,6-Dimethyl-morpholine-4-carbonylcyclopenlylamino]-5-trifluoromethyl-pyrimidin-2-ylamino-*N*-methyl-*N*-(1-methyl-piperidin-4-yl-benzamid;
4-{4-[(1R,3S)-3-(Cyclopropylmethyl-carbamoyl)-cyclopentylamino-5-trifluoromethyl-pyrimidin-2-ylamino}-*N*-methyl-*N*-(1-methyl-piperidin-4-yl)-benzamid;
4-[4-((1R,3S)3-Benzylcarbamoyl-cyclopentylamino)-5-trifluoromethylpyrimidin-2-ylamino]-*N* methyl-*N*-(1-methyl-piperidin-4-yl)-benzamid;
4-[4-((1R,3S)-3-Cyclopentylcarbamoyl-cyclopentylamino)-5-trifluomethyl-pyrimidin-2-ylamino]-*N*-methyl-*N*-(1-methyl-piperidin-4-yl)-benzamid;
4-{4-[(1R,3S)-3-(Cyanomethyl-carbamoyl)-cyclopentylamino]-5-trifluoromethyl-pyrimidin-2-ylamino}-*N*-methyl-*N*-(1-methyl-piperidin-4-yl)-benzamid;
4-{4-[(1R,3S)-3-(3-Dimethylamino-propylcarbamoyl)-cyclopentylamino]-5-trifluoromethyl-pyrimidin-2-ylamino}-*N*-methyl-*N*-(1-methyl-piperidin-4-yl)-benzamid;
4-{4-[(1R,3S)-3-(2-Dimethylamino-ethylcarbamoyl)-cyclopentylamino]-5-trifluoromethyl-pyrimidin-2-ylamino}-*N*-methyl-*N*-(1-methyl-piperidin-4-yl)-benzamid;
4-(4-[(1R,2S)-2-(4-Dimethylamino-butylcarbamoyl)-cylopentylamino]-5-trifluoromethyl-pyrmidin-2-ylamino)-*N*-methyl-*N*-(1-metyl-piperidin-4-yl)-benzamid;
*N*-Methyl-*N*-(1-methyl-piperidin-4-yl)-4-{4-[(1R,2S)-2-(3-pyrrolidin-1-yl-propylcarbamoyl)-cyclopentylamino]-5-trifluoromethyl-pyrimidin-2-ylamino}-benzamid;
*N*-Mechyl-*N*-(1-methyl-piperidin-4-yl)+(4-{1R,2S)-2-[(1-methylpiperidin-4-ylmethyl)-carbamoyl]-cyclopentylamino}-5-trifluoromethylpyrimidin-2-ylamino)-benzamid;
*N*-Methyl-*N*-(1-methyl-piperidin-4-yl)-4-{4-[(1R,2S)-2-(1-methyl-piperidin-4-ylcarbamoyl)-cyclopentylamino]-5-trifluoromethyl-pyrimidin-2-ylamino}-benzamid;
4-(4-[(1R,2S)-2-(3-Dimethylamino-2,2-dimethyl-propylcarbamoyl)-cyclopentylaminol-5-trifluoromcthyl-pyrimidin-2-ylamiaol-*N*-methyl-*N*-(1-methyl-piperidin-4-yl)-benzamid;
*N*-Methyl-*N*-(1-methyl-piperidin-4-yl)-4-{4-[(1R,2S)-2-(3-pyrrolidin-1-yl-cyclobutylcarbamoyl)-cyclopentylaminol-5-trifluoromethyl-pyrimidin-2-ylamino}-benzamid;
*N*-Methyl-*N*-(1-methyl-piperidin-4-yl)-4-{4-[(1R,2S)-2-(4-morpholin-4-yl-cyclohexylcarbamoyl)-cyclapentylamino-5-trifluoromethyl-pyrimidin-2-ylamino}-benzamid;
*N*-Methyl-4-(4-{(1R,2S)-2-[3-(4-methyl-piperazin-1-yl)-propylcarbamoyl]-cyclopentylamino)-5-trifluorvmethyl-pyrimidin-2-ylamino)-*N*-(1-methylpiperidin-4-yl)-benzamid;
(±)-4-[4-((1R*,2S*)-2-Dimethylcarbamoyl-cyclopentylamino)-5-trifluoromethyl-pyrimidin-2-ylaminol-*N*-methyl-*N*-(1-methyl-piperidin-4-yl)-benzamid;
(±)-4-[4((1R*1R*,2S*)-2-Cyclobutylcarbamoyl-cyclopentylamino)-5-trifluoromethyl-pyrimidin-2-ylaminol-*N*-inethyl-*N*-(1-methyl-piperidin-4-yl)-benzamid;
(±)-4-[4-((1R*,2S*)-2-Cyclopentylcarbomoyl-cyclopentylamino)-5-trifluoromethyl-pyrimidin-2-ylamino]-*N*-methyl-*N*-(1-methyl-piperidin-4-yl)-benzamid;
(±)-*N*-Methyl-4-{4-[(1R*,2S*)-2-(1-methyl-cyclopropylcarbamoyl)-cyclohexylamino]-5-triflunromethyl-pyrimidin-2-ylamino}-*N*-(1-methylpiperidin-4-yl)-benzamid;
(±)-4-[4-((1R*,2S*)-2-Cyclopropylcarbamoyl-cyclohexylamino)-5-trifluoromethyl-pyrimidin-2-ylamino]-*N*-methyl-*N*-(1-methyl-piperidine-4-yl)-benzamid;
(±)-4-{4-[(1R*,2S*)-2-(2,2-Difluoro-ethylcarbamoyl)-cyclohexylamino]-5-trifluoromethyl-pyünüdin-2-ylamino)-*N*-methyl-*N*-(methyl-piperidin-4-yl)-benzamid;
(±)-4-[4-((1R*,2S*)-2-Dimethylcarbamoyl-cyclohexylamino)-5-trilluoromethyl-pyrimidin-2-ylamino]-*N*-methyl-*N*-(1-methyl-piperidin-4-yl)-benzamid;
(±)-4-[4-((1R*,2S*)-2-Ethylcarbamoyl-cyclohexylamino)-5-trifluoromethylpyrimidin-2-ylamino}-*N*-methyl-*N*-1-methyl-piperidin-4-yl)-benzamid;
(±)-*N*-Methyl-*N*-(1-methyl-piperidin-4-yl)-4-(4-[(1R*,2S*)-2-(piperidin-1-carbonyl)-cyclohexylamino]-5-trifluoromethyl-pyrimidin-2-ylamino}benzamid;
(±)-*N*-Methyl-*N*-(1-methyl-piperidin-4-yl)-4-{4-[(1R*,2S*)-2-(pyrrolidine-1-carbonyl)-cyclohexylamino]-5-trifluoromethyl-pyrimidin-2-ylamino}benzamid;
(±)-4-[4-((1R*,2S*)-2-Isopropylcarbamoyl-cyclohexylamino)-5-trifluoromethyl-pyrimidin-2-ylamino]-*N*-methyl-*N*-(1-methyl-piperidin-4-yl)-benzamid;
(±)-*N* Methyl-4-[4-((1R*,2S*)-2-methylearbamoyl-cyclohexylamino)-5-trifluoromethyl-pyrimidin-2-ylamnno]-*N*-(1-methyl-piperidin-4-yl)-benzamid;
(±)-4-{4-[(1R*,2S*)-2-(2-Fluoro-ethylcarbamoyl)-cyclohexylamin]-5-trifluoramethyl-pyrimidin-2-ylamino}-*N*-methyl-*N*-{1-methyl-piperidin-4-yl)-benzamid;
*N*-Methyl-*N*-(1-methyl-piperidin-4-yl)-4-{4-[(1R,1R)-2-(tetrahydro-furan-3-ylcarbamoyl)-cydopentylanünol-5-UifluoromctJxyl-pyrirrnidin-2-ylaxnino)-benzamid;
(±)-*N*-Methyl-*N*-(1-methyl-piperidin-4-yl)-4-(4-[(1R*,2S*)-2-(tetrahydropyran-4-ylcarbamoyl)-cyclopentylamino]-5-trifluoromethyl-pyrimidin-2-ylamino}-benzamid;
*N*-Methyl-*N*-(1-methyl-piperidin-4-yl)-4-(4-[(1R,2S)-2-(2,22-trifluoroethylcarbamoyl)-cyclopentylamino]-5-trifluoromethyl-pyrimidin-2-ylamino)-benzamid;
(±)-4-{4-[(1R*,2S*)-2-(2-Dimethylamino-ethylcarbamoyl)-cyclopentylamino]-5-trifluoromethyl-pyrimidin-2-ylamino}-*N*-methyl-*N*-{1-methyl-piperidin-4-yl)-benzamid;
(±)-*N*-Methyl-*N*-(1-methyl-piperidin-4-yl)-4-{4-[(1R*,2S*)-2-(2-pyrrolidin-1-yl-ethylcarbamoyl)-cyclopentylamino]-5-trifluoremethyl-pyrimidin-2-ylamino)}-benzamid;
4-(4-((1R,3S)-3-Isopropylcarbamoyl-cyclopentylamino)-5-trifluoromethylpyrimidin-2-ylammo]-*N-*methyl-*N*-(1-methyl-piperidin-4-yl)-benzamid;
4-[4-((1R,3S)-3-Carbamoyl-cyclopentylamino)-5-trifluoromethylpyrimidin-2-ylamino]-*N*-methyl-*N*-(1-methyl-piperidin-4-yl)-benzamid;
4-[4-((1S,3R)-3-Isopropylcarbamoyl-cyclopentylamino)-5-trifluoromethylpyrimidin-2-ylamino]-*N*-methyl-*N*-(1-methyl-piperidin-4-yl)-benzamid;
4-[4-((1S,3R)-3-Carbamoyl-cyclopentylamino)-5-trifluoromethylpyrimidin-2-ylamino]-*N-*methyl-*N*-(1-methyl-piperidin-4-yl)-benzamid;
(±)-*N*-Methyl-*N*-(1-methyl-pipcridin-4-yl)-4-{4-[(1R*,2S*)-2-(2-morpholin-4-yl-ethylcarbamoyl)-cyclopentylamino]-5-trifluoromethyl-pyrimidin-2-ylamino}-benzamid;
(±)-4-[4-({1R*,2S*)-2-Carbamoyl-cyclobutylamino)-5-trifluoromethylpyrimidin-2-ylamino]-*N*-methyl-*N*-(1-methyl-piperidin-4-yl)-benzamid;
(±)-4-[4-((1R*,6S*)-6-Carbamoyl-cyclohex-3-enylamino)-5-trifluoromethyl-pyrimidin-2-ylamino]-*N*-methyl-*N*-(1-methyl-piperidin-4-yl)-benzamid;
(±)-*N*-Methyl-4-[4-((1R*,2S*)-2-methylcarbamoyl-cyclopentylamino)-5-trifluommethyl-pyrimidin-2-ylamino]-*N*-(1-methyl-piperidin-4-yl)-benzamid;
4-{4-[(1R,3S)-3-(Azetidin-1-carbonyl)-cyclopentylamino]-5-trifluoromethyl-pyrimidin-2-ylamino}-*N*-methyl-*N*-{1-methyl-piperidin-4-yl)-benzamid;
*N*-Methyl-*N*-(1-methyl-piperidin-4-yl)-4-(4-[(1R,3S)-3-piperidin-1-carbonyl)-cyclopentylamino]-5-trifluoromethyl-pyrimidin-2-ylamino}benzamid;
4-(4-[(1R,3S)-3-(2-Methoxy-ethylcarbamoyl)-cyclopentylamino]-5-trifluoromethyl-pyrimidin-2-ylamino}-*N*-methyl-*N*-(1-methyl-piperidin-4-yl}-benzamid;
*N*-Methyl-*N*-(1-methyl-piperidin-4-yl)-4-(4-[(1R,3S)-3-(pyrrolidine-1-carbonyl)-cyclopentylamino]-5-trifluoromethyl-pyrimidin-2-ylamino}benzamid;
4-[4-((1R,3S)-3-Ethylcarbamoyl-cyclopentylamino)-5-trifluoromethylpyrimidin-2-ylamino]-*N*-melhyl-*N*-(1-methyl-piperidin-4-yl)-benzamid;
(±)-(1S*,2R*)2-(2-{4-[Methyl-(1-methyl-piperidin-4-yl)-carbamoyl]-phenylamino }-5-trifluoromethyl-pyrimidin-4-ylamino)-cyclopentanecarbonsäure;
(±)-4-{4-[(1R*,2S*)-2-(Cyanomethyl-carbamoyl)-cyclopentylamino]-5-trifluoromethyl-pyrimidin-2-ylamino}-*N*-methyl-*N*-(1-methyl-piperidin-4-yl)-benzamid ;
(+)-4-{4-[(1R*,2S*)-2-(2-Cyano-ethylcarbamoyl)-cyclopentylamino]-5-trifluoromethyl-pyrimidin-2-ylamino}-*N*-methyl-*N*-(1-methyl-piperidin-4-yl)-benzamid;
(±)-4-{4-[(1R*,2S*)-2-(Carbamoylmethyl-carbamoyl)-cyclopentylamino]-5-trifluoromethyl-pyrimidin-2-ylamino}-*N*-methyl-*N*-(1-methyl-piperidin-4-yl)-benzamid;
*N*-Methyl-*N*-(1-methyl-piperidin-4-yl)-4-(4-[(1R,2S)-2-(3-oxo-piperazin-1-carbonyl)-cyclopentylamino]-5-trifluoromethyl-pyrimidin-2-ylamino}benzamid;
4-(4-{(1R,2S)-2-[(Carbamoyl-cyano-methyl)-carbamoyl]-cyclopentylamino}-5-trifluoromethyl-pyrimidin-2-ylamino)-*N*-methyl-N-(t-methyl-piperidin-4-yl)-benzamid;
4-{4-[(1R,2S)-2-((S)-1-carbamoyl-3-methyl-butylcarbamoyl)-cyclopentylamino]-5-triftuoromethyl-pyrimidin-2-ylamino}-*N*-methyl-N-(1-methyl-piperidin-4-yl)-benzamid;
(±)-4-{4-[(1R*,2S*)-2-(Dimethylcarbamoylmethyl-carbamoyl)-cyclopentylamino]-5-trifluoromethyl-pyrimidin-2-ylamino}-*N*-methyl-*N*-(1-methyl-piperidin-4-yl)-benzamid;
(+)-*N*-Methyl-4-(4-[(1R*,2S*)-2-(memylcarbamoylmethyl-carbamoyl)-cyclopentylamino]-5-trifluoromethyl-pyrimidin-2-ylamino}-*N*-(1-methylpiperidin-4-yl)-benzamid;
(±)-4-{4-[(1R*,2S*)-2-((R)-1-Carbamoyl-3-methyl-butylcarbamoyl)-cyclopentylamino]-5-trifluoromethyl-pyrimidin-2-ylamino}-*N*-methyl-N-(1-methyl-piperidin-4-yl)-benzamid;
(±)-4-{4-{(1R*,2S*)-2-(2-Carbamoyl-ethylcarbamoyl)-cyclopentylamino]-5-trifluomethyl-pyrimidin-2-ylamino)-*N*-methyl-*N*-(1methyl-piperidin-4-yl)-benzamid;
(R)-1-[(1S,2R)-2-(2-{4-[Methyl-(1-methyl-piperidin-4-yl)-carbamoyl]-phenylamino}-5-trifluoromethyl-pyrimidin-4-ylamino)-cyclopentanecarbonyl]-pyrrolidine-2-carbonsäureamid;
(S)-1-{(1S,2R)-2-(2-{4-[Methyl-(1-methyl-piperidin-4-yl)-carbamoyl]-phenylamino}-5-trifluoromethyl-pyrimidin-4-ylamino)-cyclopentancarbonyl]-pyrrolidine-2-carbonsäureamid;
4-(4-[(1R,2S)2-((S)-1-Carbamoyl-ethylcarbamoyl)-cyclopentylamino]-5-trifluoromethyl-pyrimidin-2-ylamino}-*N*-methyl-*N*-(1-methyl-piperidin-4-yl)-benzamid;
4-(4-[(1R2S)-2((R)-1-Carbamoyl-ethylcarbamoyl)-cyclopentylamino]-5-trifluoromethyl-pyrimidin-2-ylaminol-*N*-methyl-*N*-(1-methyl-piperidin-4-yl)-benzamid;
(±)-4-{4-[(1R*,2S*)-2-(2-Amino-2-methyl-propylcarbamoyl)-cyclopentylamino]-5-trifluoromethyl-pyrimidin-2-ylamino}-*N*-methyl-*N*-(1-methyl-piperidin-4-yl)-benzamid;
(±)-*N*-Methyl-*N*-(1-methyl-piperidin-4-yl)-4-{4-[(1R*,2S*)-2-(thiazol-2-ylcarbamoyl)-cyclopentylamino]-5-trifluoromethyl-pyrimidin-2-ylamino)-benzamid,
(±)-4-[4-((1R*,2S*)-2-Amino-cyclohcxylamino)-5-trifluoromethylpyrimidin-2-ylamino]-*N*-methyl-*N*-(1-methyl-piperidin-4-yl)-benzamid-,
(±)-4-[4-((1R*,2S*)-2-Acetylamino-cyclohexylamino]-5-trifluormethylpyrimidin-2-ylamino]-*N*-melhyl-*N*-(1-methyl-piperidin-4-yl)-benzamid;
(±)-4-[4-((1R*,2S*)-2-Methanesulfonylamino-cyclohexylamino)-5-trifluommethyl-pyrimidin-2-ylamino]-*N*-methyl-*N*-(1-methyl-piperidin-4-yl)-benzamid;
(±)-4-(4-((1R*,2S*)-2-Isobutyrylamino-cyclohexylamino)-5-trifluoromethyl-pyrimidin-2-ylamino]-*N*-methyl-*N*-(1-methyl-piperidin-4-yl)-benzamid:
(±)-4-{4-((1R*,2S*)-2-(Cyclopropanecarbonyl-amino)-cyclohexylamino]-5-trifluoromethyl-pyrimidin-2-ylamino)-*N*-methyl-*N*-(1-methyl-piperidin-4-yl)-benzamid;
(±)-N-Methyl-*N*-(1-methyl-piperidin-4-yl)-4-[4-((1R*,2S*)-2-propionylamino-cyclohexylamino)-5-trifluoromethyl-pyridin-2-ylamino]-benzamid;
(±)-4-[4-((1R*,2S*)-2-Ethanesulfonylamino-cyclohexylamino)-5-triflnoromethyl-pyrimidin-2-ylamino]-*N*-methyl-*N*-(1-methyl-piperidin-4-yl)-benzamid;
(±)-4-{4-(1R*,2S*)-2-(3,3-Dimethyl-ureido)-cyclohexylamino]-5-trifluoromethyl-pyrimidin-2-ylamino)-*N*-methyl-*N*-(1-methyl-piperidin-4-yl)-benzamid;
(±)-(1R*,2S*,3R*,4S*)-3-(2-{4-[Methyl-(1-methyl-piperidin-4-yl)-carbamoyl]-phenylamino}-5-trifluoromethyl-pyrimidin-4-ylamino)-bicyclo[2.2.1]hept-5-en-2-carbonsäure;
4-[4-(Adamanwn-2-ylamino)-5-trifluoromethyl-pyrimidin-2-ylamino]-*N-*methyl-*N*-(1-methyl-piperidin-4-yl)-benzamid;
(±)- (1R*,2R*,3S*,4S*)-3(2-(4-[Methyl-(1-methyl-piperidin-4-yl)-carbamoyl]-phenylamino}-5-trifluoromethyl-pyrimidin-4-ylamino)-bicyclo[2.2.1]heptan-2-carbonsäure cyclopropylamid;
(±)-4-[4-((1R*,2S*)-2-Isobutylamino-cyclopentylamino)-5-trifluoromethyl-pyrinüdin-2-ylamino]-*N*-methyl-*N*-(1-methyl-piperidin-4-yl)-benzamid;
(±)-*N*-Methyl-4-{4-[{1R*,2S*)-2-(3-methyl-butyrylamino)-cyclopentylamino]-5-trifluromethyl-pyrimidin-2-ylaminol-*N*-(1-methylpiperidin-4-yl)-benzamid,
(±)-*N*-Methyl-*N*-(1-methyl-piperidin-4-yl)-4-[4-((1R*,2S*)-2-propionylamino-cyclopentylamino)-5-trifluoromethyl-pyrimidin-2-ylamino]-benzamid;
(±)-4-{4-[(1R*,2S*)-2-(2-Methoxy-acetylamino)-cyclopentylamino]-5-trifluoromethyl-pyrimidin-2-ylamino}-*N*-methyl-*N*-(1-methyl-piperidin-4-yl)-benzamid;
(±)-4-[4-((1R*,2S*)-2-Amino-cyclopentylamino)-5-trifluoromethylpyrimidin-2-ylamino]-*N*-methyl-*N*-(1-methyl-piperidin-4-yl)-benzamid;
*N*-Methyl-*N*-(1-methyl-piperidin-4-yl)-4-(4-{(1R,2S)-2-[2-(1-methylpyrrolidin-2-yl)-ethylcarbamoyl]-cyclopentylamino}-5-trifluoromethylpyrimidin-2-ylamino)-benzamid;
4-{4-[(1R,2S)-2-(2,2-Difluoro-ethylcarbamoyl)-cyclopentylamino]-5-trifluoro-methyl-pyrimidin-2-ylamino)-*N*-methyl-*N*-(1-methyl-piperidin4-yl)-benzamid;
4-{4-[(1R,2S)-2-(2-Fluoro-ethylcarbamoyl)-cyclopentylamino]-5-trifluoromethyl-pyridin-2-ylamino)-*N*-methyl-*N*-(1-methyl-piperidin-4-yl)benzamid;
2-Methoxy-*N*-methyl-4-[4-((1R,2S)-2-methylcarbanoyl-oyclopentylamino)-5-trifluoroinethyl-pyrimidin-2-ylamino)-*N*-(1-methyl-piperidin-4-yl)-benzamid;
4-[4-((1R,2S)-2-Isopropylcarbamoyl-cyclopentylamino)-5-trifluoromethylpyrimidin-2-ylamino]-2-methoxy-*N*-methyl-*N*-(1-methyl-piperidin-4-yl)-benzamid;
4-[4-((1R,2S)-2-Cyclopropylcarbamoyl-cyclopentylamino)-5-trifluoromethyl-pyrimidin-2-ylamino]-2-methoxy-*N*-methyl-*N*-(1-methylpiperidin-4-yl)-benzamid;
4-[4-((1R,2S)-2-Isopropylcarbamoyl-cyclopentylamino)-5-trifluoromethylpyrimidin-2-ylamino]-*N*-methyl-*N*-(1-methyl-piperidin-4-yl)-2-propoxybenzamid;
2-Ethoxy-4-[4-(1R,3S)-2-isopropylcarbamoyl-cyclopentylamino)-5-trifluomethyl-pyrimidin-2-ylamino]-*N*-methyl-*N*-(1-methyl-piperidin-4-yl)-benzamid;
2-Isopropoxy-4-[4-((1R,2S)-2-isopropylcarbamoyl-cyclopentylamino)-5-trifluoromethyl-pyrimidin-2-ylamino]-*N*-methyl-*N*-(1-methyl-piperidin-4-yl)-benzamid;
4-[4-((1R,2S)-2-Isopropylcarbamoyl-cyclopentylamino)-5-trifluomethylpyrimidin-2-ylaminol-*N*-methyl-*N*-(1-methyl-piperidin-4-yl)-2-prop-2-ynyloxy-benzamid;
(±)-4-[4-((1R*,1S*)-2-Isopropylcarbamoyl-cyclobutylamino)-5-trifluormethyl-pyrituidin-2-ylamino]-*N*-methyl-*N*-(1-methyl-piperidin-4-yl)-benzamid;
(±)-4-{5-Bromo-4-[(1R*,2S*)-2-(1-methyl-cyclopropylcarbamoyl)-cyclopentylamino]-pyrimidin-2-ylamino}-*N*-methyl-*N*-(1-methyl-piperidin-4-yl)-benzamid;
(±)-4-[5-Bromo-4-((1R*,2S*)-2-Isopropylcarbamoyl-cyclopentylamino)-pyrimidui-2-ylamino]-*N*-methyl-*N*-(1-methyl-piperidin-4-yl)-benzamid;
(±)-4-[5-Brom-4-[(1R*,2S*)-2-cyclopropylcarbamoyl)-cyclopentylamino)-pyrimidin-2-ylamino]-N-methyl-*N*-(1-mohyl-piperidin-4-yl)-benzamid;
(±)-4-{5-Bromo-4-∟(1R*,2S*)-2-(2-cyano-ethylcarbamoyl)-cyclopentylamino]-pyrirnidin-2-ylamino)-*N*-methyl-*N*-(1-methyl-piperidin-4-yl)-benzamid,
(±)-4-[5-Bromo-4-((1R*,2S*)-2-cyclopentylcarbamoyl-cyclopentylamino)-pyrimidin-2-ylamino]-*N*-methyl-*N*-(1-methyl-piperidin-4-yl)-benzamid;
4-(5-Bromo-4-[(1R,2S)-2-(pyrrolidine-1-carbonyl)-cyclopentylamino]-pyrimidin-2-ylamino)-*N*-methyl-*N*-(1-methyl-piperidin-4-yl)-benzamide;
4-{4-[(1R,2S)-2-(Azetidin-1-carbonyl)-cyclopentylamino]-5-bromopyrimidin-2-ylamino}-*N*-methyl-*N*-(1-methyl-piperidin-4-yl)benzamid;
(±)-4-15-Bromo-4-((1R*,2S*)-2-cyclobutylcarbamoyl-cyclopentylamino)-pyrimidin-2-ylmino]-N-methyl-*N*-(1-methyl-piperidin-4-yl)-benzamid;
4-[4-((1R,2S)-2-Isopropylcarbamoyl-cyclopentylamino)-5-methylpyrimidin-2-ylamino]-*N*-methyl-*N*-(1-methyl-piperidin-4-yl)-benzamid;
4-[5-Bromo-4-((1R,2S)-2-isopropylcarbamoyl-cyclopentylamino)-pyrimidin-2-ylamino]-*N*-methyl-*N*-(1-methyl-piperidin-4-yl)-benzamid;
4-[5-Chloro-4-((1R,2S)-2-isopropylcarbamoyl-cyclopentylamino)-pyrimidin-2-ylamino]-*N*-methyl-*N*-(1-methyl-piperidin-4-yl)-bezamid.

2. Verbindungen, oder deren pharmazeutisch wirksamen Salze, nach Anspruch 1 zur Verwendung als Arzneimittel.

3. Verbindungen, oder deren pharmazeutisch wirksamen Salze, nach Anspruch 1 zur Herstellung eines Arzneimittels mit antiproliferativer Wirkung.

4. Pharmazeutische Zubereitungen, enthaltend als Wirkstoff eine oder mehrere Verbindungen der allgemeinen Formel (1) gemäß Anspruch 1 oder deren pharmazeutisch wirksamen Salze gegebenenfalls in Kombination mit üblichen Hilfs- und/oder Trägerstoffen.

5. Verwendung von Verbindungen der allgemeinen Formel (1) gemäß Anspruch 1 zur Herstellung eines Arzneimittels zur Behandlung und/oder Prävention von Krebs, Infektionen. Entzündungs- und Autoimmunerkrankungen.

6. Pharmazeutische Präparation umfassend eine Verbindung der allgemeinen Formel (1) gemäß Anspruch 1 und mindestens eine weitere zytostatische oder zytotoxische, von Formel (1) verschiedene Wirksubstanz, gegebenenfalls in Form ihrer Tautomeren, ihrer Racemate, ihrer Enantiomere, ihrer Diastereomere und ihrer Gemische, sowie gegebenenfalls ihrer pharmazeutisch wirksamen Salze.

## Claims

1. Compounds of general formula (1), 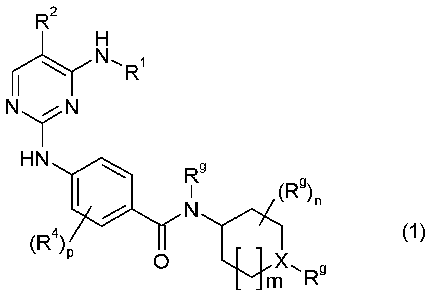 selected from the group consisting of
4-[4-((1R,2S)-2-isopropylcarbamoyl-cyclopentylamino)-5-trifluoromethylpyrimidin-2-yl-amino]-*N*-methyl-*N*-(1,2,2,6,6-pentamethyl-piperidin-4-yl)-benzamide
(±)-4-[4-((1R*,2S*)-2-isopropylcarbamoyl-cyclopentylamino)-5-trifluoromethylpyrimidin-2-yl-amino]-*N*-methyl-*N*-(1-methyl-piperidin-4-yl)-benzamide
4-[4-((1R,2S)-2-isopropylcarbamoyl-cyclopentylamino)-5-trifluoromethylpyrimidin-2-yl-amino]-*N*-methyl-*N*-(1-methyl-piperidin-4-yl)-benzamide
4-[4-((1S,2R)-2-isopropylcarbamoyl-cyclopentylamino)-5-trifluoromethylpyrimidin-2-yl-amino]-*N*-methyl-*N*-(1-methyl-piperidin-4-yl)-benzamide
*N*-(1-ethyl-piperidin-4-yl)-4-[4-((1R,2S)-2-isopropylcarbamoylcyclopentylamino)-5-trifluoromethyl-pyrimidin-2-yl-amino]-*N*-methylbenzamide
*N*-isopropyl-4-[4-((1R,2S)-2-isopropylcarbamoyl-cyclopentylamino)-5-trifluoromethyl-pyrimidin-2-yl-amino]-*N*-(1-methyl-piperidin-4-yl)-benzamide
(±)-*N*-cyclohexyl-4-[4-((1R*,2S*)-2-isopropylcarbamoyl-cyclopentylamino)-5-trifluoromethyl-pyrimidin-2-yl-amino]-*N*-methyl-benzamide
4-[4-((1R,2S)-2-isopropylcarbamoyl-cyclopentylamino)-5-trifluoromethylpyrimidin-2-yl-amino]-*N*-methyl-*N*-(1-methyl-pyrrolidine-3-yl)-benzamide
4-[4-((1R,2S)-2-ethylcarbamoyl-cyclopentylamino)-5-trifluoromethylpyrimidin-2-yl-amino]-*N*-methyl-*N*-(1-methyl-piperidin-4-yl)-benzamide
4-[4-((1R,2S)-2-cyclopropylcarbamoyl-cyclopentylamino)-5-trifluoromethylpyrimidin-2-yl-amino]-*N*-(1-methyl-piperidin-4-yl)-benzamide
4-{4-[(1R*,2S*)-2-((R)-2-hydroxy-1-methyl-ethylcarbamoyl)-cyclopentylamino]-5-trifluoromethyl-pyrimidin-2-yl-amino}-*N*-methyl-*N*-(1-methyl-piperidin-4-yl)-benzamide
(±)-4-[4-((1R*,2S*)-2-isobutylcarbamoyl-cyclopentylamino)-5-trifluoromethylpyrimidin-2-yl-amino]-*N*-methyl-*N*-(1-methyl-piperidin-4-yl)-benzamide
(±)-4-{4-[(1R*,2S*)-2-(2-ethyl-butylcarbamoyl)-cyclopentylamino]-5-trifluoromethyl-pyrimidin-2-yl-amino}-*N*-methyl-*N*-(1-methyl-piperidin-4-yl)-benzamide
(±)-4-{4-[(1R*,2S*)-2-(cyclohexylmethyl-carbamoyl)-cyclopentylamino]-5-trifluoromethyl-pyrimidin-2-yl-amino}-*N*-methyl-*N*-(1-methyl-piperidin-4-yl)-benzamide
4-{4-[(1R,2S)-2-((S)-2-hydroxy-1-methyl-ethylcarbamoyl)-cyclopentylamino]-5-trifluoromethyl-pyrimidin-2-yl-amino}-*N*-methyl-*N*-(1-methyl-piperidin-4-yl)-benzamide
(±)-*N*-methyl-*N*-(1-methyl-piperidin-4-yl)-4-{5-trifluoromethyl-4-[(1R*,2S*)-2-(3,3,3-trifluoro-propylcarbamoyl)-cyclopentylamino]-pyrimidin-2-yl-amino}benzamide
(±)-4-[4-((1R*,2S*)-2-cyclopropylcarbamoyl-cyclopentylamino)-5-trifluoromethyl-pyrimidin-2-yl-amino]-*N*-methyl-*N*-(1-methyl-piperidin-4-yl)-benzamide
4-[4-((1R,2S)-2-cyclopropylcarbamoyl-cyclopentylamino)-5-trifluoromethylpyrimidin-2-yl-amino]-*N*-methyl-*N*-(1-methyl-piperidin-4-yl)-benzamide
(±)- (2S*,3R*)-3-(2-{4-[methyl-(1-methyl-piperidin-4-yl)-carbamoyl]-phenylamino}-5-trifluoromethyl-pyrimidin-4-yl-amino)-bicyclo[2.2.2]octan-2-carboxylic acid-isopropylamide
(±)- (2S*,3R*)-3-(2-{4-[methyl-(1-methyl-piperidin-4-yl)-carbamoyl]-phenylamino}-5-trifluoromethyl-pyrimidin-4-yl-amino)-bicyclo[2.2.2]octan-2-carboxylic acid-cyclopentylamide
4-[4-(3-isopropylcarbamoyl-cyclobutylamino)-5-trifluoromethyl-pyrimidin-2-yl-amino]-*N*-methyl-*N*-(1-methyl-piperidin-4-yl)-benzamide
4-[4-(3-ethylcarbamoyl-cyclobutylamino)-5-trifluoromethyl-pyrimidin-2-yl-amino]-*N*-methyl-*N*-(1-methyl-piperidin-4-yl)-benzamide
(±)- (2S*,3R*)-3-(2-{4-[methyl-(1-methyl-piperidin-4-yl)-carbamoyl]-phenylamino}-5-trifluoromethyl-pyrimidin-4-yl-amino)-bicyclo[2.2.2]octane-2-carboxylic acid-cyclopropylamide
(±)- (2S*,3R*)-3-(2-{4-[methyl-(1-methyl-piperidin-4-yl)-carbamoyl]-phenylamino}-5-trifluoromethyl-pyrimidin-4-yl-amino)-bicyclo[2.2.2]octan-2-carboxylic acid-ethylamide
*N*-methyl-4-{4-[3-(1-methyl-cyclopropylcarbamoyl)-cyclobutylamino]-5-trifluoromethyl-pyrimidin-2-yl-amino}-*N*-(1-methyl-piperidin-4-yl)-benzamide
4-[4-(3-cyclopropylcarbamoyl-cyclobutylamino)-5-trifluoromethyl-pyrimidin-2-yl-amino]-*N*-methyl-*N*-(1-methyl-piperidin-4-yl)-benzamide
*N*-methyl-4-[4-(3-methylcarbamoyl-cyclobutylamino)-5-trifluoromethylpyrimidin-2-yl-amino]-*N*-(1-methyl-piperidin-4-yl)-benzamide
*N*-methyl-4-[4-((1R,2S)-2-methylcarbamoyl-cyclopentylamino)-5-trifluoromethyl-pyrimidin-2-ylamino]-*N*-(1-methyl-piperidin-4-yl)-benzamide
(±)-4-[4-((1R*,2S*)-2-*tert*-butylcarbamoyl-cyclopentylamino)-5-trifluoromethylpyrimidin-2-yl-amino]-*N*-methyl-*N*-(1-methyl-piperidin-4-yl)-benzamide
(±)-*N*-methyl-4-{4-[(1R*,2S*)-2-(1-methyl-cyclopropylcarbamoyl)-cyclopentylamino]-5-trifluoromethyl-pyrimidin-2-yl-amino}-*N*-(1-methylpiperidin-4-yl)-benzamide
*N*-methyl-4-{4-[(1R,2S)-2-(1-methyl-cyclopropylcarbamoyl)-cyclopentylamino]-5-trifluoromethyl-pyrimidin-2-yl-amino}-*N*-(1-methylpiperidin-4-yl)-benzamide
(±)-4-{4-[(1R*,2S*)-2-(2-hydroxy-1,1-dimethyl-ethylcarbamoyl)-cyclopentylamino]-5-trifluoromethyl-pyrimidin-2-yl-amino}-*N*-methyl-*N*-(1-methyl-piperidin-4-yl)-benzamide
(±)-4-{4-[(1R*,2S*)-2-(azetidin-1-carbonyl)-cyclopentylamino]-5-trifluoromethyl-pyrimidin-2-yl-amino}-*N*-methyl-*N*-(1-methyl-piperidin-4-yl)-benzamide
(±)-*N*-methyl-*N*-(1-methyl-piperidin-4-yl)-4-{4-[(1R*,2S*)-2-(piperidine-1-carbonyl)-cyclopentylamino]-5-trifluoromethyl-pyrimidin-2-yl-amino}benzamide
(±)-*N*-methyl-*N*-(1-methyl-piperidin-4-yl)-4-{4-[(1R*,2S*)-2-(morpholine-4-carbonyl)-cyclopentylamino]-5-trifluoromethyl-pyrimidin-2-yl-amino}benzamide
(±)-*N*-methyl-4-{4-[(1R*,2S*)-2-(4-methyl-piperazine-1-carbonyl)-cyclopentylamino]-5-trifluoromethyl-pyrimidin-2-yl-amino}-*N*-(1-methylpiperidin-4-yl)-benzamide
(±)-*N*-methyl-*N*-(1-methyl-piperidin-4-yl)-4-{4-[(1R*,2S*)-2-(thiomorpholine-4-carbonyl)-cyclopentylamino]-5-trifluoromethyl-pyrimidin-2-yl-amino}benzamide
methyl (±)-(1S*,2R*)-2-(2-{4-[methyl-(1-methyl-piperidin-4-yl)-carbamoyl]-phenylamino}-5-trifluoromethyl-pyrimidin-4-yl-amino)-cyclopentane carboxylate
(±)-4-{4-[(1R*,2S*)-2-((2R,6S)-2,6-dimethyl-morpholine-4-carbonyl)-cyclopentylamino]-5-trifluoromethyl-pyrimidin-2-yl-amino}-*N*-methyl-*N*-(1-methyl-piperidin-4-yl)-benzamide
4-{4-[(1R,3S)-3-(cyclopropylmethyl-carbamoyl)-cyclopentylamino]-5-trifluoromethyl-pyrimidin-2-yl-amino}-*N*-methyl-*N*-(1-methyl-piperidin-4-yl)-benzamide
4-[4-((1R,3S)-3-benzylcarbamoyl-cyclopentylamino)-5-trifluoromethylpyrimidin-2-yl-amino]-*N*-methyl-*N*-(1-methyl-piperidin-4-yl)-benzamide
4-[4-((1R,3S)-3-cyclopentylcarbamoyl-cyclopentylamino)-5-trifluoromethylpyrimidin-2-yl-amino]-*N*-methyl-*N*-(1-methyl-piperidin-4-yl)-benzamide
4-{4-[(1R,3S)-3-(cyanomethyl-carbamoyl)-cyclopentylamino]-5-trifluoromethyl-pyrimidin-2-yl-amino}-*N*-methyl-*N*-(1-methyl-piperidin-4-yl)-benzamide
4-{4-[(1R,3S)-3-(3-dimethylamino-propylcarbamoyl)-cyclopentylamino]-5-trifluoromethyl-pyrimidin-2-yl-amino}-*N*-methyl-*N*-(1-methyl-piperidin-4-yl)-benzamide
4-{4-[(1R,3S)-3-(2-dimethylamino-ethylcarbamoyl)-cyclopentylamino]-5-trifluoromethyl-pyrimidin-2-yl-amino}-*N*-methyl-*N*-(1-methyl-piperidin-4-yl)-benzamide
4-{4-[(1R,2S)-2-(4-dimethylamino-butylcarbamoyl)-cyclopentylamino]-5-trifluoromethyl-pyrimidin-2-yl-amino}-*N*-methyl-*N*-(1-methyl-piperidin-4-yl)-benzamide
*N*-methyl-*N*-(1-methyl-piperidin-4-yl)-4-{4-[(1R,2S)-2-(3-pyrrolidine-1-yl-propylcarbamoyl)-cyclopentylamino]-5-trifluoromethyl-pyrimidin-2-ylamino}benzamide
*N*-methyl-*N*-(1-methyl-piperidin-4-yl)-4-(4-{(1R,2S)-2-[(1-methyl-piperidin-4-yl-methyl)-carbamoyl]-cyclopentylamino}-5-trifluoromethyl-pyrimidin-2-yl-amino)-benzamide
*N*-methyl-*N*-(1-methyl-piperidin-4-yl)-4-{4-[(1R,2S)-2-(1-methyl-piperidin-4-yl-carbamoyl)-cyclopentylamino]-5-trifluoromethyl-pyrimidin-2-yl-amino}benzamide
4-{4-[(1R,2S)-2-(3-dimethylamino-2,2-dimethyl-propylcarbamoyl)-cyclopentylamino]-5-trifluoromethyl-pyrimidin-2-yl-amino}-*N*-methyl-*N*-(1-methyl-piperidin-4-yl)-benzamide
*N*-methyl-*N*-(1-methyl-piperidin-4-yl)-4-{4-[(1R,2S)-2-(3-pyrrolidin-1-yl-cyclobutylcarbamoyl)-cyclopentylamino]-5-trifluoromethyl-pyrimidin-2-yl-amino}-benzamide
*N*-methyl-*N*-(1-methyl-piperidin-4-yl)-4-{4-[(1R,2S)-2-(4-morpholin-4-yl-cyclohexylcarbamoyl)-cyclopentylamino]-5-trifluoromethyl-pyrimidin-2-ylaminol-benzamide
*N*-methyl-4-(4-{(1R,2S)-2-[3-(4-methyl-piperazine-1-yl)-propylcarbamoyl]-cyclopentylamino}-5-trifluoromethyl-pyrimidin-2-yl-amino)-*N*-(1-methylpiperidin-4-yl)-benzamide
(±)-4-[4-((1R*,2S*)-2-dimethylcarbamoyl-cyclopentylamino)-5-trifluoromethylpyrimidin-2-yl-amino]-*N*-methyl-*N*-(1-methyl-piperidin-4-yl)-benzamide
(±)-4-[4-((1R*,2S*)-2-cyclobutylcarbamoyl-cyclopentylamino)-5-trifluoromethyl-pyrimidin-2-yl-amino]-*N*-methyl-*N*-(1-methyl-piperidin-4-yl)-benzamide
(±)-4-[4-((1R*,2S*)-2-cyclopentylcarbamoyl-cyclopentylamino)-5-trifluoromethyl-pyrimidin-2-yl-amino]-*N*-methyl-*N*-(1-methyl-piperidin-4-yl)-benzamide
(±)-*N*-methyl-4-{4-[(1R*,2S*)-2-(1-methyl-cyclopropylcarbamoyl)-cyclohexylamino]-5-trifluoromethyl-pyrimidin-2-yl-amino}-*N*-(1-methylpiperidin-4-yl)-benzamide
(±)-4-[4-((1R*,2S*)-2-cyclopropylcarbamoyl-cyclohexylamino)-5-trifluoromethyl-pyrimidin-2-yl-amino]-*N*-methyl-*N*-(1-methyl-piperidin-4-yl)-benzamide
(±)-4-{4-[(1R*,2S*)-2-(2,2-difluoro-ethylcarbamoyl)-cyclohexylamino]-5-trifluoromethyl-pyrimidin-2-yl-amino}-*N*-methyl-*N*-(1-methyl-piperidin-4-yl)-benzamide
(±)-4-[4-((1R*,2S*)-2-dimethylcarbamoyl-cyclohexylamino)-5-trifluoromethylpyrimidin-2-yl-amino]-*N*-methyl-*N*-(1-methyl-piperidin-4-yl)-benzamide
(±)-4-[4-((1R*,2S*)-2-ethylcarbamoyl-cyclohexylamino)-5-trifluoromethylpyrimidin-2-yl-amino]-*N*-methyl-*N*-(1-methyl-piperidin-4-yl)-benzamide
(±)-*N*-methyl-*N*-(1-methyl-piperidin-4-yl)-4-{4-[(1R*,2S*)-2-(piperidine-1-carbonyl)-cyclohexylamino]-5-trifluoromethyl-pyrimidin-2-yl-amino}benzamide
(±)-*N*-methyl-*N*-(1-ethyl-piperidin-4-yl)-4-{4-[(1R*,2S*)-2-(pyrrolidine-1-carbonyl)-cyclohexylamino]-5-trifluoromethyl-pyrimidin-2-yl-amino}benzamide
(±)-4-[4-((1R*,2S*)-2-isopropylcarbamoyl-cyclohexylamino)-5-trifluoromethylpyrimidin-2-yl-amino]-*N*-methyl-*N*-(1-methyl-piperidin-4-yl)-benzamide
(±)-*N*-methyl-4-[4-((1R*,2S*)-2-methylcarbamoyl-cyclohexylamino)-5-trifluoromethyl-pyrimidin-2-yl-amino]-*N*-(1-methyl-piperidin-4-yl)-benzamide
(±)-4-{4-[(R*,2S*)-2-(2-fluoro-ethylcarbamoyl)-cyclohexylamino]-5-trifluoromethyl-pyrimidin-2-yl-amino}-*N*-methyl-*N*-(1-methyl-piperidin-4-yl)-benzamide
*N*-methyl-*N*-(1-ethyl-piperidin-4-yl)-4-{4-[(1R,2S)-2-(tetrahydro-furan-3-yl-carbamoyl)-cyclopentylamino]-5-trifluoromethyl-pyrimidin-2-yl-amino}benzamide
(±)-*N*-methyl-*N*-(1-methyl-piperidin-4-yl)-4-{4-[(1R*,2S*)-2-(tetrahydro-pyran-4-yl-carbamoyl)-cyclopentylamino]-5-trifluoromethyl-pyrimidin-2-yl-amino}benzamide
*N*-methyl-*N*-(1-methyl-piperidin-4-yl)-4-{4-[(1R,2S)-2-(2,2,2-trifluoroethylcarbamoyl)-cyclopentylamino]-5-trifluoromethyl-pyrimidin-2-yl-amino}benzamide
(±)-4-{4-[(1R*,2S*)-2-(2-diethylamino-ethylcarbamoyl)-cyclopentylamino]-5-trifluoromethyl-pyrimidin-2-yl-amino}-*N*-methyl-*N*-(1-methyl-piperidin-4-yl)-benzamide
(±)-*N*-methyl-*N*-(1-methyl-piperidin-4-yl)-4-{4-[(1R*,2S*)-2-(2-pyrrolidin-1-yl-ethylcarbamoyl)-cyclopentylamino]-5-trifluoromethyl-pyrimidin-2-yl-amino}benzamide
4-[4-((1R,3S)-3-isopropylcarbamoyl-cyclopentylamino)-5-trifluoromethylpyrimidin-2-yl-amino]-*N*-methyl-*N*-(1-methyl-piperidin-4-yl)-benzamide
4-[4-((1R,3S)-3-carbamoyl-cyclopentylamino)-5-trifluoromethyl-pyrimidin-2-yl-amino]-*N*-methyl-*N*-(1-methyl-piperidin-4-yl)-benzamide
4-[4-((1S,3R)-3-isopropylcarbamoyl-cyclopentylamino)-5-trifluoromethylpyrimidin-2-yl-amino]-*N*-methyl-*N*-(1-methyl-piperidin-4-yl)-benzamide
4-[4-((1S,3R)-3-carbamoyl-cyclopentylamino)-5-trifluoromethyl-pyrimidin-2-yl-amino]-*N*-methyl-*N*-(1-methyl-piperidin-4-yl)-benzamide
(±)-*N*-methyl-*N*-(1-methyl-piperidin-4-yl)-4-{4-[(1R*,2S*)-2-(2-morpholin-4-yl-ethylcarbamoyl)-cyclopentylamino]-5-trifluoromethyl-pyrimidin-2-yl-amino}benzamide
(±)-4-[4-((1R*,2S*)-2-carbamoyl-cyclobutylamino)-5-trifluoromethyl-pyrimidin-2-yl-amino]-*N*-methyl-*N*-(1-methyl-piperidin-4-yl)-benzamide
(±)-4-[4-((1R*,6S*)-6-carbamoyl-cyclohex-3-enyl-amino)-5-trifluoromethylpyrimidin-2-yl-amino]-*N*-methyl-*N*-(1-methyl-piperidin-4-yl)-benzamide
(±)-*N*-methyl-4-[4-((1R*,2S*)-2-methylcarbamoyl-cyclopentylamino)-5-trifluoromethyl-pyrimidin-2-yl-amino]-*N*-(1-methyl-piperidin-4-yl)-benzamide
4-{4-[(1R,3S)-3-(azetidin-1-carbonyl)-cyclopentylamino]-5-trifluoromethylpyrimidin-2-ylamino}-*N*-methyl-*N*-(1-methyl-piperidin-4-yl)-benzamide
*N*-methyl-*N*-(1-methyl-piperidin-4-yl)-4-{4-[(1R,3S)-3-(piperidine-1-carbonyl)-cyclopentylamino]-5-trifluoromethyl-pyrimidin-2-yl-amino}-benzamide
4-{4-[(1R,3S)-3-(2-methoxy-ethylcarbamoyl)-cyclopentylamino]-5-trifluoromethyl-pyrimidin-2-yl-amino}-*N*-methyl-*N*-(1-methyl-piperidin-4-yl)-benzamide
*N*-methyl-*N*-(1-methyl-piperidin-4-yl)-4-{4-[(1R,3S)-3-(pyrrolidine-1-carbonyl)-cyclopentylamino]-5-trifluoromethyl-pyrimidin-2-yl-amino}-benzamide
4-[4-((1R,3S)-3-ethylcarbamoyl-cyclopentylamino)-5-trifluoromethylpyrimidin-2-yl-amino]-*N*-methyl-*N*-(1-methyl-piperidin-4-yl)-benzamide
(±)-(1S*,2R*)-2-(2-{4-[methyl-(1-methyl-piperidin-4-yl)-carbamoyl]-phenylamino}-5-trifluoromethyl-pyrimidin-4-yl-amino)-cyclopentane carboxylic acid
(±)-4-{4-[(1R*,2S*)-2-(cyanomethyl-carbamoyl)-cyclopentylamino]-5-trifluoromethyl-pyrimidin-2-yl-amino}-*N*-methyl-*N*-(1-methyl-piperidin-4-yl)-benzamide
(±)-4-{4-[(1R*,2S*)-2-(2-cyano-ethylcarbamoyl)-cyclopentylamino]-5-trifluoromethyl-pyrimidin-2-yl-amino}-*N*-methyl-*N*-(1-methyl-piperidin-4-yl)-benzamide
(±)-4-{4-[(1R*,2S*)-2-(carbamoylmethyl-carbamoyl)-cyclopentylamino]-5-trifluoromethyl-pyrimidin-2-yl-amino}-*N*-methyl-*N*-(1-methyl-piperidin-4-yl)-benzamide
*N*-methyl-*N*-(1-methyl-piperidin-4-yl)-4-{4-[(R,2S)-2-(3-oxo-piperazine-1-carbonyl)-cyclopentylamino]-5-trifluoromethyl-pyrimidin-2-yl-amino}benzamide
4-(4-{(1R,2S)-2-[(carbamoyl-cyano-methyl)-carbamoyl]-cyclopentylamino}-5-trifluoromethyl-pyrimidin-2-yl-amino)-*N*-methyl-*N*-(1-methyl-piperidin-4-yl)-benzamide
4-{4-[(1R,2S)-2-((S)-1-carbamoyl-3-methyl-butylcarbamoyl)-cyclopentylamino]-5-trifluoromethyl-pyrimidin-2-yl-amino}-*N*-methyl-*N*-(1-methyl-piperidin-4-yl)-benzamide
(±)-4-{4-[(1R*,2S*)-2-(dimethylcarbamoylmethyl-carbamoyl)-cyclopentylamino]-5-trifluoromethyl-pyrimidin-2-yl-amino}-*N*-methyl-*N*-(1-methyl-piperidin-4-yl)-benzamide
(±)-*N*-methyl-4-{4-[(1R*,2S*)-2-(methylcarbamoylmethyl-carbamoyl)-cyclopentylamino]-5-trifluoromethyl-pyrimidin-2-yl-amino}-*N*-(1-methylpiperidin-4-yl)-benzamide
(±)-4-{4-[(1R*,2S*)-2-((R)-1-carbamoyl-3-methyl-butylcarbamoyl)-cyclopentylamino]-5-trifluoromethyl-pyrimidin-2-yl-amino}-*N*-methyl-*N*-(1-methyl-piperidin-4-yl)-benzamide
(±)-4-{4-[(1R*,2S*)-2-(2-carbamoyl-ethylcarbamoyl)-cyclopentylamino]-5-trifluoromethyl-pyrimidin-2-yl-amino}-*N*-methyl-*N*-(1-methyl-piperidin-4-yl)-benzamide
(R)-1-[(1S,2R)-2-(2-{4-[methyl-(1-methyl-piperidin-4-yl)-carbamoyl]-phenylamino}-5-trifluoromethyl-pyrimidin-4-yl-amino)-cyclopentanecarbonyl]-pyrrolidine-2-carboxylic acid amide
(S)-1-[(1S,2R)-2-(2-{4-[methyl-(1-methyl-piperidin-4-yl)-carbamoyl]-phenylamino}-5-trifluoromethyl-pyrimidin-4-yl-amino)-cyclopentanecarbonyl]-pyrrolidine-2-carboxylic acid amide
4-{4-[(1R,2S)-2-((S)-1-carbamoyl-ethylcarbamoyl)-cyclopentylamino]-5-trifluoromethyl-pyrimidin-2-yl-amino}-*N*-methyl-*N*-(1-methyl-piperidin-4-yl)-benzamide
4-{4-[(1R,2S)-2-((R)-1-carbamoyl-ethylcarbamoyl)-cyclopentylamino]-5-trifluoromethyl-pyrimidin-2-yl-amino}-*N*-methyl-*N*-(1-methyl-piperidin-4-yl)-benzamide
(±)-4-{4-[(1R*,2S*)-2-(2-amino-2-methyl-propylcarbamoyl)-cyclopentylamino]-5-trifluoromethyl-pyrimidin-2-ylamino}-*N*-methyl-*N*-(1-methyl-piperidin-4-yl)-benzamide
(±)-*N*-methyl-*N*-(1-methyl-piperidin-4-yl)-4-{4-[(1R*,2S*)-2-(thiazol-2-ylcarbamoyl)-cyclopentylamino]-5-trifluoromethyl-pyrimidin-2-yl-amino}benzamide
(±)-4-[4-((1R*,2S*)-2-amino-cyclohexylamino)-5-trifluoromethyl-pyrimidin-2-yl-amino]-*N*-methyl-*N*-(1-methyl-piperidin-4-yl)-benzamide
(±)-4-[4-((1R*,2S*)-2-acetylamino-cyclohexylamino)-5-trifluoromethylpyrimidin-2-yl-amino]-*N*-methyl-*N*-(1-methyl-piperidin-4-yl)-benzamide
(±)-4-[4-((1R*,2S*)-2-methanesulfonylamino-cyclohexylamino)-5-trifluoromethyl-pyrimidin-2-yl-amino]-*N*-methyl-*N*-(1-methyl-piperidin-4-yl)-benzamide
(±)-4-[4-((1R*,2S*)-2-isobutyrylamino-cyclohexylamino)-5-trifluoromethylpyrimidin-2-yl-amino]-*N*-methyl-*N*-(1-methyl-piperidin-4-yl)-benzamide
(±)-4-{4-[(1R*,2S*)-2-(cyclopropanecarbonyl-amino)-cyclohexylamino]-5-trifluoromethyl-pyrimidin-2-yl-amino}-*N*-methyl-*N*-(1-methyl-piperidin-4-yl)-benzamide
(±)-*N*-methyl-*N*-(1-methyl-piperidin-4-yl)-4-[4-((1R*,2S*)-2-propionylaminocyclohexylamino)-5-trifluoromethyl-pyrimidin-2-yl-amino]-benzamide
(±)-4-[4-((1R*,2S*)-2-ethanesulfonylamino-cyclohexylamino)-5-trifluoromethyl-pyrimidin-2-yl-amino]-*N*-methyl-*N*-(1-methyl-piperidin-4-yl)-benzamide
(±)-4-{4-[(1R*,2S*)-2-(3,3-dimethyl-ureido)-cyclohexylamino]-5-trifluoromethyl-pyrimidin-2-yl-amino}-*N*-methyl-*N*-(1-methyl-piperidin-4-yl)-benzamide
(±)-(1R*,28*,3R*,4S*)-3-(2-{4-[methyl-(1-methyl-piperidin-4-yl)-carbamoyl]-phenylamino}-5-trifluoromethyl-pyrimidin-4-yl-amino)-bicyclo[2.2.1]hept-5-ene-2-carboxylic acid
4-[4-(adamantan-2-ylamino)-5-trifluoromethyl-pyrimidin-2-yl-amino]-*N*-methyl-*N*-(1-methyl-piperidin-4-yl)-benzamide
(±)-(1R*,2R*,3S*,4S*)-3-(2-{4-[methyl-(1-methyl-piperidin-4-yl)-carbamoyl]-phenylamino}-5-trifluoromethyl-pyrimidin-4-yl-amino)-bicyclo[2.2.1]heptane-2-carboxylic acid cyclopropylamide
(±)-4-[4-((1R*,2S*)-2-isobutyrylamino-cyclopentylamino)-5-trifluoromethylpyrimidin-2-yl-amino]-*N*-methyl-*N*-(1-methyl-piperidin-4-yl)-benzamide
(±)-*N*-methyl-4-{4-[(1R*,2S*)-2-(3-methyl-butyrylamino)-cyclopentylamino]-5-trifluoromethyl-pyrimidin-2-yl-amino}-*N*-(1-methyl-piperidin-4-yl)-benzamide
(±)-*N*-methyl-*N*-(1-methyl-piperidin-4-yl)-4-[4-((1R*,2S*)-2-propionylaminocyclopentylamino)-5-trifluoromethyl-pyrimidin-2-yl-amino]-benzamide
(±)-4-{4-[(1R*,2S*)-2-(2-methoxy-acetylamino)-cyclopentylamino]-5-trifluoromethyl-pyrimidin-2-yl-amino}-*N*-methyl-*N*-(1-methyl-piperidin-4-yl)-benzamide
(±)-4-[4-((1R*,2S*)-2-amino-cyclopentylamino)-5-trifluoromethyl-pyrimidin-2-yl-amino]-*N*-methyl-*N*-(1-methyl-piperidin-4-yl)-benzamide
*N*-methyl-*N*-(1-methyl-piperidin-4-yl)-4-(4-{(1R,2S)-2-[2-(1-methyl-pyrrolidine-2-yl)-ethylcarbamoyl]-cyclopentylamino}-5-trifluoromethyl-pyrimidin-2-yl-amino)-benzamide
4-{4-[(1R,2S)-2-(2,2-difluoro-ethylcarbamoyl)-cyclopentylamino]-5-trifluoromethyl-pyrimidin-2-yl-amino}-*N*-methyl-*N*-(1-methyl-piperidin-4-yl)-benzamide
4-{4-[(1R,2S)-2-(2-fluoro-ethylcarbamoyl)-cyclopentylamino]-5-trifluoromethyl-pyrimidin-2-yl-amino}-*N*-methyl-*N*-(1-methyl-piperidin-4-yl)-benzamide
2-methoxy-*N*-methyl-4-[4-((1R,2S)-2-methylcarbamoyl-cyclopentylamino)-5-trifluoromethyl-pyrimidin-2-yl-amino]-*N*-(1-methyl-piperidin-4-yl)-benzamide
4-[4-((1R,2S)-2-isopropylcarbamoyl-cyclopentylamino)-5-trifluoromethylpyrimidin-2-yl-amino]-2-methoxy-*N*-methyl-*N*-(1-methyl-piperidin-4-yl)-benzamide
4-[4-((1R,2S)-2-cyclopropylcarbamoyl-cyclopentylamino)-5-trifluoromethylpyrimidin-2-yl-amino]-2-methoxy-*N*-methyl-*N*-(1-methyl-piperidin-4-yl)-benzamide
4-[4-((1R,2S)-2-isopropylcarbamoyl-cyclopentylamino)-5-trifluoromethylpyrimidin-2-yl-amino]-*N*-methyl-*N*-(1-methyl-piperidin-4-yl)-2-propoxybenzamide
2-ethoxy-4-[4-((1R,2S)-2-isopropylcarbamoyl-cyclopentylamino)-5-trifluoromethyl-pyrimidin-2-yl-amino]-*N*-methyl-*N*-(1-methyl-piperidin-4-yl)-benzamide
2-isopropoxy-4-[4-((1R,2S)-2-isopropylcarbamoyl-cyclopentylamino)-5-trifluoromethyl-pyrimidin-2-yl-amino]-*N*-methyl-*N*-(1-methyl-piperidin-4-yl)-benzamide
4-[4-((1R,2S)-2-isopropylcarbamoyl-cyclopentylamino)-5-trifluoromethylpyrimidin-2-yl-amino]-*N*-methyl-*N*-(1-methyl-piperidin-4-yl)-2-prop-2-ynyl-oxybenzamide
(±)-4-[4-((1R*,2S*)-2-isopropylcarbamoyl-cyclobutylamino)-5-trifluoromethylpyrimidin-2-yl-amino]-*N*-methyl-*N*-(1-methyl-piperidin-4-yl)-benzamide
(±)-4-{5-bromo-4-[(1R*,2S*)-2-(1-methyl-cyclopropylcarbamoyl)-cyclopentylamino]-pyrimidin-2-yl-amino}-*N*-methyl-*N*-(1-methyl-piperidin-4-yl)-benzamide
(±)-4-[5-bromo-4-((1R*,2S*)-2-isopropylcarbamoyl-cyclopentylamino)-pyrimidin-2-yl-amino]-*N*-methyl-*N*-(1-methyl-piperidin-4-yl)-benzamide
(±)-4-[5-bromo-4-[(1R*,2S*)-2-cyclopropylcarbamoyl)-cyclopentylamino)-pyrimidin-2-yl-amino]-*N*-methyl-*N*-(1-methyl-piperidin-4-yl)-benzamide
(±)-4-{5-bromo-4-[(1R*,2S*)-2-(2-cyano-ethylcarbamoyl)-cyclopentylamino]-pyrimidin-2-yl-amino}-*N*-methyl-*N*-(1-methyl-piperidin-4-yl)-benzamide
(±)-4-[5-bromo-4-((1R*,2S*)-2-cyclopentylcarbamoyl-cyclopentylamino)-pyrimidin-2-yl-amino]-*N*-methyl-*N*-(1-methyl-piperidin-4-yl)-benzamide
4-{5-bromo-4-[(1R,2S)-2-(pyrrolidine-1-carbonyl)-cyclopentylamino]-pyrimidin-2-yl-amino}-*N*-methyl-*N*-(1-methyl-piperidin-4-yl)-benzamide
4-{4-[(1R,2S)-2-(azetidine-1-carbonyl)-cyclopentylamino]-5-bromo-pyrimidin-2-yl-amino}-*N*-methyl-*N*-(1-methyl-piperidin-4-yl)-benzamide
(±)-4-[5-bromo-4-((1R*,2S*)-2-cyclobutylcarbamoyl-cyclopentylamino)-pyrimidin-2-yl-amino]-*N*-methyl-*N*-(1-methyl-piperidin-4-yl)-benzamide
4-[4-((1R,2S)-2-isopropylcarbamoyl-cyclopentylamino)-5-methyl-pyrimidin-2-yl-amino]-*N*-methyl-*N*-(1-methyl-piperidin-4-yl)-benzamide
4-[5-bromo-4-((1R,2S)-2-isopropylcarbamoyl-cyclopentylamino)-pyrimidin-2-yl-amino]-*N*-methyl-*N*-(1-methyl-piperidin-4-yl)-benzamide
4-[5-chloro-4-((1 R,2S)-2-isopropylcarbamoyl-cyclopentylamino)-pyrimidin-2-yl-amino]-*N*-methyl-*N*-(1-methyl-piperidin-4-yl)-benzamide.

2. Compounds, or the pharmaceutically effective salts thereof, according to claim 1, for use as medicaments.

3. Compounds, or the pharmaceutically effective salts thereof, according to claim 1, for preparing a medicament having an antiproliferative activity.

4. Pharmaceutical preparations containing as active substance one or more compounds of general formula (1) according to claim 1, or the pharmaceutically effective salts thereof, optionally in combination with conventional excipients and/or carriers.

5. Use of compounds of general formula (1) according to claim 1 for preparing a medicament for the treatment and/or prevention of cancer, infections, inflammations and autoimmune diseases.

6. Pharmaceutical preparation comprising a compound of general formula (1) according to claim 1 and at least one other cytostatic or cytotoxic active substance different from formula (1), optionally in the form of the tautomers, the racemates, the enantiomers, the diastereomers and the mixtures thereof, and optionally the pharmaceutically effective salts thereof.

## Revendications

1. Composés de formule générale (1) 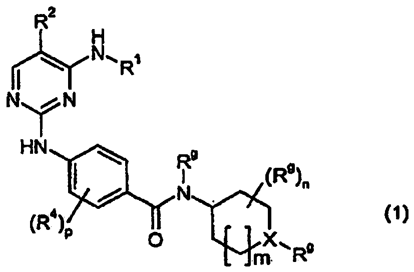 choisis dans le groupe consistant en
4-[4-((1R,2S)-2-isopropylcarbamoyl-cyclopentylamino)-5-trifluorométhylpyrimidin-2-ylamino]-*N*-méthyl-*N*-(1,2,2,6,6-pentaméthyl-pipéridin-4-yl)-benzamide ;
(±)-4-[4-((1R*,2S*)-2-isopropylcarbamoyl-cyclopentylamino)-5-triftuorométhylpyrimidin-2-ylamino]-*N*-méthyl-*N*-(1-méthyl-pipéridin-4-yl)-benzamide;
4-[4-((1R,2S)-2-isopropylcarbamoyl-cyclopentylamino)-5-trifluorométhylpyrimidin-2-ylamino]-*N*-méthyl-*N*-(1-méthyl-pipéridin-4-yl)-benzamide;
4-[4-((1S,2R)-2-isopropylcarbamoyl-cyclopentylamino)-5-trifluorométhylpyrimidin-2-ylamino]-*N*-méthyl-*N*-(1-méthyl-pipéridin-4-yl)-benzamide;
*N*-(1-éthyl-pipéridin-4-yl)-4-[4-((1R,2S)-2-isopropylcarbamoyl-cyclopentylamino)-5-trifluorométhyl-pyrimidin-2-ylamino]-*N*-méthyl-benzamide;
*N*-isopropyl-4-[4-((1R,2S)-2-isopropylcarbamoyl-cyclopentylamino)-5-trifluorométhyl-pyrimidin-2-ylamino]-*N*-(1-méthyl-pipéridin-4-yl)-benzamide;
(±)-*N*-cyclohexyl-4-[4-((1R*,2S*)-2-isopropylcarbamoyl-cyclopentylamino)-5-trifluorométhyl-pyrimidin-2-ylamino]-*N*-méthyl-benzamide;
4-[4-((1R,2S)-2-isopropylcarbamoyl-cyclopentylamino)-5-trifluorométhylpyrimidin-2-ylamino]-*N*-méthyl-*N*-(1-méthyl-pyrrolidin-3-yl)-benzamide;
4-[4-((1R,2S)-2-éthylcarbamoyl-cyclopentylamino)-5-trifluorométhyl-pyrimidin-2-ylamino]-*N*-méthyl-*N*-(1-méthyl-pipéridin-4-yl)-benzamide;
4-[4-((1R,2S)-2-cyclopropylcarbamoyl-cyclopentylamino)-5-trifluorométhylpyrimidin-2-ylamino]-*N*-(1-méthyl-pipéridin-4-yl)-benzamide;
4-{4-[(1R*,2S*)-2-((R)-2-hydroxy-1-méthyl-éthylcarbamoyl)-cyclopentylamino]-5-trifluorométhyl-pyrimidin-2-ylamino}-*N*-méthyl-*N*-(1-méthyl-pipéridin-4-yl)-benzamide ;
(±)-4-[4-((1R*,2S*)-2-isobutylcarbamoyl-cyclopentylamino)-5-trifluorométhylpyrimidin-2-ylamino]-*N*-méthyl-*N*-(1-méthyl-pipéridin-4-yl)-benzamide;
(±)-4-{4-[(1R*,2S*)-2-(2-éthyl-butylcarbamoyl)-cyclopentylamino]-5-trifluorométhyl-pyrimidin-2-ylamino}-*N*-méthyl-*N*-(1-méthyl-pipéridin-4-yl)-benzamide;
(±)-4-{4-[(1R*,2S*)-2-(cyclohexylméthyl-carbamoyl)-cyclopentylamino]-5-trifluorométhyl-pyrimidin-2-ylamino}-*N*-méthyl-*N*-(1-méthyl-pipéridin-4-yl)-benzamide ;
4-{4-[(1R,2S)-2-((S)-2-hydroxy-1-méthyl-éthylcarbamoyl)-cyclopentylamino]-5-trifluorométhyl-pyrimidin-2-ylamino}-*N*-méthyl-*N*-(1-méthyl-pipéridin-4-yl)-benzamide ;
(±)-*N*-méthyl-*N*-(1-méthyl-pipéàdin-4-yl)-4-{5-trifluorométhyl-4-[(1R*,2S*)-2-(3,3,3-trifluoro-propylcarbamoyl)-cyclopentylamino]-pyrimidin-2-ylamino}benzamide ;
(±)-4-[4-((1R*,2S*)-2-cyclopropylcarbamoyl-cyclopentylamino)-5-trifluorométhyl-pyrimidin-2-ylamino]-*N*-méthyl-*N*-(1-méthyl-pipéridin-4-yl)-benzamide;
4-[4-((1R,2S)-2-cyclopropylcarbamoyl-cyclopentylamino)-5-trifluorométhylpyrimidin-2-ylamino]-*N*-méthyl-*N*-(1-méthyl-pipéridin-4-yl)-benzamide;
isopropylamide d'acide (±)-(2S*,3R*)-3-(2-{4-[méthyl-(1-méthyl-pipéridin-4-yl)-carbamoyl]-phénylamino}-5-trifluorométhyl-pyrimidin-4-ylamino)-bicyclo-[2.2.2]octane-2-carboxylique ;
cyclopentylamide d'acide (±)-(2S*,3R*)-3-(2-{4-[méthyl-(1-méthyl-pipéridin-4-yl)-carbamoyl]-phénylamino}-5-triftuorométhyl-pyrimidin-4-ylamino)-bicyclo-[2.2.2]octane-2-carboxylique ;
4-[4-(3-isopropylcarbamoyl-cyclobutylamino)-5-trifluorométhyl-pyrimidin-2-yl-amino]-*N*-méthyl-*N*-(1-méthyl-pipéridin-4-yl)-benzamide;
4-[4-(3-éthylcarbamoyl-cyclobutylamino)-5-trifluorométhyl-pyrimidin-2-yl-amino]-*N*-méthyl-*N*-(1-méthyl-pipéridin-4-yl)-benzamide;
cyclopropylamide d'acide (±)-(2S*,3R*)-3-(2-{4-[méthyl-(1-méthyl-pipéridin-4-yl)-carbamoyl]-phénylamino}-5-triftuorométhyl-pynmidin-4-ylamino)-bicyclo-[2.2.2]octane-2-carboxylique ;
éthylamide d'acide (±)-(2S*,3R*)-3-(2-{4-[méthyl-(1-méthyl-pipéridin-4-yl)-carbamoyl]-phénylamino}-5-triftuorométhyl-pyrimidin-4-ylamino)-bicyclo-[2.2.2]octane-2-carboxylique ;
*N*-méthyl-4-{4-[3-(1-méthyl-cyclopropylcarbamoyl)-cyclobutylamino]-5-trifluorométhyl-pyrimidin-2-ylamino}-*N*-(1-méthyl-pipéridin-4-yl)-benzamide;
4-[4-(3-cyclopropylcarbamoyl-cyclobutylamino)-5-trifluorométhyl-pyrimidin-2-ylamino]-*N*-méthyl-*N*-(1-méthyl-pipéridin-4-yl)-benzamide;
*N*-méthyl-4-[4-(3-méthylcarbamoyl-cyclobutylamino)-5-trifluorométhyl-pyrimidin-2-ylamino]-*N*-(1-méthyl-pipéridin-4-yl)-benzamide;
*N*-méthyl-4-[4-((1R,2S)-2-méthylcarbamoyl-cyclopentylamino)-5-trifluorométhylpyrimidin-2-ylamino]-*N*-(1-méthyl-pipéridin-4-yl)-benzamide;
(±)-4-[4-((1R*,2S*)-2-*tert*-butylcarbamoyl-cyclopentylamino)-5-triftuorométhylpyrimidin-2-ylamino]-*N*-méthyl-*N*-(1-méthyl-pipéridin-4-yl)-benzamide;
(+)-*N*-méthyl-4-{4-[(1R*,2S*)-2-(1-méthyl-cyclopropylcarbamoyl)-cyclopentylamino]-5-trifluorométhyl-pyrimidin-2-ylamino}-*N*-(1-méthyl-pipéridin-4-yl)-benzamide ;
*N*-méthyl-4-{4-[(1R,2S)-2-(1-méthyl-cyclopropylcarbamoyl)-cyclopentylamino]-5-trifluorométhyl-pyrimidin-2-ylamino}-*N*-(1-méthyl-pipéridin-4-yl)-benzamide;
(±)-4-{4-[(1R*,25*)-2-(2-hydroxy-1,1-diméthyl-éthylcarbamoyl)-cyclopentylamino]-5-trifluorométhyl-pyrimidin-2-ylamino}-*N*-méthyl-*N*-(1-méthyl-pipéridin-4-yl)-benzamide ;
(±)-4-{4-[(1R*,2S*)-2-(azétidine-1-carbonyl)-cyclopentylamino]-5-trifluorométhyl-pyrimidin-2-ylamino}-*N*-méthyl-*N*-(1-méthyl-pipéridin-4-yl)-benzamide;
(±)-*N*-méthyl-*N*-(1-méthyl-pipéridin-4-yl)-4-{4-[(1R*,2S*)-2-(pipéridine-1-carbonyl)-cyclopentylamino]-5-trifluorométhyl-pyrimidin-2-ylamino}-benzamide;
(±)-*N*-méthyl-*N*-(1-méthyl-pipéridin-4-yl)-4-{4-[(1R*,2S*)-2-(morpholine-4-carbonyl)-cyclopentylamino]-5-trifluorométhyl-pyrimidin-2-ylamino}-benzamide ;
(±)-*N*-méthyl-4-{4-[(1R*,2S*)-2-(4-méthyl-pipérazine-1-carbonyl)-cyclopentylamino]-5-trifluorométhyl-pyrimidin-2-ylamino}*-N*-(1-méthyl-pipéridin-4-yl)-benzamide ;
(±)-*N*-méthyl-*N*-(1-méthyl-pipéridin-4-yl)-4-{4-[(1R*,2S*)-2-(thiomorpholine-4-carbonyl)-cyclopentylamino]-5-trifluorométhyl-pyrimidin-2-ylamino}- benzamide ;
méthylester d'acide (±)-(1S*,2R*)-2-(2-{4-[méthyl-(1-méthyl-pipéridin-4-yl)-carbamoyl]-phénylamino}-5-trifluorométhyl-pyrimidin-4-ylamino)-cyclopentanecarboxylique ;
(±)-4-{4-[(1R*,2S*)-2-((2R,6S)-2,6-diméthyl-morpholine-4-carbonyl)-cyclopentylamino]-5-trifluorométhyl-pyrimidin-2-ylamino}-*N*-méthyl-*N*-(1-méthylpipéridin-4-yl)-benzamide ;
4-{4-[(1R,3S)-3-(cyclopropylméthyl-carbamoyl)-cyclopentylamino]-5-trifluorométhyl-pyrimidin-2-ylamino}-*N*-méthyl-*N*-(1-méthyl-pipéridin-4-yl)-benzamide ;
4-[4-((1R,3S)-3-benzylcarbamoyl-cyclopentylamino)-5-trifluorométhyl-pyrimidin-2-ylamino]-*N*-méthyl-*N*-(1-méthyl-pipéridin-4-yl)-benzamide ;
4-[4-((1R,3S)-3-cyclopentylcarbamoyl-cyclopentylamino)-5-trifluorométhylpyrimidin-2-ylamino]-*N*-méthyl-*N*-(1-méthyl-pipéridin-4-yl)-benzamide ;
4-{4-[(1R,3S)-3-(cyanométhyl-carbamoyl)-cyclopentylamino]-5-trifluorométhylpyrimidin-2-ylamino}-*N*-méthyl-*N*-(1-méthyl-pipéridin-4-yl)-benzamide ;
4-{4-[(1R,3S)-3-(3-diméthylamino-propylcarbamoyl)-cyclopentylamino]-5-trifluorométhyl-pyrimidin-2-ylamino1-*N*-méthyl-*N*-(1-méthyl-pipéridin-4-yl)-benzamide ;
4-{4-[(1R,3S)-3-(2-diméthylamino-éthylcarbamoyl)-cyclopentylamino]-5-trifluorométhyl-pyrimidin-2-ylamino}-*N*-méthyl-*N*-(1-méthyl-pipéridin-4-yl)-benzamide ;
4-{4-[(1R,2S)-3-(4-diméthylamino-butylcarbamoyl)-cyclopentylamino]-5-trifluorométhyl-pyrimidin-2-ylamino}-*N*-méthyl-*N*-(1-méthyl-pipéridin-4-yl)-benzamide ;
*N*-méthyl-*N*-(1-méthyl-pipéridin-4-yl)-4-{4-[(1R,2S)-2-(3-pyrrolidin-1-yl-propylcarbamoyl)-cyclopentylamino]-5-trifluorométhyl-pyrimidin-2-ylamino benzamide ;
*N*-méthyl-*N*-(1-méthyl-pipéridin-4-yl)-4-(4-{(1R,25)-2-[(1-méthyl-pipéridin-4-ylméthyl)-carbamoyl]-cyclopentylamino}-5-trifluorométhyl-pyrimidin-2-ylamino)-benzamide ;
*N*-méthyl-*N*-(1-méthyl-pipéridin-4-yl)-4-{4-[(1R,2S)-2-(1-méthyl-pipéridin-4-ylcarbamoyl)-cyclopentylamino]-5-trifluorométhyl-pyrimidin-2-ylamino}benzamide ;
4-{4-[(1R,2S)-2-(3-diméthylamino-2,2-diméthyl-propylcarbamoyl)-cyclopentylamino]-5-trifluorométhyl-pyrimidin-2-ylamino}-*N*-méthyl-*N*-(1-méthylpipéridin-4-yl)-benzamide ;
*N*-méthyl-*N*-(1-méthyl-pipéridin-4-yl)-4-{4-[(1R,2S)-2-(3-pyrrolidin-1-yl-cyclobutylcarbamoyl)-cyclopentylamino]-5-triftuorométhyl-pyrimidin-2-ylamino}benzamide ;
*N*-méthyl-*N*-(1-méthyl-pipéridin-4-yl)-4-{4-[(1R,2S)-2-(4-morpholin-4-yl-cyclohexylcarbamoyl)-cyclopentylamino]-5-triftuorométhyl-pyrimidin-2-yl-amino}benzamide ;
*N*-méthyl-4-(4-{(1R,2S)-2-[3-(4-méthyl-pipérazin-1-yl)-propylcarbamoyl]-cyclopentylamino}-5-trifluorométhyl-pyrimidin-2-ylamino)-*N*-(1-méthyl-pipéridin-4-yl)-benzamide ;
(±)-4-[4-((1R*,2S*)-2-diméthylcarbamoyl-cyclopentylamino)-5-triftuorométhylpyrimidin-2-ylamino]-*N*-méthyl-*N*-(1-méthyl-pipéridin-4-yl)-benzamide ;
(±)-4-[4-((1R*,2S*)-2-cyclobutylcarbamoyl-cyclopentylamino)-5-triftuorométhylpyrimidin-2-ylamino]-*N*-méthyl-*N*-(1-méthyl-pipéridin-4-yl)-benzamide ;
(±)-4-[4-((1R*,2S*)-2-cyclopentylcarbamoyl-cyclopentylamino)-5-trifluorométhyl-pyrimidin-2-ylamino]-*N*-méthyl-*N*-(1-méthyl-pipéridin-4-yl)-benzamide;
(±)-*N*-méthyl-4-{4-[(1R*,25*)-2-(1-méthyl-cyclopropylcarbamoyl)-cyclohexylamino]-5-trifluorométhyl-pyrimidin-2-ylamino}-*N*-(1-méthyl-pipéridin-4-yl)-benzamide ;
(±)-4-[4-((1R*,2S*)-2-cyclopropylcarbamoyl-cyclohexylamino)-5-trifluorométhyl-pyrimidin-2-ylamino]-*N*-méthyl-*N*-(1-méthyl-pipéridin-4-yl)-benzamide;
(±)-4-{4-[(1R*,2S*)-2-(2,2-difluoro-éthylcarbamoyl)-cyclohexylamino]-5-trifluorométhyl-pyrimidin-2-ylamino}-*N*-méthyl-*N*-(1-méthyl-pipéridin-4-yl)-benzamide ;
(±)-4-[4-((1R*,2S*)-2-diméthylcarbamoyl-cyclohexylamino)-5-triftuorométhylpyrimidin-2-ylamino]-*N*-méthyl-*N*-(1-méthyl-pipéridin-4-yl)-benzamide;
(±)-4-[4-((1R*,2S*)-2-éthylcarbamoyl-cyclohexylamino)-5-trifluorométhylpyrimidin-2-ylamino]-*N*-méthyl-*N*-(1-méthyl-pipéridin-4-yl)-benzamide;
(±)-*N*-méthyl-*N*-(1-méthyl-pipéridin-4-yl)-4-{4-[(1R*,2S*)-2-(pipéridine-1-carbonyl)-cyclohexylamino]-5-trifluorométhyl-pyrimidin-2-ylamino}- benzamide ;
(±)-*N*-méthyl-*N*-(1-méthyl-pipéridin-4-yl)-4-{4-[(1R*,2S*)-2-(pyrrolidine-1-carbonyl)-cyclohexylamino]-5-trifluorométhyl-pyrimidin-2-ylamino}- benzamide ;
(±)-4-[4-((1R*,2S*)-2-isopropylcarbamoyl-cyclohexylamino)-5-trifluorométhylpyrimidin-2-ylamino]-*N*-méthyl-*N*-(1-méthyl-pipéridin-4-yl)-benzamide;
(±)-*N*-méthyl-4-[4-((1R*,2S*)-2-méthylcarbamoyl-cyclohexylamino)-5-trifluorométhyl-pyrimidin-2-ylamino]-*N*-(1-méthyl-pipéridin-4-yl)-benzamide;
(±)-4-{4-[(1R*,2S*)-2-(2-fluoro-éthylcarbamoyl)-cyclohexylamino]-5-trifluorométhyl-pyrimidin-2-ylamino}*-N*-méthyl-N-(1-méthyl-pipéridin-4-yl)-benzamide ;
*N*-méthyl-*N*-(1-méthyl-pipéridin-4-yl)-4-{4-[(1R,2S)-2-(tétrahydro-furan-3-yl-carbamoyl)-cyclopentylamino]-5-trifluorométhyl-pyrimidin-2-ylamino}benzamide ;
(±)-*N*-méthyl-*N*-(1-méthyl-pipéridin-4-yl)-4-{4-[(1R*,2S*)-2-(tétrahydro-pyran-4-ylcarbamoyl)-cyclopentylamino]-5-trifluorométhyl-pyrimidin-2-ylamino}benzamide ;
*N*-méthyl-*N*-(1-méthyl-pipéridin-4-yl)-4-{4-[(1R,2S)-2-(2,2,2-trifluoroéthylcarbamoyl)-cyclopentylamino]-5-trifluorométhyl-pyrimidin-2-ylamino}benzamide ;
(±)-4-{4-[(1R*,2S*)-2-(2-diméthylamino-éthylcarbamoyl)-cyclopentylamino]-5-trifluorométhyl-pyrimidin-2-ylamino}1-*N*-méthyl-*N*-(1-méthyl-pipéridin-4-yl)-benzamide ;
(±)-*N*-méthyl-*N*-(1-méthyl-pipéridin-4-yl)-4-{4-[(1R*,2S*)-2-(2-pyrrolidin-1-yl-éthylcarbamoyl)-cyclopentylamino]-5-trifluorométhyl-pyrimidin-2-ylamino}benzamide ;
4-[4-((1R,3S)-3-isopropylcarbamoyl-cyclopentylamino)-5-trifluorométhylpyrimidin-2-ylamino]-*N*-méthyl-*N*-(1-méthyl-pipéridin-4-yl)-benzamide ;
4-[4-((1R,3S)-3-carbamoyl-cyclopentylamino)-5-trifluorométhyl-pyrimidin-2-yl-amino]-*N*-méthyl-*N*-(1-méthyl-pipéridin-4-yl)-benzamide ;
4-[4-((1S,3R)-3-isopropylcarbamoyl-cyclopentylamino)-5-trifluorométhylpyrimidin-2-ylamino]-*N*-méthyl-*N*-(1-méthyl-pipéridin-4-yl)-benzamide ;
4-[4-((1S,3R)-3-carbamoyl-cyclopentylamino)-5-trifluorométhyl-pyrimidin-2-yl-amino]-*N*-méthyl-*N*-(1-méthyl-pipéridin-4-yl)-benzamide ;
(±)-*N*-méthyl-*N*-(1-méthyl-pipéridin-4-yl)-4-{4-[(1R*,2S*)-2-(2-morpholin-4-yl-éthylcarbamoyl)-cyclopentylamino]-5-trifluorométhyl-pyrimidin-2-ylamino}benzamide ;
(±)-4-[4-((1R*,2S*)-2-carbamoyl-cyclobutylamino)-5-triftuorométhyl-pyrimidin-2-ylamino]-*N*-méthyl-*N*-(1-méthyl-pipéridin-4-yl)-benzamide ;
(±)-4-[4-((1R*,6S*)-6-carbamoyl-cyclohex-3-énylamino)-5-triftuorométhylpyrimidin-2-ylamino]-*N*-méthyl-*N*-(1-méthyl-pipéridin-4-yl)-benzamide ;
(±)-*N*-méthyl-4-[4-((1R*,2S*)-2-méthylcarbamoyl-cyclopentylamino)-5-trifluorométhyl-pyrimidin-2-ylamino]-*N*-(1-méthyl-pipéridin-4-yl)-benzamide ;
4-{4-[(1R,3S)-3-(azétidine-1-carbonyl)-cyclopentylamino]-5-trifluorométhylpyrimidin-2-ylamino}-*N*-méthyl-*N*-(1-méthyl-pipéridin-4-yl)-benzamide ;
*N*-méthyl-*N*-(1-méthyl-pipéridin-4-yl)-4-{4-[(1R,3S)-3-(pipéridine-1-carbonyl)-cyclopentylamino]-5-trifluorométhyl-pyrimidin-2-ylamino}-benzamide ;
4-{4-[(1R,3S)-3-(2-méthoxy-éthylcarbamoyl)-cyclopentylamino]-5-trifluorométhyl-pyrimidin-2-ylamino}-*N*-méthyl-*N*-(1-méthyl-pipéridin-4-yl)-benzamide ;
*N*-méthyl-*N*-(1-méthyl-pipéridin-4-yl)-4-{4-[(1R,3S)-3-(pyrrolidine-1-carbonyl)-cyclopentylamino]-5-trifluorométhyl-pyrimidin-2-ylamino}-benzamide;
4-[4-((1R,3S)-3-éthylcarbamoyl-cyclopentylamino)-5-trifluorométhyl-pyrimidin-2-ylamino]-*N*-méthyl-*N*-(1-méthyl-pipéridin-4-yl)-benzamide;
acide (±)-(1S*,2R*)-2-(2-{4-[méthyl-(1-méthyl-pipéridin-4-yl)carbamoyl]-phénylamino}-5-trifluorométhyl-pyrimidin-4-ylamino)-cyclopentanecarboxylique ;
(±)-4-{4-[(1R*,2S*)-2-(cyanométhyl-carbamoyl)-cyclopentylamino]-5-trifluorométhyl-pyrimidin-2-ylamino}-*N*-méthyl-*N*-(1-méthyl-pipéridin-4-yl)-benzamide ;
(±)-4-{4-[(1R*,2S*)-2-(2-cyano-éthylcarbamoyl)-cyclopentylamino]-5-trifluorométhyl-pyrimidin-2-ylamino}-*N*-méthyl-*N*-(1-méthyl-pipéridin-4-yl)-benzamide ;
(±)-4-{4-[(1R*,2S*)-2-(carbamoylméthyl-carbamoyl)-cyclopentylamino]-5-trifluorométhyl-pyrimidin-2-ylamino}-*N*-méthyl-*N*-(1-méthyl-pipéridin-4-yl)-benzamide;
*N*-méthyl-*N*-(1-méthyl-pipéridin-4-yl)-4-{4-[(1R,2S)-2-(3-oxo-pipérazine-1-carbonyl)-cyclopentylamino]-5-trifluorométhyl-pyrimidin-2-ylamino}-benzamide ;
4-(4-{(1R,2S)-2-[(carbamoyl-cyano-méthyl)-carbamoyl]-cyclopentylamino}-5-trifluorométhyl-pyrimidin-2-ylamino)-*N*-méthyl-*N*-(1-méthyl-pipéridin-4-yl)-benzamide ;
4-{4-[(1R,2S)-2-((S)-1-carbamoyl-3-méthyl-butylcarbamoyl)-cyclopentylamino]-5-trifluorométhyl-pyrimidin-2-ylamino}-*N*-méthyl-*N*-(1-méthyl-pipéridin-4-yl)-benzamide ;
(±)-4-{4-[(1R*,2S*)-2-(diméthylcarbamoylméthyl-carbamoyl)-cyclopentylamino]-5-trifluorométhyl-pyrimidin-2-ylamino}-*N*-méthyl-*N*-(1-méthyl-pipéridin-4-yl)-benzamide ;
(±)-*N*-méthyl-4-{4-[(1R*,2S*)-2-(méthylcarbamoylméthyl-carbamoyl)-cyclopentylamino]-5-trifluorométhyl-pyrimidin-2-ylamino}-*N*-(1-méthyl-pipéridin-4-yl)-benzamide ;
(±)-4-{4-[(1R*,2S*)-2-((R)-1-carbamoyl-3-méthyl-butylcarbamoyl)-cyclopentylamino]-5-trifluorométhyl-pyrimidin-2-ylamino}-*N*-méthyl-*N*-(1-méthyl-pipéridin-4-yl)-benzamide ;
(±)-4-{4-[(1R*,2S*)-2-(2-carbamoyl-éthylcarbamoyl)-cyclopentylamino]-5-trifluorométhyl-pyrimidin-2-ylamino}-*N*-méthyl-*N*-(1-méthyl-pipéridin-4-yl)-benzamide ;
(R)-1-[(1S,2R)-2-(2-{4-[méthyl-(1-méthyl-pipéridin-4-yl)-carbamoyl]-phénylamino}-5-triftuorométhyl-pyrimidin-4-ylamino)-cyclopentanecarbonyl]-pyrrolidine-2-carboxamide ;
(S)-1-[(1S,2R)-2-(2-{4-[méthyl-(1-méthyl-pipéridin-4-yl)-carbamoyl]-phénylamino}-5-trifluorométhyl-pyrimidin-4-ylamino)-cyclopentanecarbonyl]-pyrrolidine-2-carboxamide;
4-{4-[(1R,2S)-2-((S)-1-carbamoyl-éthylcarbamoyl)-cyclopentylamino]-5-trifluorométhyl-pyrimidin-2-ylamino-*N*-méthyl-*N*-(1-méthyl-pipéridin-4-yl)-benzamide ;
4-{4-[(1R,2S)-2-((R)-1-carbamoyl-éthylcarbamoyl)-cyclopentylamino]-5-trifluorométhyl-pyrimidin-2-ylamino}-*N*-méthyl-*N*-(1-méthyl-pipéridin-4-yl)-benzamide ;
(±)-4-{4-[(1R*,2S*)-2-(2-amino-2-méthyl-propylcarbamoyl)-cyclopentylamino]-5-trifluorométhyl-pyrimidin-2-ylamino}-*N*-méthyl-*N*-(1-méthyl-pipéridin-4-yl)-benzamide ;
(±)-*N*-méthyl-*N*-(1-méthyl-pipéridin-4-yl)-4-{4-[(1R*,2S*)-2-(2-thiazol-2-yl-carbamoyl)-cyclopentylamino]-5-trifluorométhyl-pyrimidin-2-ylamino}benzamide ;
(±)-4-[4-((1R*,2S*)-2-amino-cyclohexylamino)-5-trifluorométhyl-pyrimidin-2-yl-amino]-*N*-méthyl-*N*-(1-méthyl-pipéridin-4-yl)-benzamide;
(±)-4-[4-((1R*,2S*)-2-acétylamino-cyclohexylamino)-5-trifluorométhyl-pyrmidin-2-ylamino]-*N*-méthyl-*N*-(1-méthyl-pipéridin-4-yl)-benzamide;
(±)-4-[4-((1R*,2S*)-2-méthanesulfonylamino-cyclohexylamino)-5-trifluorométhyl-pyrimidin-2-ylamino]-*N*-méthyl-*N*-(1-méthyl-pipéridin-4-yl)-benzamide ;
(±)-4-[4-((1R*,2S*)-2-isobutyrylamino-cyclohexylamino)-5-trifluorométhylpyrimidin-2-ylamino]-*N*-méthyl-*N*-(1-méthyl-pipéridin-4-yl)-benzamide ;
(±)-4-{4-[(1R*,2S*)-2-(cyclopropanecarbonyl-amino)-cyclohexylamino]-5-trifluorométhyl-pyrimidin-2-ylamino}-*N*-méthyl-*N*-(1-méthyl-pipéridin-4-yl)-benzamide ;
()-*N*-méthyl-*N*-(1-méthyl-pipéridin-4-yl)-4-[4-((1R*,2S*)-2-propionylaminocyclohexylamino)-5-trifluorométhyl-pyrimidin-2-ylamino]-benzamide ;
(±)-4-[4-((1R*,2S*)-2-éthanesulfonylamino-cyclohexylamino)-5-trifluorométhylpyrimidin-2-ylamino]-*N*-méthyl-*N*-(1-méthyl-pipéridin-4-yl)-benzamide ;
(±)-4-{4-[(1R*,2S*)-2-(3,3-diméthyl-uréido)-cyclohexylamino]-5-trifluorométhylpyrimidin-2-ylamino}-*N*-méthyl-*N*-(1-méthyl-pipéridin-4-yl)-benzamide ;
acide (±)-(1R*,2S*,3R*,4S*)-3-(2-{4-[méthyl-(1-méthyl-pipéridin-4-yl)-carbamoyl]-phénylamino}-5-trifluorométhyl-pyrimidin-4-ylamino)-bicyclo-[2.2.1]hept-5-ène-2-carboxylique ;
4-[4-(adamantan-2-ylamino)-5-trifluorométhyl-pyrimidin-2-ylamino]-*N*-méthyl-*N-*(1-méthyl-pipéridin-4-yl)-benzamide ;
cyclopropylamide d'acide (±)-(1R*,2R*,3S*,4S*)-3-(2-{4-[méthyl-(1-méthylpipéridin-4-yl)-carbamoyl]-phénylamino}-5-trifluorométhyl-pyrimidin-4-yl-amino)-bicydo[2.2.1]heptane-2-carboxylique ;
(±)-4-[4-((1R*,2S*)-2-isobutyrylamino-cyclopentylamino)-5-trifluorométhylpyrimidin-2-ylamino]-*N*-méthyl-*N*-(1-méthyl-pipéridin-4-yl)-benzamide ;
(±)-*N*-méthyl-4-{4-[(1R*,2S*)-2-(3-méthyl-butyrylamino)-cyclopentylamino]-5-trifluorométhyl-pyrimidin-2-ylamino}-*N*-(1-méthyl-pipéridin-4-yl)-benzamide ;
(±)-*N*-méthyl-*N*-(1-méthyl-pipéridin-4-yl)-4-[4-((1R*,25*)-2-propionylaminocyclopentylamino)-5-trifluorométhyl-pyrimidin-2-ylamino]-benzamide ;
(±)-4-{4-[(1R*,2S*)-2-(2-méthoxy-acétylamino)-cyclopentylamino]-5-trifluorométhyl-pyàmidin-2-ylamino)-*N*-méthyl-*N*-(1-méthyl-pipéridin-4-yl)-benzamide ;
(±)-4-[4-((1R*,2S*)-2-amino-cyclopentylamino)-5-trifluorométhyl-pyrimidin-2-ylamino]-*N*-méthyl-*N*-(1-méthyl-pipéridin-4-yl)-benzamide ;
*N*-méthyl-*N*-(1-méthyl-pipériudin-4-yl)-4-(4-{(1R,2S)-2-[2-(1-méthyl-pyrrolidin-2-yl)-éthylcarbamoyl]-cyclopentylamino}-5-triftuorométhyl-pyrimidin-2-ylamino)-benzamide ;
4-{4-[(1R,2S)-2-(2,2-difluoro-éthylcarbamoyl)-cyclopentylamino]-5-trifluorométhyl-pyrimidin-2-ylamino}-*N*-méthyl-N-(1-méthyl-pipéridin-4-yl)-benzamide ;
4-{4-[(1R,2S)-2-(2-fluoro-éthylcarbamoyl)-cyclopentylamino]-5-trifluorométhylpyrimidin-2-ylamino}-*N*-méthyl-*N*-(1-méthyl-pipéridin-4-yl)-benzamide ;
2-méthoxy-*N*-méthyl-4-[4-((1R,2S)-2-méthylcarbamoyl-cyclopentylamino)-5-trifluorométhyl-pyrimidin-2-ylamino]-*N*-(1-méthyl-pipéridin-4-yl)-benzamide ;
4-[4-((1R,2S)-2-isopropylcarbamoyl-cyclopentylamino)-5-trifluorométhylpyrimidin-2-ylamino]-2-méthoxy-*N*-méthyl-*N*-(1-méthyl-pipéridin-4-yl)-benzamide ;
4-[4-((1R,2S)-2-cyclopropylcarbamoyl-cyclopentylamino)-5-trifluorométhylpyrimidin-2-ylamino]-2-méthoxy-*N*-méthyl-*N*-(1-méthyl-pipéridin-4-yl)-benzamide ;
4-[4-((1R,2S)-2-isopropylcarbamoyl-cyclopentylamino)-5-trifluorométhylpyrimidin-2-ylamino]-*N*-méthyl-*N*-(1-méthyl-pipéridin-4-yl)-2-propoxybenzamide ;
2-éthoxy-4-[4-((1R,2S)-2-isopropylcarbamoyl-cyclopentylamino)-5-trifluorométhyl-pyrimidin-2-ylamino]-*N*-méthyl-*N*-(1-méthyl-pipéridin-4-yl)-benzamide ;
2-isopropoxy-4-[4-((1R,2S)-2-isopropylcarbamoyl-cyclopentylamino)-5-trifluorométhyl-pyrimidin-2-ylamino]-*N*-méthyl-*N*-(1-méthyl-pipéridin-4-yl)-benzamide ;
4-[4-((1R,2S)-2-isopropylcarbamoyl-cyclopentylamino)-5-trifluorométhylpyrimidin-2-ylamino]-*N*-méthyl-*N*-(1-méthyl-pipéridin-4-yl)-2-prop-2-ynyloxybenzamide ;
(±)-4-[4-((1R*,2S*)-2-isopropylcarbamoyl-cyclobutylamino)-5-trifluorométhylpyrimidin-2-ylamino]-*N*-méthyl-*N*-(1-méthyl-pipéridin-4-yl)-benzamide ;
(±)-4-{5-bromo-4-[(1R*,2S*)-2-(1-méthyl-cyclopropylcarbamoyl)-cyclopentylamino]-pyrimidin-2-ylamino}-*N*-méthyl-*N*-(1-méthyl-pipéridin-4-yl)-benzamide ;
(±)-4-[5-bromo-4-((1R*,2S*)-2-isopropylcarbamoyl-cyclopentylamino)-pyrimidin-2-ylamino]-*N*-méthyl-*N*-(1-méthyl-pipéridin-4-yl)-benzamide ;
(±)-4-[5-bromo-4-((1R*,2S*)-2-cyclopropylcarbamoyl)-cyclopentylamino)-pyrimidin-2-ylamino]-*N*-méthyl-*N*-(1-méthyl-pipéridin-4-yl)-benzamide ;
(±)-4-{5-bromo-4-[(1R*,2S*)-2-(2-cyano-éthylcarbamoyl)-cyclopentylamino]-pyrimidin-2-ylamino}-*N*-méthyl-*N*-(1-méthyl-pipéridin-4-yl)-benzamide ;
(±)-4-[5-bromo-4-((1R*,2S*)-2-cyclopentylcarbamoyl-cyclopentylamino)-pyrimidin-2-ylamino]-*N*-méthyl-*N*-(1-méthyl-pipéridin-4-yl)-benzamide ;
4-{5-bromo-4-[(1R,2S)-2-(pyrrolidine-1-carbonyl)-cyclopentylamino]-pyrimidin-2-ylamino}-*N*-méthyl-*N*-(1-méthyl-pipéridin-4-yl)-benzamide ;
4-{4-[(1R,2S)-2-(azétidine-1-carbonyl)-cyclopentylamino]-5-bromo-pyrimidin-2-ylamino}-*N*-méthyl-*N*-(1-méthyl-pipéridin-4-yl)-benzamide ;
(±)-4-[5-bromo-4-((1R*,2S*)-2-cyclobutylcarbamoyl-cyclopentylamino)-pyrimidin-2-ylamino]-*N*-méthyl-*N*-(1-méthyl-pipéridin-4-yl)-benzamide ;
4-[4-((1R,2S)-2-isopropylcarbamoyl-cyclopentylamino)-5-méthyl-pyrimidin-2-ylamino]-*N*-méthyl-*N*-(1-méthyl-pipéridin-4-yl)-benzamide ;
4-[5-bromo-4-((1R,2S)-2-isopropylcarbamoyl-cyclopentylamino)-pyrimidin-2-ylamino]-*N*-méthyl-*N*-(1-méthyl-pipéridin-4-yl)-benzamide ;
4-[5-chloro-4-((1R,2S)-2-isopropylcarbamoyl-cyclopentylamino)-pyrimidin-2-ylamino]-*N*-méthyl-*N*-(1-méthyl-pipéridin-4-yl)-benzamide.

2. Composés, ou leurs sels pharmaceutiquement actifs, selon la revendication 1 destinés à être utilisés comme médicament.

3. Composés, ou leurs sels pharmaceutiquement actifs, selon la revendication 1 pour la fabrication d'un médicament ayant une action antiproliférative.

4. Préparations pharmaceutiques contenant comme principe actif un ou plusieurs composés de formule générale (1) selon la revendication 1 ou leurs sels pharmaceutiquement actifs éventuellement en combinaison avec des adjuvants et/ou vecteurs courants.

5. Utilisation de composés de formule générale (1) selon la revendication 1 pour la fabrication d'un médicament pour le traitement et/ou la prévention du cancer, des infections, des maladies inflammatoires et auto-immunes.

6. Préparation pharmaceutique comprenant un composé de formule générale (1) selon la revendication 1 et au moins une autre substance active cytostatique ou cytotoxique, différente de la formule (1), éventuellement sous forme de ses tautomères, de ses racémates, de ses énantiomères, de ses diastéréoisomères et de leurs mélanges, et éventuellement de ses sels pharmaceutiquement actifs.
